(19) Europäisches Patentamt
European Patent Office
Office européen des brevets

(11) EP 3 437 743 A1

(12) # EUROPEAN PATENT APPLICATION

(43) Date of publication:
**06.02.2019 Bulletin 2019/06**

(21) Application number: **18197314.0**

(22) Date of filing: **12.05.2011**

(51) Int Cl.:
***B05D 5/08*** *(2006.01)* ***A61M 5/315*** *(2006.01)*
***B05D 7/02*** *(2006.01)* ***B05D 1/00*** *(2006.01)*
***B65D 25/14*** *(2006.01)* ***C23C 16/04*** *(2006.01)*
***C23C 16/40*** *(2006.01)* ***C23C 16/50*** *(2006.01)*
***C23C 16/505*** *(2006.01)* ***C23C 16/54*** *(2006.01)*
***C23C 16/52*** *(2006.01)* ***G01N 15/08*** *(2006.01)*
***C03C 17/30*** *(2006.01)* ***C03C 17/00*** *(2006.01)*
*G01N 33/00* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.05.2010 PCT/US2010/034582**
**12.05.2010 EP 10162758**
**12.05.2010 US 77900710**
**09.08.2010 US 37196710 P**
**12.11.2010 US 41335510 P**
**14.03.2011 US 201161452526 P**
**11.05.2011 PCT/US2011/036097**

(62) Document number(s) of the earlier application(s) in accordance with Art. 76 EPC:
**11722664.7 / 2 569 611**

(71) Applicant: **SiO2 Medical Products, Inc.**
**Auburn, AL 36832 (US)**

(72) Inventors:
- **FELTS, John T.**
  **Alameda, CA California 94501 (US)**
- **FISK, Thomas E.**
  **Green Valley, AZ Arizona 85614 (US)**
- **ABRAMS, Robert S.**
  **Albany, NY New York 12203 (US)**
- **FERGUSON, John**
  **Auburn, AL Alabama 36832 (US)**
- **FREEDMAN, Jonathan R.**
  **Auburn, AL Alabama 36830 (US)**
- **PANGBORN, Robert J.**
  **Harbor Springs, MI Michigan 49740 (US)**
- **SAGONA, Peter J.**
  **Pottstown, PA Pennsylvania 19465 (US)**

(74) Representative: **Maiwald Patent- und Rechtsanwaltsgesellschaft mbH**
**Elisenhof**
**Elisenstraße 3**
**80335 München (DE)**

Remarks:
This application was filed on 27-09-2018 as a divisional application to the application mentioned under INID code 62.

(54) **SYRINGE WITH LUBRICITY COATING**

(57) A syringe is provided having a barrel and a plunger, the barrel having an inner surface and the plunger having a side surface engaging the barrel inner surface, wherein at least one of the barrel inner surface and the plunger side surface serves as substrate which is coated with a lubricity layer; wherein the lubricity layer comprises a layer of $Si_wO_xC_yH_z$ or $Si_wN_xC_yH_z$, where w is 1, x is from about 0.5 to 2.4, y is from about 0.6 to about 3, and z is from 2 to about 9; wherein the surface of the lubricity layer has a lower plunger sliding force or breakout force than the untreated substrate.

EP 3 437 743 A1

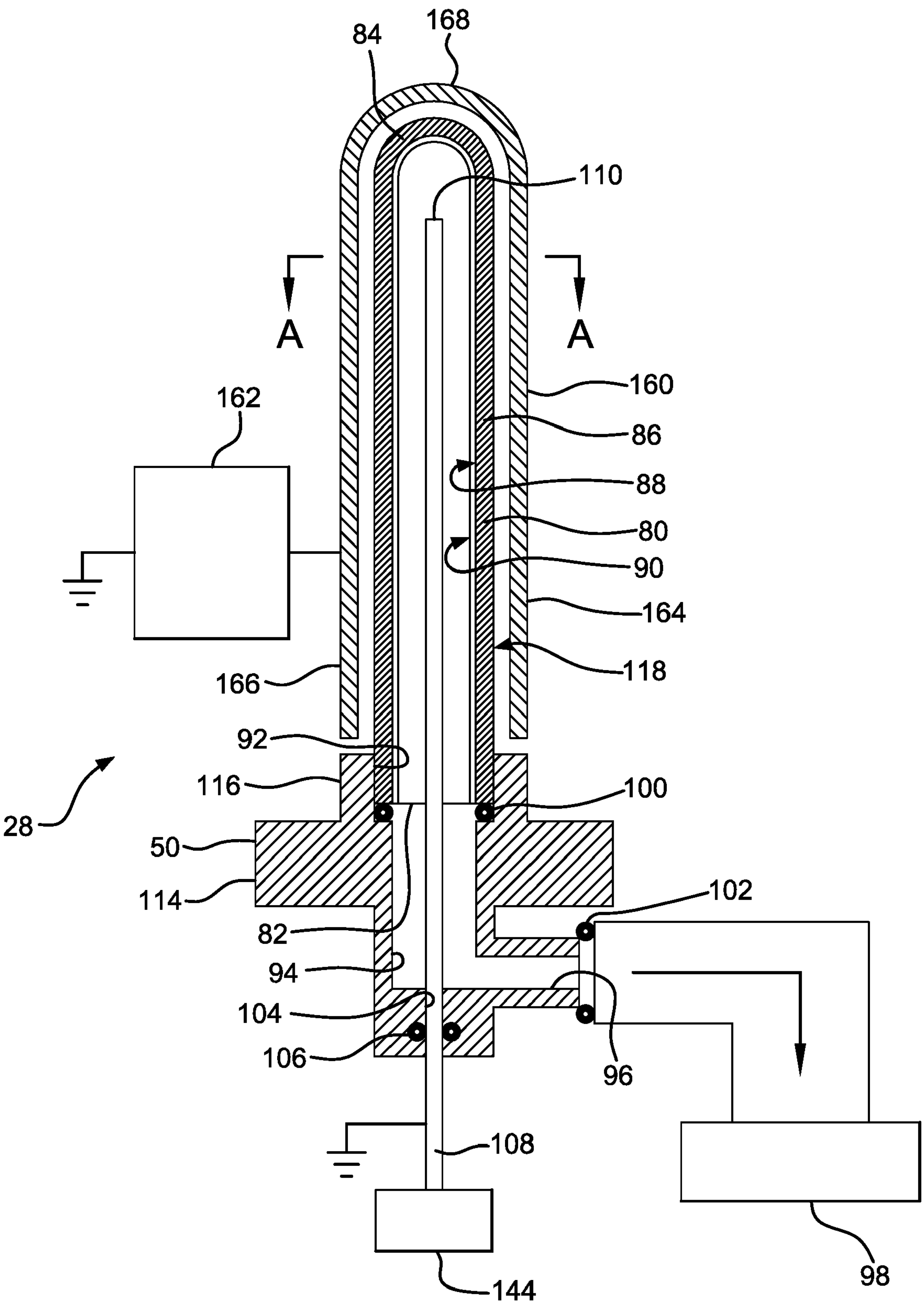
168
84
110
A
A
160
162
86
88
80
90
164
118
166
92
28
116
100
50
114
102
82
94
104
96
106
108
144
98

FIG. 2

## Description

U.S. Provisional Serial Nos. 61/177,984 filed May 13, 2009; 61/222,727, filed July 2, 2009; 61/213,904, filed July 24, 2009; 61/234,505, filed Aug. 17, 2009; 61/261,321, filed November 14, 2009; 61/263,289, filed November 20, 2009; 61/285,813, filed December 11, 2009; 61/298,159, filed January 25, 2010; 61/299,888, filed January 29, 2010; 61/318,197, filed March 26, 2010; 61/333,625, filed May 11, 2010; and 61/413,334, filed November 12, 2010; and US Ser. No. 12/779,007, filed May 12, 2010, are all incorporated here by reference in their entirety.

Also incorporated by reference in their entirety are the following European patent applications: EP10162755.2 filed May 12, 2010; EP10162760.2 filed May 12, 2010; EP10162756.0 filed May 12, 2010; EP10162758.6 filed May 12, 2010; EP10162761.0 filed May 12, 2010; and EP10162757.8 filed May 12, 2010. These European patent applications describe apparatus, vessels, precursors, coatings and methods (in particular coating methods and inspection methods for examining the coatings) which can generally be used in performing the present invention, unless stated otherwise herein. They also describe SiOx barrier coatings and lubricity coatings to which reference is made herein.

Further incorporated by reference is application PCT/US11/36097 filed on 11 May 2011 which also describes coatings and coated items (in particular syringes) which can be inspected by the method of present invention.

Throughout this specification, reference is made to EP2251671 A2 (which corresponds to EP 10162758.6 mentioned above and which also forms a priority application to present application). Therein, the general outgassing method which forms the basis of present invention is described and exemplified, and its contents in this regard are therefore specifically incorporated here by reference.

## FIELD OF THE INVENTION

The present invention relates to the technical field of fabrication of coated vessels for storing biologically active compounds or blood. In particular, the invention relates to a vessel processing system for coating of a vessel, vessel processing system for coating and inspection of a vessel, to a portable vessel holder for a vessel processing system, to a plasma enhanced chemical vapour deposition apparatus for coating an interior surface of a vessel, to a method for coating an interior surface of a vessel, to a method for coating an inspection of a vessel, to a method of processing a vessel, to the use of a vessel processing system, to a computer-readable medium and to a program element.

A method for inspecting the product of a coating process is provided. Therein, the release of at least one volatile species from the coated surface into the gas space adjacent to the coated surface is measured and the result is compared with the result for at least one reference object measured under the same test conditions. Thus the presence or absence of the coating, and/or a physical and/or chemical property of the coating can be determined. The method is useful for inspecting any coated articles, e.g. vessels. Its application on the inspection of PECVD coatings made from organosilicon precursors, especially of barrier coatings, is also disclosed.

The present disclosure also relates to improved methods for processing vessels, for example multiple identical vessels used for venipuncture and other medical sample collection, pharmaceutical preparation storage and delivery, and other purposes. Such vessels are used in large numbers for these purposes, and must be relatively economical to manufacture and yet highly reliable in storage and use.

## BACKGROUND OF THE INVENTION

Evacuated blood collection tubes are used for drawing blood from a patient for medical analysis. The tubes are sold evacuated. The patient's blood is communicated to the interior of a tube by inserting one end of a double-ended hypodermic needle into the patient's blood vessel and impaling the closure of the evacuated blood collection tube on the other end of the double-ended needle. The vacuum in the evacuated blood collection tube draws the blood (or more precisely, the blood pressure of the patient pushes the blood) through the needle into the evacuated blood collection tube, increasing the pressure within the tube and thus decreasing the pressure difference causing the blood to flow. The blood flow typically continues until the tube is removed from the needle or the pressure difference is too small to support flow.

Evacuated blood collection tubes should have a substantial shelf life to facilitate efficient and convenient distribution and storage of the tubes prior to use. For example, a one-year shelf life is desirable, and progressively longer shelf lives, such as 18 months, 24 months, or 36 months, are also desired in some instances. The tube desirably remains essentially fully evacuated, at least to the degree necessary to draw enough blood for analysis (a common standard is that the tube retains at least 90% of the original draw volume), for the full shelf life, with very few (optimally no) defective tubes being provided.

A defective tube is likely to cause the phlebotomist using the tube to fail to draw sufficient blood. The phlebotomist might then need to obtain and use one or more additional tubes to obtain an adequate blood sample.

Prefilled syringes are commonly prepared and sold so the syringe does not need to be filled before use. The

syringe can be prefilled with saline solution, a dye for injection, or a pharmaceutically active preparation, for some examples.

Commonly, the prefilled syringe is capped at the distal end, as with a cap, and is closed at the proximal end by its drawn plunger. The prefilled syringe can be wrapped in a sterile package before use. To use the prefilled syringe, the packaging and cap are removed, optionally a hypodermic needle or another delivery conduit is attached to the distal end of the barrel, the delivery conduit or syringe is moved to a use position (such as by inserting the hypodermic needle into a patient's blood vessel or into apparatus to be rinsed with the contents of the syringe), and the plunger is advanced in the barrel to inject the contents of the barrel.

One important consideration in manufacturing pre-filled syringes is that the contents of the syringe desirably will have a substantial shelf life, during which it is important to isolate the material filling the syringe from the barrel wall containing it, to avoid leaching material from the barrel into the prefilled contents or vice versa.

A further consideration when regarding syringes is to ensure that the plunger can move when it is pressed in the barrel. For this purpose, a lubricity coating, e.g. like the lubricity coating described in the applications cited above, is desirable.

Since many of these vessels are inexpensive and used in large quantities, for certain applications it will be useful to reliably obtain the necessary shelf life without increasing the manufacturing cost to a prohibitive level.

For decades, most parenteral therapeutics have been delivered to end users in Type I medical grade borosilicate glass containers such as vials or pre-filled syringes. The relatively strong, impermeable and inert surface of borosilicate glass has performed adequately for most drug products. However, the recent advent of costly, complex and sensitive biologics as well as such advanced delivery systems as auto injectors has exposed glass' physical and chemical shortcomings including possible contamination from metals and breakage, among other problems. Moreover, glass contains several components which can leach out during storage and cause damage to the stored material. In more detail, borosilicate vessels exhibit a number of drawbacks:

- Glass is manufactured from sand containing a heterogeneous mixture of many elements (silicon, oxygen, boron, aluminum, sodium, calcium) with trace levels of other alkali and earth metals. Type I borosilicate glass consists of approximately 76% $SiO_2$, 10.5% $B_2O_3$, 5% $Al_2O_3$, 7% $Na_2O$ and 1.5% CaO and often contains trace metals such as iron, magnesium, zinc, copper and others. The heterogeneous nature of borosilicate glass creates a non-uniform surface chemistry at the molecular level. Glass forming processes used to create glass containers expose some portions of the containers to temperatures as great as 1200°C. Under such high temperatures alkali ions migrate to the local surface and form oxides. The presence of ions extracted from borosilicate glass devices may be involved in degradation, aggregation and denaturation of some biologics. Many proteins and other biologics must be lyophilized (freeze dried), because they are not sufficiently stable in solution in glass vials or syringes.
- In glass syringes, silicon oil is typically used as a lubricant to allow the plunger to slide in the barrel. Silicon oil has been implicated in the precipitation of protein solutions such as insulin and some other biologics. Additionally, the silicon oil coating is often non-uniform, resulting in syringe failures in the market.
- Glass vessels are prone to breakage or degradation during manufacture, filling operations, shipping and use, which means that glass particulates may enter the drug. The presence of glass particles has led to many FDA Warning Letters and to product recalls.
- Glass-forming processes do not yield the tight dimensional tolerances required for some of the newer auto-injectors and delivery systems.

As a result, some companies have turned to plastic vessels, which provide greater dimensional tolerance and less breakage than glass but lack its impermeability.

Although plastic is superior to glass with respect to breakage, dimensional tolerances and surface uniformity, plastic's use for primary pharmaceutical packaging remains limited due to the following shortcomings:

- Surface characteristics: Plastics suitable for pre-syringes and vials generally exhibit hydrophobic surfaces, which often reduce the stability of the biologic drug contained in the device.
- Gas (oxygen) permeability: Plastic allows small molecule gases to permeate into (or out of) the device. Plastics' permeability to gases is significantly greater than that of glass and, in many cases (as with oxygen-sensitive drugs such as epinephrine), plastics are unacceptable for that reason.
- Water vapor transmission: Plastics allow water vapors to pass through devices to a greater degree than glass. This can be detrimental to the shelf life of a solid (lyophilized) drug. Alternatively, a liquid product may lose water in an arid environment.
- Leachables and extractables: Plastic vessels contain organic compounds that can leach out or be extracted into the drug product. These compounds can contaminate the drug and/or negatively impact the drug's stability.

Clearly, while plastic and glass vessels each offer certain advantages in pharmaceutical primary packaging, neither is optimal for all drugs, biologics or other therapeutics. Thus, there is a desire for plastic vessels, in particular plastic syringes, with gas and solute barrier properties which approach the properties of glass. Moreover, there is a need for plastic syringes with sufficient lubricity properties and a lubricity coating which is compatible with the syringe contents.

Coating plastic vessels with a barrier coating or lubricity coating made by PECVD using organosilicon precursors as described in one of the above cited applications can provide such desired vessels. However, in order to ensure their economic production, an inspection method allowing the inspection of the coating is also required.

A non-exhaustive list of patents of possible relevance includes US Patents 6,068,884 and 4,844,986 and U.S. Published Applications 20060046006 and 20040267194.

## SUMMARY OF THE INVENTION

The present invention provides a method for inspecting a surface, in particular a coated surface and specifically a plastic surface coated with a PECVD coating made from organosilicon precursors. The inspection in performed by detecting outgassing from the inspected item, in particular outgassing from the coated substrate. The invention in its main application provides a method for vessel inspection by detecting the outgassing of a vessel wall, e.g. through a barrier layer coated onto the vessel wall. The invention further pertains to a method encompassing the coating and inspection of a vessel, wherein the inspection is performed by the outgassing method according to present invention. In a particular aspect, the invention pertains to a method encompassing a PECVD coating step and an inspection step wherein the latter is performed by the outgassing method.

The outgassing method of present invention allows a quick and accurate determination of the properties of an inspected material. For example, it allows the quick and accurate determination whether a coating, e.g. a barrier coating is present on a plastic substrate.

The invention further pertains to an apparatus (e.g. a vessel processing station of a vessel processing system) configured for performing the above and/or below mentioned method steps. Examples of such processing stations 20, 30, 5501-5504 are depicted in FIGS. 1 and 15-17, for example.

The invention further pertains to a computer-readable medium, in which a computer program for inspection of a surface is stored which, when being executed by a processor of an apparatus, such as a vessel processing system, is adapted to instruct the processor to control the apparatus such that it carries out the above and/or below mentioned method steps.

The invention further pertains to a program element or computer program for inspection of a surface, which, when being executed by a processor of an apparatus, such as a vessel processing system, is adapted to instruct the processor to control the apparatus such that it carries out the above and/or below mentioned method steps.

The processor may thus be equipped to carry out exemplary embodiments of the methods of the present invention. The computer program may be written in any suitable programming language, for example, C++ and may be stored on the computer-readable medium, such as a CD-ROM. Also, the computer program may be available from a network, such as the WorldWideWeb, from which it may be downloaded into image processing units or processors, or any suitable computers.

An aspect of the invention is a method and system for inspecting a coating or barrier layer on a substrate or other object made of a first material and having a surface, for example an $SiO_x$ coating on a cyclic olefin copolymer ("COC") thermoplastic molded substrate as the object made of the first material, such as a syringe or blood collection tube, by measuring a volatile species outgassed by the coated article, which may be a volatile species originating from the material of the inspected item or originating from a charging material, for example a charging material comprising for example carbon dioxide, nitrogen, argon, xenon, krypton, a lower hydrocarbon having from 1 to 4 carbon atoms, for example ethylene, ethane, or methane, a lower halogenated hydrocarbon having from 1 to four carbon atoms, for example methyl chloride, methylene chloride, methyl fluoride or trifluoroethane, or a lower hydrocarbon ether having from 1 to four carbon atoms, for example diethylether or dimethylether or ethyl-methyl-ether, which is brought into contact with the inspected item before measuring of the outgassed volatile species takes place ("Outgassing method"). The method can be used for inspecting the product of a coating process wherein a coating has been applied to the surface of a substrate to form a coated surface. The method is carried out under conditions effective to determine, e.g., the presence or absence of a barrier layer. For example, the method can be used as an inline process control for a coating process in order to identify and eliminate coated products not meeting a predetermined standard or damaged coating products.

Generally, the "volatile species" is a gas or vapor at test conditions, and may be selected from the group consisting of a noble gas, carbon dioxide, a hydrocarbon, a halogenated hydrocarbon, an ether, air, nitrogen, oxygen, water vapor, volatile coating components, volatile substrate components, volatile reaction products of the coating, and a combination thereof, optionally is air, nitrogen, oxygen, carbon dioxide, argon, water vapor, or a combination thereof. The volatile species may be charged on the inspected item before inspection, or it may be present in the material without charging (e.g. as volatile substrate components or residual reaction products of the coating). The method can be used

to measure just one or a few volatile species, but optionally a plurality of different volatile species is measured, and optionally substantially all the volatile species released from the inspection object are measured.

Another aspect of the invention is a method for inspecting an object, e.g. for the presence of a barrier layer, wherein the method encompasses charging the inspected object with a charging material. In this method, an object is provided, made at least in part of a first material, the object having a surface, the object optionally having at least a partial barrier layer between the first material and the surface (in one aspect, the barrier layer forms the surface of the object, i.e. the object is coated with the barrier layer). Optionally, a charging material is provided that is soluble in, absorbed by, or adsorbed by the first material, and the object is contacted with the charging material. Outgassing is measured from at least a portion of the surface. The method is carried out under conditions effective to determine, e.g., the presence or absence of a barrier layer.

The charging material can enhance the sensitivity of the outgassing method. In particular, it can simplify discrimination between two inspected species, e.g. a substrate which is once coated and once uncoated. The charging material can also allow discrimination between two materialsif it is bound and outgassed by different materials in different amounts.

The discrimination of uncoated versus $SiO_x$-coated plastic articles relies on the capability of a plastic article to imbibe gases (e.g. a charging material) which can subsequently be outgassed and measured. The solubility of gases in plastics is a key determinant of the amount of gas which can reside in a plastic, and therefore a good estimate of the potential for a particular gas to provide suitable uncoated versus coated article discrimination. It is in one aspect of the invention advantageous to increase the amount of gas which is absorbed or adsorbed in the plastic by charging the plastic with a charging material. This can result in higher amounts of outgassed volatile species emitted during the outgassing measurement and thus enhance readout.

In a specific aspect of present invention,increased sensitivity of the outgassing measurement can be realized by using a carbon dioxide flush and/or by spiking the substrate with carbon dioxide (e.g. carbon dioxide gas), $N_2$, Ar, xenon, krypton, a lower hydrocarbon having from 1 to 4 carbon atoms, for example ethylene, ethane, or methane, a lower halogenated hydrocarbon having from 1 to four carbon atoms, for example methyl chloride, methylene chloride, methyl fluoride or trifluoroethane, or a lower hydrocarbon ether having from 1 to four carbon atoms, for example diethylether or dimethylether or ethyl-methyl-ether,. The use of $CO_2$ is particularly useful when testing COC substrates where the solubility for nitrogen, water and oxygen are low.

Generally, the outgassing method according to present invention comprises the steps:

(a) providing a product as inspection object;

(c) measuring the release of at least one volatile species from the inspection object into the gas space adjacent to a surface of the object; and

(d) comparing the result of step (c) with the result of step (c) for at least one reference object measured under the same test conditions, thus determining the presence or absence of a specific material or a specific component of the inspection object, e.g. of a coating on a substrate.

Typically, an outgassing method for inspecting a coated substrate according to present invention comprises the steps:

(a) providing a product comprising a coating as inspection object;

(c) measuring the release of at least one volatile species from the inspection object into the gas space adjacent to the coated surface; and

(d) comparing the result of step (c) with the result of step (c) for at least one reference object measured under the same test conditions, thus determining the presence or absence of the coating, and/or a physical and/or chemical property of the coating or of the coated substrate.

In the outgassing method, the physical and/or chemical property of the coating to be determined can be selected from the group consisting of its barrier effect, its wetting tension, and its composition, and optionally is its barrier effect.

Advantageously, step (c) is performed by measuring the mass flow rate or volume flow rate of the at least one volatile species in the gas space adjacent to the coated surface.

Optionally, the reference object (i) is an uncoated substrate; or (ii) is a substrate coated with a reference coating. This depends on, e.g., whether the outgassing method is used to determine the presence or absence of a coating (then the reference object can be an uncoated substrate) or to determine the properties of the coating, e.g. in comparison to a coating with known properties, or e.g. of the inspected material in general in comparison to a known material. For

determining the coating's identity with a specific coating, a reference coating will also be a typical choice.

The outgassing method can also comprise as an additional step between steps (a) and (c) the step of (b) changing the atmospheric pressure in the gas space adjacent to the coated surface such that a pressure differential through the coated surface is provided and a higher mass flow rate or volume flow rate of the volatile species can be realized than without the pressure differential. In this case, the volatile species will migrate into the direction of the lower side of the pressure differential. If the coated object is a vessel, the pressure differential is established between the vessel lumen and the exterior in order to measure the outgassing of the volatile species from the coated vessel wall. The pressure differential can e.g. be provided by at least partially evacuating the gas space in the vessel. In this case, the volatile species can be measured which is outgassed into the lumen of the vessel.

If a vacuum is applied to create a pressure differential, the measurement can be performed by using a measurement cell interposed between the coated surface of the substrate and a source of vacuum.

In another aspect, the inspection object can be contacted with a volatile species in step (a), optionally a volatile species selected from the group consisting of air, nitrogen, oxygen, water vapor, carbon dioxide, a noble gas, and a combination thereof, optionally in order to allow the adsorption or absorption of the volatile species onto or into the material of the inspection object. Then, the subsequent release of volatile species from the inspection object is measured in step (c). As different materials (like, e.g., the coating and the substrate) have different adsorption and absorption characteristics, this can simplify the determination of the presence and characteristics of a coating.

The substrate can be a polymeric compound, optionally is a polyester, a polyolefin, a cyclic olefin copolymer, a polycarbonate, or a combination of these. The outgassing method of the invention is of particular interest for the inspection of COC, polyethylene terephtalate and polypropylene.

In the context of present invention, the coating characterized by the outgassing method may be any coating, but is typically a coating prepared by PECVD from, e.g., an organosilicon precursor as described herein and in the applications cited in paragraphs 1 to 3 of this description, to which applications explicit reference is made herewith in order to point out coatings on which present invention can be applied. For example, the coating can be a barrier layer, optionally is a $SiO_x$ layer wherein x is from about 1.5 to about 2.9. For another example, the coating can be a lubricity layer, characterized as defined in the Definition Section or in the cited applications and/or a hydrophobic layer, characterized as defined in the Definition Section or in the cited applications.

When the coating process whose product is inspected by the outgassing method is a PECVD coating performed under vacuum conditions, the subsequent outgassing measurement can even be conducted without breaking the vacuum used for PECVD.

The volatile species measured can be a volatile species released from the coating, a volatile species release from the substrate, or a combination of both. In one aspect, the volatile species is a volatile species released from the coating, optionally is a volatile coating component, and the inspection is performed to determine the presence, the properties and/or the composition of the coating. In another aspect, the volatile species is a volatile species released from the substrate and the inspection is performed to determine the presence of the coating and/or the barrier effect of the coating.

The outgassing method of the present invention is suitable to determine the presence and characteristics of a coating on a vessel wall. Thus, the coated substrate can be a vessel having a wall which is at least partially coated on its inner or outer surface during the coating process. For example, the coating is disposed on the inner surface of the vessel wall.

The conditions effective to distinguish the presence or absence of the coating, and/or to determine a physical and/or chemical property of the coating can include a test duration of less than one hour, or less than one minute, or less than 50 seconds, or less than 40 seconds, or less than 30 seconds, or less than 20 seconds, or less than 15 seconds, or less than 10 seconds, or less than 8 seconds, or less than 6 seconds, or less than 4 seconds, or less than 3 seconds, or less than 2 seconds, or less than 1 second.

In order to increase, e.g., the difference between a reference object and the inspection object with regard to the release rate and/or kind of the measured volatile species, the release rate of the volatile species can be modified by modifying the ambient pressure and/or temperature, and/or humidity. The use of a charging material according to present invention serves the same purpose.

The measurement may, e.g., be carried out using a microflow technique.

In an aspect, the outgassing is measured using a microcantilever measurement technique. E.g., the measuring can be carried out by

(i) (a) providing at least one microcantilever which has the property, when in the presence of an outgassed material, of moving or changing to a different shape;

(b) exposing the microcantilever to the outgassed material under conditions effective to cause the microcantilever to move or change to a different shape; and

(c) detecting the movement or different shape, optionally by reflecting an energetic incident beam, e.g. a laser beam, from a portion of the microcantilever that changes shape, before and after exposing the microcantilever to outgassing, and measuring the resulting deflection of the reflected beam at a point spaced from the cantilever; or by

(ii) (a) providing at least one microcantilever which resonates at a different frequency when in the presence of an outgassed material;

(b) exposing the microcantilever to the outgassed material under conditions effective to cause the microcantilever to resonate at a different frequency; and

(c) detecting the different resonant frequency, e.g. using a harmonic vibration sensor.

An apparatus for performing the outgassing method is also considered, for example an apparatus comprising a microcantilever as described above.

Using the outgassing method of the present invention, for example a barrier layer on a material that outgasses a vapor can be inspected, wherein the inspection method has several steps. A sample of material that outgasses a gas and has at least a partial barrier layer is provided. In an aspect of the invention, a pressure differential is provided across the barrier layer, such that at least some of the material that outgasses is on the higher-pressure side of the barrier layer. The outgassed gas passing through the barrier layer is measured. If a pressure differential is present, the measurement typically is performed on the lower-pressure side of the barrier layer.

Other aspects of the invention will be apparent from this disclosure and the accompanying drawings.

BRIEF DESCRIPTION OF THE DRAWINGS

FIG. 1 is a schematic diagram showing a vessel processing system according to an embodiment of the disclosure.

FIG. 2 is a schematic sectional view of a vessel holder in a coating station according to an embodiment of the disclosure.

FIG. 3 is an exploded longitudinal sectional view of a syringe and cap adapted for use as a prefilled syringe.

FIG. 4 is schematic sectional view of another embodiment of the invention for processing syringe barrels and other vessels.

FIG. 5 is an enlarged detail view of the processing vessel of FIG. 4.

FIG. 6 is a schematic view showing outgassing of a material through a coating.

FIG. 7 is a schematic sectional view of a test set-up for causing outgassing of the wall of a vessel to the interior of the vessel and measurement of the outgassing using a measurement cell interposed between the vessel and a source of vacuum.

FIG. 8 is a plot of outgassing mass flow rate measured on the test-set-up of FIG. 7 for multiple vessels.

FIG. 9 is a bar graph showing a statistical analysis of the endpoint data shown in FIG. 8.

FIG.10 is a perspective view of a double-walled blood collection tube assembly.

FIG. 11 is an alternative construction for a vessel holder usable, for example, with the embodiments of FIGS. 1, 2, and 4.

FIG. 12 is a schematic view of an assembly for treating vessels. The assembly is usable with the apparatus of any of the preceding figures.

FIG. 13 is a plot of outgassing mass flow rate measured in Example 19 of EP2251671 A2.

FIG. 14 shows a linear rack.

FIG. 15 shows a schematic representation of a vessel processing system according to an exemplary embodiment of the present invention.

FIG. 16 shows a schematic representation of a vessel processing system according to another exemplary embodiment of the present invention.

FIG. 17 shows a processing station of a vessel processing system according to an exemplary embodiment of the present invention.

FIG. 18 shows a portable vessel holder according to an exemplary embodiment of the present invention.

FIG. 19 is a plot of outgassing mass flow rate measured on the test-set-up of FIG. 7, representing the data of Example 10 of EP2251671 A2.

The following reference characters are used in the drawing figures:

| | |
|---|---|
| 20 | Vessel processing system |
| 22 | Injection molding machine |
| 24 | Visual inspection station |
| 26 | Inspection station (pre-coating) |
| 28 | Coating station |
| 30 | Inspection station (post-coating) |
| 32 | Optical source transmission station (thickness) |
| 34 | Optical source transmission station (defects) |
| 36 | Output |
| 38 | Vessel holder |
| 40 | Vessel holder |
| 42 | Vessel holder |
| 44 | Vessel holder |
| 46 | Vessel holder |
| 48 | Vessel holder |
| 50 | Vessel holder |
| 52 | Vessel holder |
| 54 | Vessel holder |
| 56 | Vessel holder |
| 58 | Vessel holder |
| 60 | Vessel holder |
| 62 | Vessel holder |
| 64 | Vessel holder |
| 66 | Vessel holder |
| 68 | Vessel holder |
| 70 | Conveyor |
| 72 | Transfer mechanism (on) |
| 74 | Transfer mechanism (off) |
| 80 | Vessel |
| 82 | Opening |
| 84 | Closed end |
| 86 | Wall |
| 88 | Interior surface |

| | |
|---|---|
| 90 | Barrier layer |
| 92 | Vessel port |
| 94 | Vacuum duct |
| 96 | Vacuum port |
| 98 | Vacuum source |
| 100 | O-ring (of 92) |
| 102 | O-ring (of 96) |
| 104 | Gas inlet port |
| 106 | O-ring (of 100) |
| 108 | Probe (counter electrode) |
| 110 | Gas delivery port (of 108) |
| 114 | Housing (of 50 or 112) |
| 116 | Collar |
| 118 | Exterior surface (of 80) |
| 120 | Vessel holder (array) |
| 122 | Vessel port (FIG. 14) |
| 144 | PECVD gas source |
| 152 | Pressure gauge |
| 160 | Electrode |
| 162 | Power supply |
| 164 | Sidewall (of 160) |
| 166 | Sidewall (of 160) |
| 168 | Closed end (of 160) |
| 250 | Syringe barrel |
| 252 | Syringe |
| 254 | Interior surface (of 250) |
| 256 | Back end (of 250) |
| 258 | Plunger (of 252) |
| 260 | Front end (of 250) |
| 262 | Cap |
| 264 | Interior surface (of 262) |
| 268 | Vessel |
| 270 | Closure |
| 274 | Lumen |
| 290 | Apparatus for coating, for example, 250 |

| | |
|---|---|
| 292 | Inner surface (of 294) |
| 294 | Restricted opening (of 250) |
| 296 | Processing vessel |
| 298 | Outer surface (of 250) |
| 300 | Lumen (of 250) |
| 302 | Larger opening (of 250) |
| 304 | Processing vessel lumen |
| 306 | Processing vessel opening |
| 308 | Inner electrode |
| 310 | Interior passage (of 308) |
| 312 | Proximal end (of 308) |
| 314 | Distal end (of 308) |
| 316 | Distal opening (of 308) |
| 318 | Plasma |
| 332 | First fitting (male Luer taper) |
| 334 | Second fitting (female Luer taper) |
| 336 | Locking collar (of 332) |
| 338 | First abutment (of 332) |
| 340 | Second abutment (of 332) |
| 342 | O-ring |
| 344 | Dog |
| 346 | Wall |
| 348 | Coating (on 346) |
| 350 | Permeation path |
| 354 | Gas molecule |
| 355 | Gas molecule |
| 356 | Interface (between 346 and 348) |
| 357 | Gas molecule |
| 358 | PET vessel |
| 359 | Gas molecule |
| 360 | Seal |
| 362 | Measurement cell |
| 364 | Vacuum pump |

| | |
|---|---|
| 366 | Arrows |
| 368 | Conical passage |
| 370 | Bore |
| 372 | Bore |
| 374 | Chamber |
| 376 | Chamber |
| 378 | Diaphragm |
| 380 | Diaphragm |
| 382 | Conductive surface |
| 384 | Conductive surface |
| 386 | Bypass |
| 390 | Plot (glass tube) |
| 392 | Plot (PET uncoated) |
| 394 | Main plot ($SiO_2$ coated) |
| 396 | Outliers ($SiO_2$ coated) |
| 404 | Vent |
| 408 | Inner wall (FIG. 10) |
| 410 | Outer wall (FIG. 10) |
| 482 | Vessel holder body |
| 484 | Upper portion (of 482) |
| 486 | Base portion (of 482) |
| 488 | Joint (between 484 and 486) |
| 490 | O-ring |
| 492 | Annular pocket |
| 494 | Radially extending abutment surface |
| 496 | Radially extending wall |
| 498 | Screw |
| 500 | Screw |
| 502 | Vessel port |
| 504 | Second O-ring |
| 506 | Inner diameter (of 490) |
| 508 | Vacuum duct (of 482) |
| 574 | Main vacuum valve |
| 576 | Vacuum line |

| | |
|---|---|
| 578 | Manual bypass valve |
| 580 | Bypass line |
| 582 | Vent valve |
| 584 | Main reactant gas valve |
| 586 | Main reactant feed line |
| 588 | Organosilicon liquid reservoir |
| 590 | Organosilicon feed line (capillary) |
| 592 | Organosilicon shut-off valve |
| 594 | Oxygen tank |
| 596 | Oxygen feed line |
| 598 | Mass flow controller |
| 600 | Oxygen shut-off valve |
| 614 | Headspace |
| 616 | Pressure source |
| 618 | Pressure line |
| 620 | Capillary connection |
| 630 | Plots for uncoated COC |
| 632 | Plots for SiOx coated COC |
| 634 | Plots for glass |
| 5501 | First processing station |
| 5502 | Second processing station |
| 5503 | Third processing station |
| 5504 | Fourth processing station |
| 5505 | Processor |
| 5506 | User interface |
| 5507 | Bus |
| 5701 | PECVD apparatus |
| 5702 | First detector |
| 5703 | Second detector |
| 5704 | Detector |
| 5705 | Detector |
| 5706 | Detector |
| 5707 | Detector |
| 7001 | Conveyor exit branch |
| 7002 | Conveyor exit branch |
| 7003 | Conveyor exit branch |
| 7004 | Conveyor exit branch |

## DETAILED DESCRIPTION OF THE INVENTION

The present invention will now be described more fully *inter alia* with reference to the accompanying drawings, in which several embodiments are shown. This invention can, however, be embodied in many different forms and should not be construed as limited to the embodiments set forth here. Rather, these embodiments are examples of the invention, which has the full scope indicated by the language of the claims. Like numbers refer to like or corresponding elements

throughout.

**DEFINITION SECTION**

In the context of the present invention, the following definitions and abbreviations are used:
RF is radio frequency; sccm is standard cubic centimeters per minute.

The term "at least" in the context of the present invention means "equal or more" than the integer following the term. The word "comprising" does not exclude other elements or steps, and the indefinite article "a" or "an" does not exclude a plurality unless indicated otherwise. Whenever a parameter range is indicated, it is intended to disclose the parameter values given as limits of the range and all values of the parameter falling within said range.

"First" and "second" or similar references to, e.g., processing stations or processing devices refer to the minimum number of processing stations or devices that are present, but do not necessarily represent the order or total number of processing stations and devices. These terms do not limit the number of processing stations or the particular processing carried out at the respective stations.

For purposes of the present invention, an "organosilicon precursor" is a compound having at least one of the linkage:

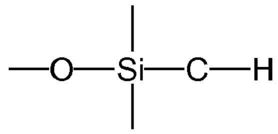

or

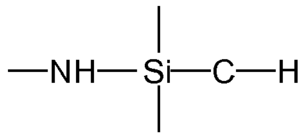

which is a tetravalent silicon atom connected to an oxygen atom and an organic carbon atom (an organic carbon atom being a carbon atom bonded to at least one hydrogen atom). A volatile organosilicon precursor, defined as such a precursor that can be supplied as a vapor in a PECVD apparatus, is an optional organosilicon precursor. Optionally, the organosilicon precursor is selected from the group consisting of a linear siloxane, a monocyclic siloxane, a polycyclic siloxane, a polysilsesquioxane, an alkyl trimethoxysilane, a linear silazane, a monocyclic silazane, a polycyclic silazane, a polysilsesquiazane, and a combination of any two or more of these precursors.

The feed amounts of PECVD precursors, gaseous reactant or process gases, and carrier gas are sometimes expressed in "standard volumes" in the specification and claims. The standard volume of a charge or other fixed amount of gas is the volume the fixed amount of the gas would occupy at a standard temperature and pressure (without regard to the actual temperature and pressure of delivery). Standard volumes can be measured using different units of volume, and still be within the scope of the present disclosure and claims. For example, the same fixed amount of gas could be expressed as the number of standard cubic centimeters, the number of standard cubic meters, or the number of standard cubic feet. Standard volumes can also be defined using different standard temperatures and pressures, and still be within the scope of the present disclosure and claims. For example, the standard temperature might be 0°C and the standard pressure might be 760 Torr (as is conventional), or the standard temperature might be 20°C and the standard pressure might be 1 Torr. But whatever standard is used in a given case, when comparing relative amounts of two or more different gases without specifying particular parameters, the same units of volume, standard temperature, and standard pressure are to be used relative to each gas, unless otherwise indicated.

The corresponding feed rates of PECVD precursors, gaseous reactant or process gases, and carrier gas are expressed in standard volumes per unit of time in the specification. For example, in the working examples the flow rates are expressed as standard cubic centimeters per minute, abbreviated as sccm. As with the other parameters, other units of time can be used, such as seconds or hours, but consistent parameters are to be used when comparing the flow rates of two or more gases, unless otherwise indicated.

A "vessel" in the context of the present invention can be any type of vessel with at least one opening and a wall defining an interior surface. The coating substrate can be the inside wall of a vessel having a lumen. Though the invention is not necessarily limited to vessels of a particular volume, vessels are contemplated in which the lumen has a void volume of from 0.5 to 50 mL, optionally from 1 to 10 mL, optionally from 0.5 to 5 mL, optionally from 1 to 3 mL.

The substrate surface can be part or all of the inner surface of a vessel having at least one opening and an inner surface.

The term "at least" in the context of the present invention means "equal or more" than the integer following the term. Thus, a vessel in the context of the present invention has one or more openings. One or two openings, like the openings of a sample tube (one opening) or a syringe barrel (two openings) are preferred. If the vessel has two openings, they can be of same or different size. If there is more than one opening, one opening can be used for the gas inlet for a PECVD coating method according to the present invention, while the other openings are either capped or open. A vessel according to the present invention can be a sample tube, e.g. for collecting or storing biological fluids like blood or urine, a syringe (or a part thereof, for example a syringe barrel) for storing or delivering a biologically active compound or composition, e.g. a medicament or pharmaceutical composition, a vial for storing biological materials or biologically active compounds or compositions, a pipe, e.g. a catheter for transporting biological materials or biologically active compounds or compositions, or a cuvette for holding fluids, e.g. for holding biological materials or biologically active compounds or compositions.

A vessel can be of any shape, a vessel having a substantially cylindrical wall adjacent to at least one of its open ends being preferred. Generally, the interior wall of the vessel is cylindrically shaped, like, e.g. in a sample tube or a syringe barrel. Sample tubes and syringes or their parts (for example syringe barrels) are contemplated.

A "hydrophobic layer" in the context of the present invention means that the coating lowers the wetting tension of a surface coated with the coating, compared to the corresponding uncoated surface. Hydrophobicity is thus a function of both the uncoated substrate and the coating. The same applies with appropriate alterations for other contexts wherein the term "hydrophobic" is used. The term "hydrophilic" means the opposite, i.e. that the wetting tension is increased compared to reference sample. The present hydrophobic layers are primarily defined by their hydrophobicity and the process conditions providing hydrophobicity, and optionally can have a composition according to the empirical composition or sum formula $Si_wO_xC_yH_z$.

It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

These values of w, x, y and z are applicable to the empirical composition $Si_wO_xC_yH_z$, throughout this specification. The values of w, x, y, and z used throughout this specification should be understood as ratios or an empirical formula (e.g. for a coating), rather than as a limit on the number or type of atoms in a molecule. For example, octamethylcyclotetrasiloxane, which has the molecular composition $Si_4O_4C_8H_{24}$, can be described by the following empirical formula, arrived at by dividing each of w, x, y, and z in the molecular formula by 4, the largest common factor: $Si_1O_1C_2H_6$. The values of w, x, y, and z are also not limited to integers. For example, (acyclic) octamethyltrisiloxane, molecular composition $Si_3O_2C_8H_{24}$, is reducible to $Si_1O_{0.67}C_{2.67}H_8$.

"Wetting tension" is a specific measure for the hydrophobicity or hydrophilicity of a surface. An optional wetting tension measurement method in the context of the present invention is ASTM D 2578 or a modification of the method described in ASTM D 2578. This method uses standard wetting tension solutions (called dyne solutions) to determine the solution that comes nearest to wetting a plastic film surface for exactly two seconds. This is the film's wetting tension. The procedure utilized is varied herein from ASTM D 2578 in that the substrates are not flat plastic films, but are tubes made according to the Protocol for Forming PET Tube and (except for controls) coated according to the Protocol for Coating Tube Interior with Hydrophobic Layer (see Example 9 of EP2251671 A2).

A "lubricity layer" according to the present invention is a coating which has a lower frictional resistance than the uncoated surface. In other words, it reduces the frictional resistance of the coated surface in comparison to a reference surface which is uncoated. The present lubricity layers are primarily defined by their lower frictional resistance than the uncoated surface and the process conditions providing lower frictional resistance than the uncoated surface, and optionally can have a composition according to the empirical composition $Si_wO_xC_yH_z$. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

"Frictional resistance" can be static frictional resistance and/or kinetic frictional resistance. One of the optional embodiments of the present invention is a syringe part, e.g. a syringe barrel or plunger, coated with a lubricity layer. In

this contemplated embodiment, the relevant static frictional resistance in the context of the present invention is the breakout force as defined herein, and the relevant kinetic frictional resistance in the context of the present invention is the plunger sliding force as defined herein. For example, the plunger sliding force as defined and determined herein is suitable to determine the presence or absence and the lubricity characteristics of a lubricity layer in the context of the present invention whenever the coating is applied to any syringe or syringe part, for example to the inner wall of a syringe barrel. The breakout force is of particular relevance for evaluation of the coating effect on a prefilled syringe, i.e. a syringe which is filled after coating and can be stored for some time, e.g. several months or even years, before the plunger is moved again (has to be "broken out").

"Slidably" means that the plunger, closure, or other removable part is permitted to slide in a syringe barrel or other vessel.

In the context of this invention, "substantially rigid" means that the assembled components (ports, duct, and housing, explained further below) can be moved as a unit by handling the housing, without significant displacement of any of the assembled components respecting the others. Specifically, none of the components are connected by hoses or the like that allow substantial relative movement among the parts in normal use. The provision of a substantially rigid relation of these parts allows the location of the vessel seated on the vessel holder to be nearly as well known and precise as the locations of these parts secured to the housing.

In the following, the apparatus for performing the present invention will be described first, followed by the coating methods, coatings and coated vessels, and then the outgassing method according to the present invention will be described in more detail.

## I. VESSEL PROCESSING SYSTEM HAVING MULTIPLE PROCESSING STATIONS AND MULTIPLE VESSEL HOLDERS

I. A vessel processing system is contemplated comprising a first processing station, a second processing station, a multiplicity of vessel holders, and a conveyor. The first processing station is configured for processing a vessel having an opening and a wall defining an interior surface. The second processing station is spaced from the first processing station and configured for processing a vessel having an opening and a wall defining an interior surface.

I. At least some, optionally all, of the vessel holders include a vessel port configured to receive and seat the opening of a vessel for processing the interior surface of a seated vessel via the vessel port at the first processing station. The conveyor is configured for transporting a series of the vessel holders and seated vessels from the first processing station to the second processing station for processing the interior surface of a seated vessel via the vessel port at the second processing station.

I. Referring first to FIG. 1, a vessel processing system generally indicated as 20 is shown. The vessel processing system can include processing stations which more broadly are contemplated to be processing devices. The vessel processing system 20 of the illustrated embodiment can include an injection molding machine 22 (which can be regarded as a processing station or device), additional processing stations or devices 24, 26, 28, 30, 32, and 34, and an output 36 (which can be regarded as a processing station or device). At a minimum, the system 20 has at least a first processing station, for example station 28, and a second processing station, for example 30, 32, or 34.

I. Any of the processing stations 22-36 in the illustrated embodiment can be a first processing station, any other processing station can be a second processing station, and so forth.

I. The embodiment illustrated in FIG. 1 can include eight processing stations or devices: 22, 24, 26, 28, 30, 32, 34, and 36. The exemplary vessel processing system 20 includes an injection molding machine 22, a post-molding inspection station 24, a pre-coating inspection station 26, a coating station 28, a post-coating inspection station 30, an optical source transmission station 32 to determine the thickness of the coating, an optical source transmission station 34 to examine the coating for defects, and an output station 36.

I. The system 20 can include a transfer mechanism 72 for moving vessels from the injection molding machine 22 to a vessel holder 38. The transfer mechanism 72 can be configured, for example, as a robotic arm that locates, moves to, grips, transfers, orients, seats, and releases the vessels 80 to remove them from the vessel forming machine 22 and install them on the vessel holders such as 38.

I. The system 20 also can include a transfer mechanism at a processing station 74 for removing the vessel from

one or more vessel holders such as 66, following processing the interior surface of the seated vessel such as 80 (FIG. 1). The vessels 80 are thus movable from the vessel holder 66 to packaging, storage, or another appropriate area or process step, generally indicated as 36. The transfer mechanism 74 can be configured, for example, as a robotic arm that locates, moves to, grips, transfers, orients, places, and releases the vessels 80 to remove them from the vessel holders such as 38 and place them on other equipment at the station 36.

I. The processing stations or devices 32, 34, and 36 shown in FIG. 1 optionally carry out one or more appropriate steps downstream of the coating and inspection system 20, after the individual vessels 80 are removed from the vessel holders such as 64. Some non-limiting examples of functions of the stations or devices 32, 34, and 36 include:

- placing the treated and inspected vessels 80 on a conveyor to further processing apparatus;
- adding chemicals to the vessels;
- capping the vessels;
- placing the vessels in suitable processing racks;
- packaging the vessels; and
- sterilizing the packaged vessels.

I. The vessel processing system 20 as illustrated in FIG. 1 also can include a multiplicity of vessel holders (or "pucks," as they can in some embodiments resemble a hockey puck) respectively 38 through 68, and a conveyor generally indicated as an endless band 70 for transporting one or more of the vessel holders 38-68, and thus vessels such as 80, to or from the processing stations 22, 24, 26, 28, 30, 32, 34, and 36.

I. The processing station or device 22 can be a device for forming the vessels 80. One contemplated device 22 can be an injection molding machine. Another contemplated device 22 can be a blow molding machine. Vacuum molding machines, draw molding machines, cutting or milling machines, glass drawing machines for glass or other draw-formable materials, or other types of vessel forming machines are also contemplated. Optionally, the vessel forming station 22 can be omitted, as vessels can be obtained already formed.

**II. VESSEL HOLDERS**

II.A. The portable vessel holders 38-68 are provided for holding and conveying a vessel having an opening while the vessel is processed. The vessel holder includes a vessel port, a second port, a duct, and a conveyable housing.

II.A. The vessel port is configured to seat a vessel opening in a mutually communicating relation. The second port is configured to receive an outside gas supply or vent. The duct is configured for passing one or more gases between a vessel opening seated on the vessel port and the second port. The vessel port, second port, and duct are attached in substantially rigid relation to the conveyable housing. Optionally, the portable vessel holder weighs less than five pounds. An advantage of a lightweight vessel holder is that it can more readily be transported from one processing station to another.

II.A. In certain embodiments of the vessel holder the duct more specifically is a vacuum duct and the second port more specifically is a vacuum port. The vacuum duct is configured for withdrawing a gas via the vessel port from a vessel seated on the vessel port. The vacuum port is configured for communicating between the vacuum duct and an outside source of vacuum. The vessel port, vacuum duct, and vacuum port can be attached in substantially rigid relation to the conveyable housing.

II.A. The vessel holders of embodiments II.A. and II.A.1. are shown, for example, in FIG. 2. The vessel holder 50 has a vessel port 82 configured to receive and seat the opening of a vessel 80. The interior surface of a seated vessel 80 can be processed via the vessel port 82. The vessel holder 50 can include a duct, for example a vacuum duct 94, for withdrawing a gas from a vessel 80 seated on the vessel port 92. The vessel holder can include a second port, for example a vacuum port 96 communicating between the vacuum duct 94 and an outside source of vacuum, such as the vacuum pump 98. The vessel port 92 and vacuum port 96 can have sealing elements, for example O-ring butt seals, respectively 100 and 102, or side seals between an inner or outer cylindrical wall of the vessel port 82 and an inner or outer cylindrical wall of the vessel 80 to receive and form a seal with the vessel 80 or outside source of vacuum 98 while allowing communication through the port. Gaskets or other sealing arrangements can or also be used.

II.A. The vessel holder such as 50 can be made of any material, for example thermoplastic material and/or electrically nonconductive material. Or, the vessel holder such as 50 can be made partially, or even primarily, of electrically conductive material and faced with electrically nonconductive material, for example in the passages defined by the vessel port 92, vacuum duct 94, and vacuum port 96. Examples of suitable materials for the vessel holder 50 are: a polyacetal, for example Delrin® acetal material sold by E. I. du Pont De Nemours and Company, Wilmington Delaware; polytetrafluoroethylene (PTFE), for example Teflon® PTFE sold by E. I. du Pont De Nemours and Company, Wilmington Delaware; Ultra-High-Molecular-Weight Polyethylene (UHMWPE); High density Polyethylene (HDPE); or other materials known in the art or newly discovered.

II.A. FIG. 2 also illustrates that the vessel holder, for example 50, can have a collar 116 for centering the vessel 80 when it is approaching or seated on the port 92.

**Array of Vessel Holders**

II.A. Yet another approach to treat, inspect, and/or move parts through a production system can be to use an array of vessel holders. The array can be comprised of individual pucks or be a solid array into which the devices are loaded. An array can allow more than one device, optionally many devices, to be tested, conveyed or treated/coated simultaneously. The array can be one-dimensional, for example grouped together to form a linear rack, or two-dimensional, similar to a tub or tray.

II.A. FIG. 14 shows an array approach. FIG. 14 shows a solid array 120 into (or onto) which the devices or vessels 80 are loaded. In this case, the devices or vessels 80 can move through the production process as a solid array, although they can be removed during the production process and transferred to individual vessel holders. A single vessel holder 120 has multiple vessel ports such as 122 for conveying an array of seated vessels such as 80, moving as a unit. In this embodiment, multiple individual vacuum ports such as 96 can be provided to receive an array of vacuum sources 98. Or, a single vacuum port connected to all the vessel ports such as 96 can be provided. Multiple gas inlet probes such as 108 can also be provided in an array. The arrays of gas inlet probes or vacuum sources can be mounted to move as a unit to process many vessels such as 80 simultaneously. Or, the multiple vessel ports such as 122 can be addressed one or more rows at a time, or individually, in a processing station. The number of devices in the array can be related to the number of devices that are molded in a single step or to other tests or steps that can allow for efficiency during the operation. In the case of treating/coating an array, the electrodes can either be coupled together (to form one large electrode), or can be individual electrodes each with its own power supply. All of the above approaches can still be applicable (from the standpoint of the electrode geometry, frequency etc.).

II.A. FIG. 14 shows a linear rack. If a linear rack is used, another option, in addition to those explained above, is to transport the rack in single file fashion through a processing station, processing the vessels serially.

**II.B. Vessel Holder Including O-ring Arrangement**

II.B. FIG. 11 is a construction for a vessel holder 482 usable, for example, with the embodiments of any other figure. The vessel holder 482 comprises an upper portion 484 and a base 486 joined together at a joint 488. A sealing element, for example an O-ring 490 (the right side of which is cut away to allow the pocket retaining it to be described) is captured between the upper portion 484 and the base 486 at the joint 488. In the illustrated embodiment, the O-ring 490 is received in an annular pocket 492 to locate the O-ring when the upper portion 484 is joined to the base 486.

II.B. In this embodiment, the O-ring 490 is captured and bears against a radially extending abutment surface 494 and the radially extending wall 496 partially defining the pocket 492 when the upper portion 484 and the base 486 are joined, in this case by the screws 498 and 500. The O-ring 490 thus seats between the upper portion 484 and base 486. The O-ring 490 captured between the upper portion 484 and the base 486 also receives the vessel 80 (removed in this figure for clarity of illustration of other features) and forms a first O-ring seal of the vessel port 502 about the vessel 80 opening.

II.B. In this embodiment, though not a requirement, the vessel port 502 has both the first O-ring 490 seal and a

second axially spaced O-ring 504 seal, each having an inner diameter such as 506 sized to receive the outer diameter of a vessel such as 80 for sealing between the vessel port 502 and a vessel such as 80. The spacing between the O-rings 490 and 504 provides support for a vessel such as 80 at two axially spaced points, preventing the vessel such as 80 from being skewed with respect to the O-rings 490 and 504 or the vessel port 502. In this embodiment, though not a requirement, the radially extending abutment surface 494 is located proximal of the O-ring 490 and 506 seals and surrounding the vacuum duct 508.

## III. METHODS FOR TRANSPORTING VESSELS - PROCESSING VESSELS SEATED ON VESSEL HOLDERS

### III.A. Transporting Vessel Holders To Processing Stations

III.A. FIGS. 1 and 2 show a method for processing a vessel 80. The method can be carried out as follows.

III.A. A vessel 80 can be provided having an opening 82 and a wall 86 defining an interior surface 88. As one embodiment, the vessel 80 can be formed in and then removed from a mold such as 22. Optionally within 60 seconds, or within 30 seconds, or within 25 seconds, or within 20 seconds, or within 15 seconds, or within 10 seconds, or within 5 seconds, or within 3 seconds, or within 1 second after removing the vessel from the mold, or as soon as the vessel 80 can be moved without distorting it during processing (assuming that it is made at an elevated temperature, from which it progressively cools), the vessel opening 82 can be seated on the vessel port 92. Quickly moving the vessel 80 from the mold 22 to the vessel port 92 reduces the dust or other impurities that can reach the surface 88 and occlude or prevent adhesion of the barrier or other type of coating 90. Also, the sooner a vacuum is drawn on the vessel 80 after it is made, the less chance any particulate impurities have of adhering to the interior surface 88.

III.A. A vessel holder such as 50 comprising a vessel port 92 can be provided. The opening 82 of the vessel 80 can be seated on the vessel port 92. Before, during, or after seating the opening 82 of the vessel 80 on the vessel port 92, the vessel holder such as 40 can be transported into engagement with one or more bearing surfaces to position the vessel holder 40 with respect to the processing device or station such as 24.

III.A. One, more than one, or all of the processing stations can include a bearing surface for supporting one or more vessel holders such as 40 in a predetermined position while processing the interior surface 88 of the seated vessel 80 at the processing station or device such as 24. These bearing surfaces can be part of stationary or moving structure, for example tracks or guides that guide and position the vessel holder such as 40 while the vessel is being processed. For example, the downward-facing bearing surfaces 222 and 224 locate the vessel holder 40 and act as a reaction surface to prevent the vessel holder 40 from moving upward when the probe 108 is being inserted into the vessel holder 40. The reaction surface 236 locates the vessel holder and prevents the vessel holder 40 from moving to the left while a vacuum source 98 (per FIG. 2) is seated on the vacuum port 96. The bearing surfaces 220, 226, 228, 232, 238, and 240 similarly locate the vessel holder 40 and prevent horizontal movement during processing. The bearing surfaces 230 and 234 similarly locate the vessel holder such as 40 and prevent it from moving vertically out of position. Thus, a first bearing surface, a second bearing surface, a third bearing surface, or more can be provided at each of the processing stations.

III.A. The interior surface 88 of the seated vessel 80 can be then processed via the vessel port 92 at the first processing station, which can be, as one example, the barrier application or other type of coating station 28 shown in FIG. 2. The vessel holder 50 and seated vessel 80 are transported from the first processing station 28 to the second processing station, for example the processing station 32. The interior surface 88 of the seated vessel 80 can be processed via the vessel port 92 at the second processing station such as 32.

III.A. Any of the above methods can include the further step of removing the vessel 80 from the vessel holder such as 66 following processing the interior surface 88 of the seated vessel 80 at the second processing station or device.

III.A. Any of the above methods can include the further step, after the removing step, of providing a second vessel 80 having an opening 82 and a wall 86 defining an interior surface 88. The opening 82 of the second vessel such as 80 can be seated on the vessel port 92 of another vessel holder such as 38. The interior surface of the seated second vessel 80 can be processed via the vessel port 92 at the first processing station or device such as 24. The vessel holder such as 38 and seated second vessel 80 can be transported from the first processing station or device 24 to the second processing station or device such as 26. The seated second vessel 80 can be processed via the

vessel port 92 by the second processing station or device 26.

### III.B. Transporting Processing Devices To Vessel Holders or vice versa.

III.B. Or, the processing stations can more broadly be processing devices, and either the vessel holders can be conveyed relative to the processing devices, the processing devices can be conveyed relative to the vessel holders, or some of each arrangement can be provided in a given system. In still another arrangement, the vessel holders can be conveyed to one or more stations, and more than one processing device can be deployed at or near at least one of the stations. Thus, there is not necessarily a one-to-one correspondence between the processing devices and processing stations.

III.B. A method including several parts is contemplated for processing a vessel. A first processing device such as the probe 108 (FIG. 2) and a second processing device such as a light source are provided for processing vessels such as 80. A vessel 80 is provided having an opening 82 and a wall 86 defining an interior surface 88. A vessel holder 50 is provided comprising a vessel port 92. The opening 82 of the vessel 80 is seated on the vessel port 92.

III.B. The first processing device such as the probe 108 is moved into operative engagement with the vessel holder 50, or vice versa. The interior surface 88 of the seated vessel 80 is processed via the vessel port 92 using the first processing device or probe 108.

III.B. The second processing device is then moved into operative engagement with the vessel holder 50, or vice versa. The interior surface 88 of the seated vessel 80 is processed via the vessel port 92 using the second processing device such as a light source.

III.B. Optionally, any number of additional processing steps can be provided. For example, a third processing device 34 can be provided for processing vessels 80. The third processing device 34 can be moved into operative engagement with the vessel holder 50, or vice versa. The interior surface of the seated vessel 80 can be processed via the vessel port 92 using the third processing device 34.

III.B. In another method for processing a vessel, the vessel 80 can be provided having an opening 82 and a wall 86 defining an interior surface 88. A vessel holder such as 50 comprising a vessel port 92 can be provided. The opening 82 of the vessel 80 can be seated on the vessel port 92. The interior surface 88 of the seated vessel 80 can be processed via the vessel port 92 at by the first processing device, which can be, as one example, the barrier or other type of coating device 28 shown in FIG. 2. The vessel holder 50 and seated vessel 80 are transported from the first processing device 28 to the second processing device, for example the processing device 34 shown in FIG. 1. The interior surface 88 of the seated vessel 80 can be then processed via the vessel port 92 by the second processing device such as 34.

## IV. PECVD APPARATUS FOR MAKING VESSELS

### IV.A. PECVD Apparatus Including Vessel Holder, Internal Electrode, Vessel As Reaction Chamber

IV.A. Another embodiment is a PECVD apparatus which is configured for performing the outgassing inspection, including a vessel holder, an inner electrode, an outer electrode, and a power supply. A vessel seated on the vessel holder defines a plasma reaction chamber, which optionally can be a vacuum chamber. Optionally, a source of vacuum, a reactant gas source, a gas feed or a combination of two or more of these can be supplied. Optionally, a gas drain, not necessarily including a source of vacuum, is provided to transfer gas to or from the interior of a vessel seated on the port to define a closed chamber.

IV.A. The PECVD apparatus can be used for atmospheric-pressure PECVD, in which case the plasma reaction chamber does not need to function as a vacuum chamber.

IV.A. In the embodiment illustrated in FIG. 2, the vessel holder 50 comprises a gas inlet port 104 for conveying a gas into a vessel seated on the vessel port. The gas inlet port 104 has a sliding seal provided by at least one O-ring 106, or two O-rings in series, or three O-rings in series, which can seat against a cylindrical probe 108 when the probe 108 is inserted through the gas inlet port 104. The probe 108 can be a gas inlet conduit that extends to a gas delivery port at its distal end 110. The distal end 110 of the illustrated embodiment can be inserted deep into the vessel 80 for providing one or more PECVD reactants and other process gases.

IV.A. Optionally in the embodiment illustrated in FIG. 2, or more generally in any embodiment disclosed, a plasma screen 610 can be provided to confine the plasma formed within the vessel 80 generally to the volume above the plasma screen 610. The plasma screen 610 is a conductive, porous material, several examples of which are steel wool, porous sintered metal or ceramic material coated with conductive material, or a foraminous plate or disk made of metal (for example brass) or other conductive material. An example is a pair of metal disks having central holes sized to pass the gas inlet 108 and having 0.02-inch (0.5 mm) diameter holes spaced 0.04 inches (1 mm) apart, center-to-center, the holes providing 22% open area as a proportion of the surface area of the disk.

IV.A. The plasma screen 610, for example for embodiments in which the probe 108 also functions as an counter electrode, can make intimate electrical contact with the gas inlet 108 at or near the opening 82 of the tube, syringe barrel, or other vessel 80 being processed. Alternatively, the plasma screen 610 can be grounded, optionally having a common potential with the gas inlet 108. The plasma screen 610 reduces or eliminates the plasma in the vessel holder 50 and its internal passages and connections, for example the vacuum duct 94, the gas inlet port 104, the vicinity of the O-ring 106, the vacuum port 96, the O-ring 102, and other apparatus adjacent to the gas inlet 108. At the same time, the porosity of the plasma screen allows process gases, air, and the like to flow out of the vessel 80 into the vacuum port 96 and downstream apparatus.

IV.A. FIG. 12 shows additional optional details of the coating station 28 that are usable, for example, with the embodiments of FIGS. 1, 2, 4-5, and 7. The coating station 28 can also have a main vacuum valve 574 in its vacuum line 576 leading to the pressure sensor 152. A manual bypass valve 578 is provided in the bypass line 580. A vent valve 582 controls flow.

IV.A. Flow out of the PECVD gas source 144 is controlled by a main reactant gas valve 584 regulating flow through the main reactant feed line 586. One component of the gas source 144 is the organosilicon liquid reservoir 588. The contents of the reservoir 588 are drawn through the organosilicon capillary line 590, which is provided at a suitable length to provide the desired flow rate. Flow of organosilicon vapor is controlled by the organosilicon shut-off valve 592. Pressure is applied to the headspace 614 of the liquid reservoir 588, for example a pressure in the range of 0-15 psi (0 to 78 cm. Hg), from a pressure source 616 such as pressurized air connected to the headspace 614 by a pressure line 618 to establish repeatable organosilicon liquid delivery that is not dependent on atmospheric pressure (and the fluctuations therein). The reservoir 588 is sealed and the capillary connection 620 is at the bottom of the reservoir 588 to ensure that only neat organosilicon liquid (not the pressurized gas from the headspace 614) flows through the capillary tube 590. The organosilicon liquid optionally can be heated above ambient temperature, if necessary or desirable to cause the organosilicon liquid to evaporate, forming an organosilicon vapor. Oxygen is provided from the oxygen tank 594 via an oxygen feed line 596 controlled by a mass flow controller 598 and provided with an oxygen shut-off valve 600.

IV.A. In the apparatus of FIG. 1, the vessel coating station 28 can be, for example, a PECVD apparatus as further described below, operated under suitable conditions to deposit a $SiO_x$ barrier or other type of coating 90 on the interior surface 88 of a vessel 80, as shown in FIG. 2.

IV.A. Referring especially to FIGS. 1 and 2, the processing station 28 can include an electrode 160 fed by a radio frequency power supply 162 for providing an electric field for generating plasma within the vessel 80 during processing. In this embodiment, the probe 108 is also electrically conductive and is grounded, thus providing a counter-electrode within the vessel 80. Alternatively, in any embodiment the outer electrode 160 can be grounded and the probe 108 directly connected to the power supply 162.

IV.A. In the embodiment of FIG. 2, the outer electrode 160 can either be generally cylindrical as illustrated in FIG 2 or a generally U-shaped elongated channel as illustrated in FIG. 2. Each illustrated embodiment has one or more sidewalls, such as 164 and 166, and optionally a top end 168, disposed about the vessel 80 in close proximity.

IV.A The electrode 160 shown in FIG. 2 can be shaped like a "U" channel with its length into the page and the puck or vessel holder 50 can move through the activated (powered) electrode during the treatment/coating process. Note that since external and internal electrodes are used, this apparatus can employ a frequency between 50 Hz and 1

GHz applied from a power supply 162 to the U channel electrode 160. The probe 108 can be grounded to complete the electrical circuit, allowing current to flow through the low-pressure gas(es) inside of the vessel 80. The current creates plasma to allow the selective treatment and/or coating of the interior surface 88 of the device.

IV.A The electrode in FIG. 2 can also be powered by a pulsed power supply. Pulsing allows for depletion of reactive gases and then removal of by-products prior to activation and depletion (again) of the reactive gases. Pulsed power systems are typically characterized by their duty cycle which determines the amount of time that the electric field (and therefore the plasma) is present. The power-on time is relative to the power-off time. For example a duty cycle of 10% can correspond to a power on time of 10% of a cycle where the power was off for 90% of the time. As a specific example, the power might be on for 0.1 second and off for 1 second. Pulsed power systems reduce the effective power input for a given power supply 162, since the off-time results in increased processing time. When the system is pulsed, the resulting coating can be very pure (no by products or contaminants). Another result of pulsed systems is the possibility to achieve atomic layer deposition (ALD). In this case, the duty cycle can be adjusted so that the power-on time results in the deposition of a single layer of a desired material. In this manner, a single atomic layer is contemplated to be deposited in each cycle. This approach can result in highly pure and highly structured coatings (although at the temperatures required for deposition on polymeric surfaces, temperatures optionally are kept low (<100°C) and the low-temperature coatings can be amorphous).

An alternative coating station employs a microwave cavity instead of an outer electrode. The energy applied can be a microwave frequency, e.g. 2.45 GHz. However, in the context of present invention, a radiofrequency is preferred.

## V. PECVD METHODS FOR MAKING COATINGS

### V.1 Precursors for PECVD Coating

The precursor for the PECVD coating of the present invention is broadly defined as an organometallic precursor. An organometallic precursor is defined in this specification as comprehending compounds of metal elements from Group III and/or Group IV of the Periodic Table having organic residues, e.g. hydrocarbon, aminocarbon or oxycarbon residues. Organometallic compounds as presently defined include any precursor having organic moieties bonded to silicon or other Group III/ IV metal atoms directly, or optionally bonded through oxygen or nitrogen atoms. The relevant elements of Group III of the Periodic Table are Boron, Aluminum, Gallium, Indium, Thallium, Scandium, Yttrium, and Lanthanum, Aluminum and Boron being preferred. The relevant elements of Group IV of the Periodic Table are Silicon, Germanium, Tin, Lead, Titanium, Zirconium, Hafnium, and Thorium, with Silicon and Tin being preferred. Other volatile organic compounds can also be contemplated. However, organosilicon compounds are preferred for performing present invention.

An organosilicon precursor is contemplated, where an "organosilicon precursor" is defined throughout this specification most broadly as a compound having at least one of the linkages:

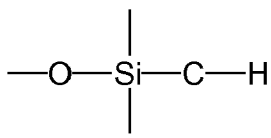

or

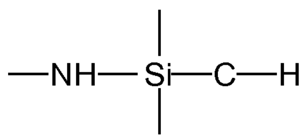

The first structure immediately above is a tetravalent silicon atom connected to an oxygen atom and an organic carbon atom (an organic carbon atom being a carbon atom bonded to at least one hydrogen atom). The second structure immediately above is a tetravalent silicon atom connected to an -NH- linkage and an organic carbon atom (an organic carbon atom being a carbon atom bonded to at least one hydrogen atom). Optionally, the organosilicon precursor is selected from the group consisting of a linear siloxane, a monocyclic siloxane, a polycyclic siloxane, a polysilsesquioxane, a linear silazane, a monocyclic silazane, a polycyclic silazane, a polysilsesquiazane, and a combination of any two or

more of these precursors. Also contemplated as a precursor, though not within the two formulas immediately above, is an alkyl trimethoxysilane.

If an oxygen-containing precursor (e.g. a siloxane) is used, a representative predicted empirical composition resulting from PECVD under conditions forming a hydrophobic or lubricating coating would be $Si_wO_xC_yH_z$ as defined in the Definition Section, while a representative predicted empirical composition resulting from PECVD under conditions forming a barrier coating would be $SiO_x$, where x in this formula is from about 1.5 to about 2.9. If a nitrogen-containing precursor (e.g. a silazane) is used, the predicted composition would be $Si_{w^*}N_{x^*}C_{y^*}H_{z^*}$, i.e. in $Si_wO_xC_yH_z$ as specified in the Definition Section, O is replaced by N and the indices are adapted to the higher valency of N as compared to O (3 instead of 2). The latter adaptation will generally follow the ratio of w, x, y and z in a siloxane to the corresponding indices in its aza counterpart. In a particular aspect of the invention, $Si_{w^*}N_{x^*}C_{y^*}H_{z^*}$ in which w*, x*, y*, and z* are defined the same as w, x, y, and z for the siloxane counterparts, but for an optional deviation in the number of hydrogen atoms.

One type of precursor starting material having the above empirical formula is a linear siloxane, for example a material having the following formula:

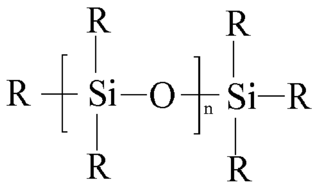

in which each R is independently selected from alkyl, for example methyl, ethyl, propyl, isopropyl, butyl, isobutyl, t-butyl, vinyl, alkyne, or others, and n is 1, 2, 3, 4, or greater, optionally two or greater. Several examples of contemplated linear siloxanes are

- hexamethyldisiloxane (HMDSO),
- octamethyltrisiloxane,
- decamethyltetrasiloxane,
- dodecamethylpentasiloxane,

or combinations of two or more of these. The analogous silazanes in which - NH- is substituted for the oxygen atom in the above structure are also useful for making analogous coatings. Several examples of contemplated linear silazanes are octamethyltrisilazane, decamethyltetrasilazane, or combinations of two or more of these.

V.C. Another type of precursor starting material is a monocyclic siloxane, for example a material having the following structural formula:

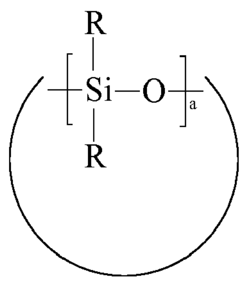

in which R is defined as for the linear structure and "a" is from 3 to about 10, or the analogous monocyclic silazanes. Several examples of contemplated hetero-substituted and unsubstituted monocyclic siloxanes and silazanes include

- 1,3,5-trimethyl-1,3,5-tris(3,3,3-trifluoropropyl)methyl]cyclotrisiloxane
- 2,4,6,8-tetramethyl-2,4,6,8-tetravinylcyclotetrasiloxane,
- pentamethylcyclopentasiloxane,
- pentavinylpentamethylcyclopentasiloxane,
- hexamethylcyclotrisiloxane,
- hexaphenylcyclotrisiloxane,
- octamethylcyclotetrasiloxane (OMCTS),
- octaphenylcyclotetrasiloxane,

- decamethylcyclopentasiloxane
- dodecamethylcyclohexasiloxane,
- methyl(3,3,3-trifluoropropl)cyclosiloxane,
- Cyclic organosilazanes are also contemplated, such as
- Octamethylcyclotetrasilazane,
- 1,3,5,7-tetravinyl-1,3,5,7-tetramethylcyclotetrasilazane hexamethylcyclotrisilazane,
- octamethylcyclotetrasilazane,
- decamethylcyclopentasilazane,
- dodecamethylcyclohexasilazane, or

combinations of any two or more of these.

V.C. Another type of precursor starting material is a polycyclic siloxane, for example a material having one of the following structural formulas:

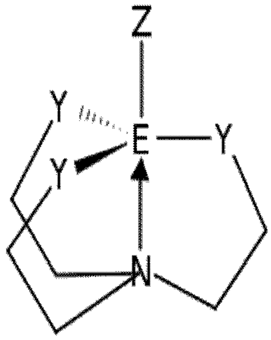
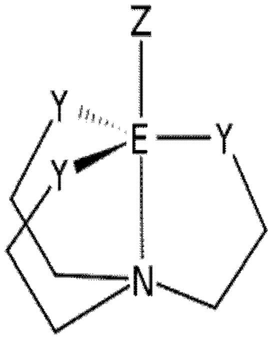
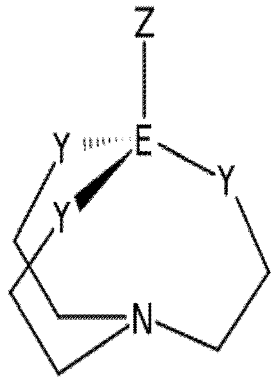

in which Y can be oxygen or nitrogen, E is silicon, and Z is a hydrogen atom or an organic substituent, for example alkyl such as methyl, ethyl, propyl, isopropyl, butyl, isobutyl, t-butyl, vinyl, alkyne, or others. When each Y is oxygen, the respective structures, from left to right, are a silatrane, a silquasilatrane, and a silproatrane. When Y is nitrogen, the respective structures are an azasilatrane, an azasilquasiatrane, and an azasilproatrane.

V.C. Another type of polycyclic siloxane precursor starting material is a polysilsesquioxane, with the empirical formula $RSiO_{1.5}$ and the structural formula:

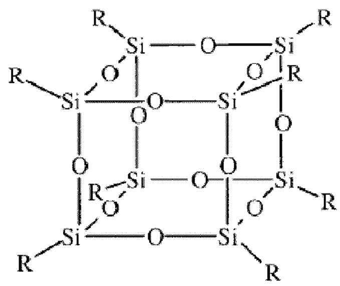

$T_8$ cube

in which each R is a hydrogen atom or an organic substituent, for example alkyl such as methyl, ethyl, propyl, isopropyl, butyl, isobutyl, t-butyl, vinyl, alkyne, or others. Two commercial materials of this sort are SST-eM01 poly(methylsilsesquioxane), in which each R is methyl, and SST-3MH1.1 poly(Methyl-Hydridosilsesquioxane), in which 90% of the R groups are methyl, 10% are hydrogen atoms. This material is available in a 10% solution in tetrahydrofuran, for example. Combinations of two or more of these are also contemplated. Other examples of a contemplated precursor are methylsilatrane, CAS No. 2288-13-3, in which each Y is oxygen and Z is methyl, methylazasilatrane, poly(methylsilsesquioxane) (e.g. SST-eM01 poly(methylsilsesquioxane)), in which each R optionally can be methyl, SST-3MH1.1 poly(Methyl-Hydridosilsesquioxane) (e.g. SST-3MH1.1 poly(Methyl-Hydridosilsesquioxane)), in which 90% of the R groups are methyl and 10% are hydrogen atoms, or a combination of any two or more of these.

V.C. The analogous polysilsesquiazanes in which -NH- is substituted for the oxygen atom in the above structure are also useful for making analogous coatings. Examples of contemplated polysilsesquiazanes are a poly(methylsilsesquiazane), in which each R is methyl, and a poly(Methyl-Hydridosilsesquiazane, in which 90% of the R groups are methyl, 10% are hydrogen atoms. Combinations of two or more of these are also contemplated.

V.C. One particularly contemplated precursor for the lubricity layer according to the present invention is a monocyclic siloxane, for example is octamethylcyclotetrasiloxane.
One particularly contemplated precursor for the hydrophobic layer according to the present invention is a monocyclic siloxane, for example is octamethylcyclotetrasiloxane.
One particularly contemplated precursor for the barrier coating according to the present invention is a linear siloxane, for example is HMDSO.

V.C. In any of the coating methods according to the present invention, the applying step optionally can be carried out by vaporizing the precursor and providing it in the vicinity of the substrate. E.g., OMCTS is usually vaporized by heating it to about 50°C before applying it to the PECVD apparatus.

**V.2 General PECVD Method**

In the context of the present invention, the following PECVD method is generally applied, which contains the following steps:

(a) providing a process gas comprising a precursor as defined herein (typically an organosilicon precursor), optionally a carrier gas, optionally $O_2$, and optionally a hydrocarbon; and

(b) generating a plasma from the process gas, thus forming a coating on the substrate surface by plasma enhanced chemical vapor deposition (PECVD).

The plasma coating technology used herein is based on Plasma Enhanced Chemical Vapor Deposition (PECVD). Methods and apparatus suitable to perform said PECVD coatings are described in EP10162755.2 filed May 12, 2010; EP10162760.2 filed May 12, 2010; EP10162756.0 filed May 12, 2010; EP10162758.6 filed May 12, 2010; EP10162761.0 filed May 12, 2010; and EP10162757.8 filed May 12, 2010. The PECVD methods and apparatus as described therein are suitable to perform the present invention and are therefore incorporated herein by reference.

An exemplary preferred embodiment of the PECVD technology will be described in the following sections.

The process utilizes a silicon containing vapor that can be combined with oxygen at reduced pressures (mTorr range - atmospheric pressure is 760 Torr) inside a container.

An electrical field generated at, e.g., 13.56 MHz [radio frequency range] is then applied between an external electrode and an internal grounded gas inlet to create a plasma. At the pressures and powers that are used to coat a container, the plasma process is driven by electron impact ionization, which means the electrons in the process are the driving force behind the chemistry. Specifically, the plasma drives the chemical reaction through electron impact ionization of the silicon containing material [e.g., hexamethyldisiloxane (HMDSO) or other reactants like octamethylcyclotretrasiloxane (OMCTS)] resulting in a silicon dioxide or $Si_wO_xC_yH_z$ coating deposited onto the interior surfaces of the container. These coatings are in a typical embodiment on the order of 20 or more nanometers in thickness. HMDSO consists of an Si-O-Si backbone with six (6) methyl groups attached to the silicon atoms. The process breaks the Si-C bonds and (at the surface of the tube or syringe) reacts with oxygen to create silicon dioxide. Since the coating is grown on an atomic basis, dense, conformal coatings with thicknesses of 20-30 nanometers can achieve significant barrier properties. The silicon oxide acts as a physical barrier to gases, moisture, and small organic molecules, and is of greater purity than commercial glasses. OMCTS results in coatings with lubricity or anti-adhesion properties. Their average thickness is generally higher than the thickness of the $SiO_x$ barrier coating, e.g. from 30 to 1000 nm on average.

The technology is unique in several aspects:

(a) The process utilizes the rigid container as the vacuum chamber. PECVD conventionally uses a secondary vacuum vessel into which the part(s) are loaded and coated. Utilizing the container as a vacuum chamber significantly simplifies the process apparatus and reduces cycle/processing time, and thus manufacturing cost and capital. This approach also reduces scale-up issues since scale-up is as simple as replicating the number of tubes or syringes required to meet the throughput requirements.

(b) Radio Frequency excitation of the plasma allows energy to be imparted to the ionized gas with little heating of the part. Unlike microwave excitation energies, typically used in PECVD, which will impart significant energy to water molecules in the part itself, radio frequency will not preferentially heat the polymeric tubes or syringes. Controlled heat absorption is critical to prevent substrate temperature increases approaching plastic glass transition temperatures, causing loss of dimensional integrity (collapse under vacuum).

(c) Single layer gas barrier coating - the new technology can generate a single layer of silicon dioxide directly on the interior surface of the part. Most other barrier technologies (thin film) require at least two layers.

(d) Combination barrier-lubricity coatings - the new technology utilizes a combination $SiO_x$ / $Si_wO_xC_yH_z$ coating to provide multiple performance attributes (barrier/lubricity).

The plasma deposition technology in a preferred aspect utilizes a simple manufacturing configuration. The system is based on a "puck," which is used in transportation of tubes and syringes in and out of the coating station. The device-puck interface (see Figure 6) is critical, since once coating/characterization conditions are established at the pilot scale, there are no scaling issues when moving to full scale production; one simply increases the number of pucks through the same process. The puck is manufactured from a polymeric material (e.g. Delrin™) to provide an electrically insulated base. The container (e.g. a tabe as in Fig. 6) is mounted into the puck with the largest opening sealing against an o-ring (mounted in the puck itself). The o-ring provides the vacuum seal between the part and the puck so that the ambient air (principally nitrogen and oxygen with some water vapor) can be removed (pressure reduced) and the process gases introduced. The puck has several key features in addition to the o-ring seal. The puck provides a means of connection to the vacuum pump (which pumps away the atmospheric gases and the by-products of the silicon dioxide reaction), a means of accurately aligning the gas inlet in the part, and a means of providing a vacuum seal between the puck and gas inlet.

For $SiO_2$ deposition, HMDSO and oxygen gases are then admitted into the container through the grounded gas inlet which extends up into the part. At this point, the puck and container are moved into the electrode area. The electrode is constructed from a conductive material (for example copper) and provides a tunnel through which the part passes. The electrode does not make physical contact with the container or the puck and is supported independently. An RF impedance matching network and power supply are connected directly to the electrode. The power supply provides energy (at 13.56 MHz) to the impedance matched network. The RF matching network acts to match the output impedance of the power supply to the complex (capacitive and inductive) impedance of the ionized gases. The matching network delivers maximum power delivery to the ionized gas which ensures deposition of the silicon dioxide coating.

Once the container is coated (as the puck moves the container through the electrode channel - which is stationary), the gases are stopped and atmospheric air (or pure nitrogen) is allowed inside the puck/container to bring it back to atmospheric pressure. At this time, the container can be removed from the puck and moved to the next processing station.

The above describes clearly the means of coating a container having just one opening. Syringes require an additional step before and after loading onto the puck. Since the syringes have openings at both ends (one for connection to a needle and the second for installation of a plunger), the needle end must be sealed prior to coating. The above process allows reaction gases to be admitted into the plastic part interior, an electrical current to pass through the gas inside of the part and a plasma to be established inside the part. The plasma (an ionized composition of the HMDSO or OMCTS and oxygen gases) is what drives the chemistry and the deposition of the plasma coating.

In the method, the coating characteristics are advantageously set by one or more of the following conditions: the plasma properties, the pressure under which the plasma is applied, the power applied to generate the plasma, the presence and relative amount of $O_2$ in the gaseous reactant, the plasma volume, and the organosilicon precursor. Optionally, the coating characteristics are set by the presence and relative amount of $O_2$ in the gaseous reactant and/or the power applied to generate the plasma.

In all embodiments of the present invention, the plasma is in an optional aspect a non-hollow-cathode plasma when an $SiO_x$ coating is prepared.

In a further preferred aspect, the plasma is generated at reduced pressure (as compared to the ambient or atmospheric pressure). Optionally, the reduced pressure is less than 300 mTorr, optionally less than 200 mTorr, even optionally less than 100 mTorr.

The PECVD optionally is performed by energizing the gaseous reactant containing the precursor with electrodes powered at a frequency at microwave or radio frequency, and optionally at a radio frequency. The radio frequency preferred to perform an embodiment of the invention will also be addressed as "RF frequency". A typical radio frequency range for performing the present invention is a frequency of from 10 kHz to less than 300 MHz, optionally from 1 to 50 MHz, even optionally from 10 to 15 MHz. A frequency of 13.56 MHz is most preferred, this being a government sanctioned frequency for conducting PECVD work.

There are several advantages for using a RF power source versus a microwave source: Since RF operates a lower power, there is less heating of the substrate/vessel. Because the focus of the present invention is putting a plasma coating on plastic substrates, lower processing temperature are desired to prevent melting/distortion of the substrate. To prevent substrate overheating when using microwave PECVD, the microwave PECVD is applied in short bursts, by pulsing the power. The power pulsing extends the cycle time for the coating, which is undesired in the present invention. The higher frequency microwave can also cause offgassing of volatile substances like residual water, oligomers and other materials in the plastic substrate. This offgassing can interfere with the PECVD coating. It can also interfere with the outgassing measurement of present invention, as it will change the content and composition of volatile compounds in the coated substrate. A major concern with using microwave for PECVD is delamination of the coating from the substrate. Delamination occurs because the microwaves change the surface of the substrate prior to depositing the coating layer. To mitigate the possibility of delamination, interface coating layers have been developed for microwave PECVD to achieve good bonding between the coating and the substrate. No such interface coating layer is needed with RF PECVD as there is no risk of delamination. Finally, the lubricity layer and hydrophobic layer according to the present invention are advantageously applied using lower power. RF power operates at lower power and provides more control over the PECVD process than microwave power. Nonetheless, microwave power, though less preferred, is usable under suitable process conditions.

Furthermore, for all PECVD methods described herein, there is a specific correlation between the power (in Watts) used to generate the plasma and the volume of the lumen wherein the plasma is generated. Typically, the lumen is the lumen of a vessel coated according to the present invention. The RF power should scale with the volume of the vessel if the same electrode system is employed. Once the composition of a gaseous reactant, for example the ratio of the precursor to $O_2$, and all other parameters of the PECVD coating method but the power have been set, they will typically not change when the geometry of a vessel is maintained and only its volume is varied. In this case, the power will be directly proportional to the volume. Thus, starting from the power to volume ratios provided by present description, the power which has to be applied in order to achieve the same or a similar coating in a vessel of same geometry, but different size, can easily be found. The influence of the vessel geometry on the power to be applied is illustrated by the results of the Examples for tubes in comparison to the Examples for syringe barrels.

For any coating of the present invention, the plasma is generated with electrodes powered with sufficient power to form a coating on the substrate surface. For a lubricity coating or hydrophobic coating, in the method according to an embodiment of the invention the plasma is optionally generated

(i) with electrodes supplied with an electric power of from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W.; and/or (ii) wherein the ratio of the electrode power to the plasma volume is less than 10 W/ml, optionally is from 6 W/ml to 0.1 W/ml, optionally is from 5 W/ml to 0.1 W/ml, optionally is from 4 W/ml to 0.1 W/ml, optionally is from 3 W/ml to 0.2 W/ml, optionally is from 2 W/ml to 0.2 W/ml.

Low power levels are believed by the inventors to be most advantageous (e.g. power levels of from 2 to 3.5 W and the power levels given in the Examples) to prepare a lubricity coating.

For a barrier coating or $SiO_x$ coating, the plasma is optionally generated

(i) with electrodes supplied with an electric power of from 8 to 500 W, optionally from 20 to 400 W, optionally from 35 to 350 W, even optionally from 44 to 300 W, optionally from 44 to 70 W; and/or

(ii) the ratio of the electrode power to the plasma volume is equal or more than 5 W/ml, optionally is from 6 W/ml to 150 W/ml, optionally is from 7 W/ml to 100 W/ml, optionally from 7 W/ml to 20 W/ml.

The vessel geometry can also influence the choice of the gas inlet used for the PECVD coating. In a particular aspect, a syringe can be coated with an open tube inlet, and a tube can be coated with a gas inlet having small holes which is extended into the tube.

The power (in Watts) used for PECVD also has an influence on the coating properties. Typically, an increase of the power will increase the barrier properties of the coating, and a decrease of the power will increase the lubricity and hydrophobicity of the coating. E.g., for a coating on the inner wall of a syringe barrel having a volume of about 3 ml, a power of less than 30 W will lead to a coating which is predominantly a lubricity coating, while a power of more than 30 W will lead to a coating which is predominantly a barrier coating (see Examples). This is also demonstrated in the Examples of EP 2 251 455 A2, to which explicit reference is made herewith.

A further parameter determining the coating properties is the ratio of $O_2$ (or another oxidizing agent) to the precursor (e.g. organosilicon precursor) in the gaseous reactant used for generating the plasma. Typically, an increase of the $O_2$ ratio in the gaseous reactant will increase the barrier properties of the coating, and a decrease of the $O_2$ ratio

will increase the lubricity and hydrophobicity of the coating.
If a lubricity layer or coating is desired, then $O_2$ is optionally present in a volume-volume ratio to the gaseous reactant of from 0:1 to 5:1, optionally from 0:1 to 1:1, even optionally from 0:1 to 0.5:1 or even from 0:1 to 0.1:1. It is preferred that some $O_2$ is present, optionally in an amount of from 0.01:1 to 0.5:1, even optionally from 0.05:1 to 0.4:1, in particular from 0.1:1 to 0.2:1 in relation to the organosilicon precursor. The presence of $O_2$ in a volume of about 5 % to about 35 % (v/v in sccm) in relation to the organosilicon precursor, in particular of about 10 % to about 20 % and in a ratio as given in the Examples is specifically suitable to achieve a lubricity coating.

The same applies to a hydrophobic layer.

If, on the other hand, a barrier or $SiO_x$ coating is desired, then the $O_2$ is optionally present in a volume : volume ratio to the gaseous reactant of from 1 : 1 to 100 : 1 in relation to the silicon containing precursor, optionally in a ratio of from 5 : 1 to 30 : 1, optionally in a ratio of from 10 : 1 to 20 : 1, even optionally in a ratio of 15 : 1.

### V.A. PECVD to apply $SiO_x$ barrier coating, using plasma that is substantially free of hollow cathode plasma

V.A. A specific embodiment is a method of applying a barrier coating of $SiO_x$, defined in this specification (unless otherwise specified in a particular instance) as a coating containing silicon, oxygen, and optionally other elements, in which x, the ratio of oxygen to silicon atoms, is from about 1.5 to about 2.9, or 1.5 to about 2.6, or about 2. These alternative definitions of x apply to any use of the term $SiO_x$ in this specification. The barrier coating is applied to the interior of a vessel, for example a sample collection tube, a syringe barrel, or another type of vessel. The method includes several steps.

V.A. A vessel wall is provided, as is a reaction mixture comprising plasma forming gas, i.e. an organosilicon compound gas, optionally an oxidizing gas, and optionally a hydrocarbon gas.

V.A. Plasma is formed in the reaction mixture that is substantially free of hollow cathode plasma. The vessel wall is contacted with the reaction mixture, and the coating of $SiO_x$ is deposited on at least a portion of the vessel wall.

V.A. In certain embodiments, the generation of a uniform plasma throughout the portion of the vessel to be coated is contemplated, as it has been found in certain instances to generate an $SiO_x$ coating providing a better barrier against oxygen. Uniform plasma means regular plasma that does not include a substantial amount of hollow cathode plasma (which has a higher emission intensity than regular plasma and is manifested as a localized area of higher intensity interrupting the more uniform intensity of the regular plasma).

V.A. The hollow cathode effect is generated by a pair of conductive surfaces opposing each other with the same negative potential with respect to a common anode. If the spacing is made (depending on the pressure and gas type) such that the space charge sheaths overlap, electrons start to oscillate between the reflecting potentials of the opposite wall sheaths leading to multiple collisions as the electrons are accelerated by the potential gradient across the sheath region. The electrons are confined in the space charge sheath overlap which results in very high ionization and high ion density plasmas. This phenomenon is described as the hollow cathode effect. Those skilled in the art are able to vary the processing conditions, such as the power level and the feed rates or pressure of the gases, to form uniform plasma throughout or to form plasma including various degrees of hollow cathode plasma.

V.A. The plasma is typically generated using RF energy for the reasons given above. In an alternate, but less typical method, microwave energy can be used to generate the plasma in a PECVD process. The processing conditions can be different, however, as microwave energy applied to a thermoplastic vessel will excite (vibrate) water molecules. Since there is a small amount of water in all plastic materials, the microwaves will heat the plastic. As the plastic heats, the large driving force created by the vacuum inside of the device relative to atmospheric pressure outside the device will pull free or easily desorb materials to the interior surface 88 where they will either become volatile or will be weakly bound to the surface. The weakly bound materials will then create an interface that can hinder subsequent coatings (deposited from the plasma) from adhering to the plastic interior surface 88 of the device.

V.A. As one way to negate this coating hindering effect, a coating can be deposited at very low power (in the example above 5 to 20 Watts at 2.45 GHz) creating a cap onto which subsequent coatings can adhere. This results in a two-step coating process (and two coating layers). In the example above, the initial gas flows (for the capping layer) can be changed to 2 sccm ("standard cubic centimeters per minute") HMDSO and 20 sccm oxygen with a process power of 5 to 20 Watts for approximately 2-10 seconds. Then the gases can be adjusted to the flows in the example above

and the power level increased to 20-50 Watts so that an $SiO_x$ coating, in which x in this formula is from about 1.5 to about 2.9, alternatively from about 1.5 to about 2.6, alternatively about 2, can be deposited. Note that the capping layer might provide little to no functionality in certain embodiments, except to stop materials from migrating to the vessel interior surface 88 during the higher power $SiO_x$ coating deposition. Note also that migration of easily desorbed materials in the device walls typically is not an issue at lower frequencies such as most of the RF range, since the lower frequencies do not excite (vibrate) molecular species.

V.A. As another way to negate the coating hindering effect described above, the vessel 80 can be dried to remove embedded water before applying microwave energy. Desiccation or drying of the vessel 80 can be accomplished, for example, by thermally heating the vessel 80, as by using an electric heater or forced air heating. Desiccation or drying of the vessel 80 also can be accomplished by exposing the interior of the vessel 80, or gas contacting the interior of the vessel 80, to a desiccant. Other expedients for drying the vessel, such as vacuum drying, can also be used. These expedients can be carried out in one or more of the stations or devices illustrated or by a separate station or device.

V.A. Additionally, the coating hindering effect described above can be addressed by selection or processing of the resin from which the vessels 80 are molded to minimize the water content of the resin.

**V.B. PECVD Coating Restricted Opening of Vessel (Syringe Capillary)**

V.B. FIGS. 4 and 5 show a method and apparatus generally indicated at 290 for coating an inner surface 292 of a restricted opening 294 of a generally tubular vessel 250 to be processed, for example the restricted front opening 294 of a syringe barrel 250, by PECVD. The previously described process is modified by connecting the restricted opening 294 to a processing vessel 296 and optionally making certain other modifications.

V.B. The generally tubular vessel 250 to be processed includes an outer surface 298, an inner or interior surface 254 defining a lumen 300, a larger opening 302 having an inner diameter, and a restricted opening 294 that is defined by an inner surface 292 and has an inner diameter smaller than the inner diameter of the larger opening 302.

V.B. The processing vessel 296 has a lumen 304 and a processing vessel opening 306, which optionally is the only opening, although in other embodiments a second opening can be provided that optionally is closed off during processing. The processing vessel opening 306 is connected with the restricted opening 294 of the vessel 250 to be processed to establish communication between the lumen 300 of the vessel 250 to be processed and the processing vessel lumen via the restricted opening 294.

V.B. At least a partial vacuum is drawn within the lumen 300 of the vessel 250 to be processed and lumen 304 of the processing vessel 296. A PECVD reactant is flowed from the gas source 144 through the first opening 302, then through the lumen 300 of the vessel 250 to be processed, then through the restricted opening 294, then into the lumen 304 of the processing vessel 296.

V.B. The PECVD reactant can be introduced through the larger opening 302 of the vessel 250 by providing a generally tubular inner electrode 308 having an interior passage 310, a proximal end 312, a distal end 314, and a distal opening 316, in an alternative embodiment multiple distal openings can be provided adjacent to the distal end 314 and communicating with the interior passage 310. The distal end of the electrode 308 can be placed adjacent to or into the larger opening 302 of the vessel 250 to be processed. A reactant gas can be fed through the distal opening 316 of the electrode 308 into the lumen 300 of the vessel 250 to be processed. The reactant will flow through the restricted opening 294, then into the lumen 304, to the extent the PECVD reactant is provided at a higher pressure than the vacuum initially drawn before introducing the PECVD reactant.

V.B. Plasma 318 is generated adjacent to the restricted opening 294 under conditions effective to deposit a coating of a PECVD reaction product on the inner surface 292 of the restricted opening 294. In the embodiment shown in FIG. 4, the plasma is generated by feeding RF energy to the generally U-shaped outer electrode 160 and grounding the inner electrode 308. The feed and ground connections to the electrodes could also be reversed, though this reversal can introduce complexity if the vessel 250 to be processed, and thus also the inner electrode 308, are moving through the U-shaped outer electrode while the plasma is being generated.

V.B. The plasma 318 generated in the vessel 250 during at least a portion of processing can include hollow cathode plasma generated inside the restricted opening 294 and/or the processing vessel lumen 304. The generation of hollow cathode plasma 318 can contribute to the ability to successfully apply a barrier coating at the restricted opening 294, although the invention is not limited according to the accuracy or applicability of this theory of operation. Thus, in one contemplated mode of operation, the processing can be carried out partially under conditions generating a uniform plasma throughout the vessel 250 and the gas inlet, and partially under conditions generating a hollow cathode plasma, for example adjacent to the restricted opening 294.

V.B. The process is desirably operated under such conditions, as explained here and shown in the drawings, that the plasma 318 extends substantially throughout the syringe lumen 300 and the restricted opening 294. The plasma 318 also desirably extends substantially throughout the syringe lumen 300, the restricted opening 294, and the lumen 304 of the processing vessel 296. This assumes that a uniform coating of the interior 254 of the vessel 250 is desired. In other embodiments non-uniform plasma can be desired.

V.B. It is generally desirable that the plasma 318 have a substantially uniform color throughout the syringe lumen 300 and the restricted opening 294 during processing, and optionally a substantially uniform color substantially throughout the syringe lumen 300, the restricted opening 294, and the lumen 304 of the processing vessel 296. The plasma desirably is substantially stable throughout the syringe lumen 300 and the restricted opening 294, and optionally also throughout the lumen 304 of the processing vessel 296.

V.B. The order of steps in this method is not contemplated to be critical.

V.B. In the embodiment of FIGS. 4 and 5, the restricted opening 294 has a first fitting 332 and the processing vessel opening 306 has a second fitting 334 adapted to seat to the first fitting 332 to establish communication between the lumen 304 of the processing vessel 296 and the lumen 300 of the vessel 250 to be processed.

V.B. In the embodiment of FIGS. 4 and 5, the first and second fittings are male and female Luer lock fittings 332 and 334, respectively integral with the structure defining the restricted opening 294 and the processing vessel opening 306. One of the fittings, in this case the male Luer lock fitting 332, comprises a locking collar 336 with a threaded inner surface and defining an axially facing, generally annular first abutment 338 and the other fitting 334 comprises an axially facing, generally annular second abutment 340 facing the first abutment 338 when the fittings 332 and 334 are engaged.

V.B. In the illustrated embodiment a seal, for example an O-ring 342 can be positioned between the first and second fittings 332 and 334. For example, an annular seal can be engaged between the first and second abutments 338 and 340. The female Luer fitting 334 also includes dogs 344 that engage the threaded inner surface of the locking collar 336 to capture the O-ring 342 between the first and second fittings 332 and 334. Optionally, the communication established between the lumen 300 of the vessel 250 to be processed and the lumen 304 of the processing vessel 296 via the restricted opening 294 is at least substantially leak proof.

V.B. As a further option, either or both of the Luer lock fittings 332 and 334 can be made of electrically conductive material, for example stainless steel. This construction material forming or adjacent to the restricted opening 294 might contribute to formation of the plasma in the restricted opening 294.

V.B. The desirable volume of the lumen 304 of the processing vessel 296 is contemplated to be a trade-off between a small volume that will not divert much of the reactant flow away from the product surfaces desired to be coated and a large volume that will support a generous reactant gas flow rate through the restricted opening 294 before filling the lumen 304 sufficiently to reduce that flow rate to a less desirable value (by reducing the pressure difference across the restricted opening 294). The contemplated volume of the lumen 304, in an embodiment, is less than three times the volume of the lumen 300 of the vessel 250 to be processed, or less than two times the volume of the lumen 300 of the vessel 250 to be processed, or less than the volume of the lumen 300 of the vessel 250 to be processed, or less than 50% of the volume of the lumen 300 of the vessel 250 to be processed, or less than 25% of the volume of the lumen 300 of the vessel 250 to be processed. Other effective relationships of the volumes of the respective lumens are also contemplated.

V.B. The inventors have found that the uniformity of coating can be improved in certain embodiments by repositioning the distal end of the electrode 308 relative to the vessel 250 so it does not penetrate as far into the lumen 300 of the vessel 250 as the position of the inner electrode shown in previous Figures. For example, although in certain

embodiments the distal opening 316 can be positioned adjacent to the restricted opening 294, in other embodiments the distal opening 316 can be positioned less than 7/8 the distance, optionally less than ¾ the distance, optionally less than half the distance to the restricted opening 294 from the larger opening 302 of the vessel to be processed while feeding the reactant gas. Or, the distal opening 316 can be positioned less than 40%, less than 30%, less than 20%, less than 15%, less than 10%, less than 8%, less than 6%, less than 4%, less than 2%, or less than 1% of the distance to the restricted opening 294 from the larger opening of the vessel to be processed while feeding the reactant gas.

V.B. Or, the distal end of the electrode 308 can be positioned either slightly inside or outside or flush with the larger opening 302 of the vessel 250 to be processed while communicating with, and feeding the reactant gas to, the interior of the vessel 250. The positioning of the distal opening 316 relative to the vessel 250 to be processed can be optimized for particular dimensions and other conditions of treatment by testing it at various positions. One particular position of the electrode 308 contemplated for treating syringe barrels 250 is with the distal end 314 penetrating about a quarter inch (about 6 mm) into the vessel lumen 300 above the larger opening 302.

V.B. The inventors presently contemplate that it is advantageous to place at least the distal end 314 of the electrode 308 within the vessel 250 so it will function suitably as an electrode, though that is not necessarily a requirement. Surprisingly, the plasma 318 generated in the vessel 250 can be made more uniform, extending through the restricted opening 294 into the processing vessel lumen 304, with less penetration of the electrode 308 into the lumen 300 than has previously been employed. With other arrangements, such as processing a closed-ended vessel, the distal end 314 of the electrode 308 commonly is placed closer to the closed end of the vessel than to its entrance.

V.B. Or, the distal end 314 of the electrode 308 can be positioned at the restricted opening 294 or beyond the restricted opening 294, for example within the processing vessel lumen 304, as illustrated for example in FIG. 33. Various expedients can optionally be provided, such as shaping the processing vessel 296 to improve the gas flow through the restricted opening 294.

V.B. As another alternative, the composite inner electrode and gas supply tube can have distal gas supply openings, optionally located near the larger opening 302, and an extension electrode extending distal of the distal gas supply openings, optionally extending to a distal end adjacent to the restricted opening 294, and optionally further extending into the processing vessel. This construction is contemplated to facilitate formation of plasma within the inner surface 292 adjacent to the restricted opening 294.

V.B. In yet another contemplated embodiment, the inner electrode 308, as in FIG. 4, can be moved during processing, for example, at first extending into the processing vessel lumen 304, then being withdrawn progressively proximally as the process proceeds. This expedient is particularly contemplated if the vessel 250, under the selected processing conditions, is long, and movement of the inner electrode facilitates more uniform treatment of the interior surface 254. Using this expedient, the processing conditions, such as the gas feed rate, the vacuum draw rate, the electrical energy applied to the outer electrode 160, the rate of withdrawing the inner electrode 308, or other factors can be varied as the process proceeds, customizing the process to different parts of a vessel to be treated.

V.B. Conveniently, as in the other processes described in this specification, the larger opening of the generally tubular vessel 250 to be processed can be placed on a vessel support, as by seating the larger opening 302 of the vessel 250 to be processed on a port of the vessel support. Then the inner electrode 308 can be positioned within the vessel 250 seated on the vessel support before drawing at least a partial vacuum within the lumen 300 of the vessel 250 to be processed.

### V.C. Method of Applying a Lubricity Layer

V.C. Another embodiment is a method of applying a lubricity layer derived from an organosilicon precursor. This method and the lubricity coatings and coated items are also described in PCT/US11/36097 filed on 11 May 2011. Explicit reference is made herewith to said application in prder to point out such methods, coatings and coated items and their features.

A "lubricity layer" or any similar term is generally defined as a coating that reduces the frictional resistance of the coated surface, relative to the uncoated surface. If the coated object is a syringe (or syringe part, e.g. syringe barrel) or any other item generally containing a plunger or movable part in sliding contact with the coated surface, the frictional resistance has two main aspects - breakout force and plunger sliding force.

The plunger sliding force test is a specialized test of the coefficient of sliding friction of the plunger within a

syringe, accounting for the fact that the normal force associated with a coefficient of sliding friction as usually measured on a flat surface is addressed by standardizing the fit between the plunger or other sliding element and the tube or other vessel within which it slides. The parallel force associated with a coefficient of sliding friction as usually measured is comparable to the plunger sliding force measured as described in this specification. Plunger sliding force can be measured, for example, as provided in the ISO 7886-1:1993 test.

The plunger sliding force test can also be adapted to measure other types of frictional resistance, for example the friction retaining a stopper within a tube, by suitable variations on the apparatus and procedure. In one embodiment, the plunger can be replaced by a closure and the withdrawing force to remove or insert the closure can be measured as the counterpart of plunger sliding force.

Also or instead of the plunger sliding force, the breakout force can be measured. The breakout force is the force required to start a stationary plunger moving within a syringe barrel, or the comparable force required to unseat a seated, stationary closure and begin its movement. The breakout force is measured by applying a force to the plunger that starts at zero or a low value and increases until the plunger begins moving. The breakout force tends to increase with storage of a syringe, after the prefilled syringe plunger has pushed away the intervening lubricant or adhered to the barrel due to decomposition of the lubricant between the plunger and the barrel. The breakout force is the force needed to overcome "sticktion," an industry term for the adhesion between the plunger and barrel that needs to be overcome to break out the plunger and allow it to begin moving.

V.C. Some utilities of coating a vessel in whole or in part with a lubricity layer, such as selectively at surfaces contacted in sliding relation to other parts, is to ease the insertion or removal of a stopper or passage of a sliding element such as a piston in a syringe or a stopper in a sample tube. The vessel can be made of glass or a polymer material such as polyester, for example polyethylene terephthalate (PET), a cyclic olefin copolymer (COC), an olefin such as polypropylene, or other materials. COC is particularly suitable for syringes and syringe barrels. Applying a lubricity layer by PECVD can avoid or reduce the need to coat the vessel wall or closure with a sprayed, dipped, or otherwise applied organosilicon or other lubricant that commonly is applied in a far larger quantity than would be deposited by a PECVD process.

V.C. In any of the above embodiments V.C., a plasmais formed in the vicinity of the substrate

In any of embodiments V.C. the precursor optionally can be provided in the substantial absence of nitrogen.
V.C. In any of embodiments V.C., the precursor optionally can be provided at less than 1 Torr absolute pressure.
V.C. In any of embodiments V.C., the precursor optionally can be provided to the vicinity of a plasma emission.
V.C. In any of embodiments V.C., the coating optionally can be applied to the substrate at a thickness of 1 to 5000 nm, or 10 to 1000 nm, or 10 to 500 nm, or 10 to 200 nm, or 20 to 100 nm, or 30 to 1000 nm, or 30 to 500 nm thick. A typical thickness is from 30 to 1000 nm or from 20 to 100 nm, a very typical thickness is from 80 to 150 nm. These ranges are representing average thicknesses, as a certain roughness may enhance the lubricious properties of the lubricity coating. Thus its thickness is advantageously not uniform throughout the coating (see below). However, a uniformly thick lubricity coating is also considered.
The absolute thickness of the lubricity coating at single measurement points can be higher or lower than the range limits of the average thickness, with maximum deviations of preferably +/- 50%, more preferably +/- 25% and even more preferably +/- 15% from the average thickness. However, it typically varies within the thickness ranges given for the average thickness in this description.
The thickness of this and other coatings can be measured, for example, by transmission electron microscopy (TEM).
V.C. The TEM can be carried out, for example, as follows. Samples can be prepared for Focused Ion Beam (FIB) cross-sectioning in two ways. Either the samples can be first coated with a thin layer of carbon (50-100nm thick) and then coated with a sputtered layer of platinum (50-100nm thick) using a K575X Emitech coating system, or the samples can be coated directly with the protective sputtered Pt layer. The coated samples can be placed in an FEI FIB200 FIB system. An additional layer of platinum can be FIB-deposited by injection of an oregano-metallic gas while rastering the 30kV gallium ion beam over the area of interest. The area of interest for each sample can be chosen to be a location half way down the length of the syringe barrel. Thin cross sections measuring approximately 15μm ("micrometers") long, 2μm wide and 15μm deep can be extracted from the die surface using a proprietary in-situ FIB lift-out technique. The cross sections can be attached to a 200 mesh copper TEM grid using FIB-deposited platinum. One or two windows in each section, measuring ~ 8μm wide, can be thinned to electron transparency using the gallium ion beam of the FEI FIB.
V.C. Cross-sectional image analysis of the prepared samples can be performed utilizing either a Transmission Electron Microscope (TEM), or a Scanning Transmission Electron Microscope (STEM), or both. All imaging data can be recorded digitally. For STEM imaging, the grid with the thinned foils can be transferred to a Hitachi HD2300 dedicated STEM. Scanning transmitted electron images can be acquired at appropriate magnifications in atomic number contrast mode (ZC) and transmitted electron mode (TE). The following instrument settings can be used.

| 1. Instrument | Scanning Transmission Electron Microscope |
|---|---|
| Manufacturer/Model | Hitachi HD2300 |
| Accelerating Voltage | 200kV |
| Objective Aperture | #2 |
| Condenser Lens 1 Setting | 1.672 |
| Condenser Lens 2 Setting | 1.747 |
| Approximate Objective Lens Setting | 5.86 |
| ZC Mode Projector Lens | 1.149 |
| TE Mode Projector Lens | 0.7 |
| Image Acquisition | |
| Pixel Resolution | 1280x960 |
| Acquisition Time | 20sec.(x4) |

V.C. For TEM analysis the sample grids can be transferred to a Hitachi HF2000 transmission electron microscope. Transmitted electron images can be acquired at appropriate magnifications. The relevant instrument settings used during image acquisition can be those given below.

| Instrument | Transmission Electron Microscope |
|---|---|
| Manufacturer/Model | Hitachi HF2000 |
| Accelerating Voltage | 200 kV |
| Condenser Lens 1 | 0.78 |
| Condenser Lens 2 | 0 |
| Objective Lens | 6.34 |
| Condenser Lens Aperture | #1 |
| Objective Lens Aperture for imaging<br>Selective Area Aperture for SAD | #3<br>N/A |

V.C. In any of embodiments V.C., the substrate can comprise glass or a polymer, for example a polycarbonate polymer, an olefin polymer, a cyclic olefin copolymer, a polypropylene polymer, a polyester polymer, a polyethylene terephthalate polymer or a combination of any two or more of these.

V.C. In any of embodiments V.C., the PECVD optionally can be performed by energizing the gaseous reactant containing the precursor with electrodes powered at a RF frequency as defined above, for example a frequency from 10 kHz to less than 300 MHz, optionally from 1 to 50 MHz, even optionally from 10 to 15 MHz, optionally a frequency of 13.56 MHz.

V.C. In any of embodiments V.C., the plasma can be generated by energizing the gaseous reactant comprising the precursor with electrodes supplied with electric power sufficient to form a lubricity layer. Optionally, the plasma is generated by energizing the gaseous reactant containing the precursor with electrodes supplied with an electric power of from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally is from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity coatings to syringes and sample tubes and vessels of similar geometry having a void volume of 1 to 3 mL in which PECVD plasma is generated. It is contemplated that for larger or smaller objects the power applied should be increased or reduced accordingly to scale the process to the size of the substrate.

V.C. In any of embodiments V.C., one preferred combination of process gases includes octamethylcyclotetrasiloxane (OMCTS) or another cyclic siloxane as the precursor, in the presence of oxygen as the oxidizing gas and argon as the

carrier gas. Without being bound to the accuracy of this theory, the inventors believe this particular combination is effective for the following reasons.

V.C. It is believed that the OMCTS or other cyclic siloxane molecule provides several advantages over other siloxane materials. First, its ring structure results in a less dense coating (as compared to coatings prepared from HMDSO). The molecule also allows selective ionization so that the final structure and chemical composition of the coating can be directly controlled through the application of the plasma power. Other organosilicon molecules are readily ionized (fractured) so that it is more difficult to retain the original structure of the molecule.

V.C. Since the addition of Argon gas improves the lubricity performance (see the working examples below), it is believed that additional ionization of the molecule in the presence of Argon contributes to providing lubricity. The Si-O-Si bonds of the molecule have a high bond energy followed by the Si-C, with the C-H bonds being the weakest. Lubricity appears to be achieved when a portion of the C-H bonds are broken. This allows the connecting (cross-linking) of the structure as it grows. Addition of oxygen (with the Argon) is understood to enhance this process. A small amount of oxygen can also provide C-O bonding to which other molecules can bond. The combination of breaking C-H bonds and adding oxygen all at low pressure and power leads to a chemical structure that is solid while providing lubricity.

In a specific embodiment of the present invention, the lubricity can also be influenced by the roughness of the lubricity coating which results from the PECVD process using the precursors and conditions described herein. It has now surprisingly been found in the context of present invention by performing scanning electron microscopy (SEM) and atomic force microscopy (AFM), that a rough, non-continuous OMCTS plasma coating offers lower plunger force (Fi, Fm as described in PCT/US11/36097 filed on 11 May 2011) than a smooth, continuous OMCTS plasma coating. This is demonstrated by Examples O to V of PCT/US11/36097 filed on 11 May 2011.

While not bound by theory, the inventors assume that this particular effect could be, in part, based on one or both of the following mechanistic effects:

(a) lower surface contact of the plunger with the lubricity coating (e.g. the circular rigid plunger surface contacting only the peaks of a rough coating), either initially and/or throughout the plunger movement, resulting in overall lower contact and thus friction. (b) upon plunger movement, the plunger causes the initial non-uniform, rough coating to be spread and smoothed into the uncoated "valleys".

The roughness of the lubricity coating is increased with decreasing power (in Watts) energizing the plasma, and by the presence of $O_2$ in the amounts described above. The roughness can be expressed as "RMS roughness" or "RMS" determined by AFM. RMS is the standard deviation of the difference between the highest and lowest points in an AFM image (the difference is designated as "Z"). It is calculated according to the formula:

$$R_q = \{\Sigma(Z_1 - Z_{avg})^2/N\}^{-2}$$

where $Z_{avg}$ is the average Z value within the image; $Z_1$ is the current value of Z; and N is the number of points in the image.

The RMS range in this specific embodiment is typically from 7 to 20 nm, preferably from 12 to 20 nm. A lower RMS can, however, still lead to satisfying lubricity properties.

V.C. One contemplated product optionally can be a syringe having a barrel treated by the method of any one or more of embodiments V.C. Said syringe can either have just a lubricity coating according to present invention, or it can have the lubricity coating and one or more other coatings in addition, e.g. a $SiO_x$ barrier coating under or over the lubricity coating.

### V.D. Liquid-applied Coatings

V.D. Another example of a suitable barrier or other type of coating, usable in conjunction with PECVD-applied coatings or other PECVD treatment as disclosed here, can be a liquid barrier, lubricant, surface energy tailoring, or other type of coating 90 applied to the interior surface of a vessel, either directly or with one or more intervening PECVD-applied coatings described in this specification, for example $SiO_x$, a lubricity layer characterized as defined in the Definition Section, or both.

V.D. Suitable liquid barriers or other types of coatings 90 also optionally can be applied, for example, by applying a liquid monomer or other polymerizable or curable material to the interior surface of the vessel 80 and curing, polymerizing, or crosslinking the liquid monomer to form a solid polymer. Suitable liquid barrier or other types of

coatings 90 can also be provided by applying a solvent-dispersed polymer to the surface 88 and removing the solvent.

V.D. Either of the above methods can include as a step forming a coating 90 on the interior 88 of a vessel 80 via the vessel port 92 at a processing station or device 28. One example is applying a liquid coating, for example of a curable monomer, prepolymer, or polymer dispersion, to the interior surface 88 of a vessel 80 and curing it to form a film that physically isolates the contents of the vessel 80 from its interior surface 88. The prior art describes polymer coating technology as suitable for coating plastic blood collection tubes. For example, the acrylic and polyvinylidene chloride (PVdC) coating materials and coating methods described in US Patent 6,165,566, which is hereby incorporated by reference, optionally can be used.

V.D. Either of the above methods can also or include as a step forming a coating on the exterior outer wall of a vessel 80. The coating optionally can be a barrier coating, optionally an oxygen barrier coating, or optionally a water barrier coating. One example of a suitable coating is polyvinylidene chloride, which functions both as a water barrier and an oxygen barrier. Optionally, the barrier coating can be applied as a water-based coating. The coating optionally can be applied by dipping the vessel in it, spraying it on the vessel, or other expedients. A vessel having an exterior barrier coating as described above is also contemplated.

## VI. VESSEL INSPECTION

VI. The vessel inspection by the outgassing method of present invention will be described in the following in more detail. It should be understood that the method, however, is also applicable to inspect other items than vessels, e.g. plastic films or soild three-dimensional objects. The inspection of such other items is also encompassed by the present invention.

VI. One station or device shown in FIG. 1 is the processing station or device 30, which can be configured to inspect the interior surface of a vessel 80 for defects, as by measuring the air pressure loss or mass flow rate or volume flow rate through a vessel wall or outgassing of a vessel wall. The device 30 can operate similarly to the device 26, except that better performance (less leakage or permeation at given process conditions) can be required of the vessel to pass the inspection provided by the device 30, since in the illustrated embodiment a barrier or other type of coating has been applied by the station or device 28 before the station or device 30 is reached. In an embodiment, this inspection of the coated vessel 80 can be compared to the inspection of the same vessel 80 at the device or station 26. Less leakage or permeation at the station or device 30 indicates that the barrier coating is functioning at least to a degree.

VI. The identity of a vessel 80 measured at two different stations or by two different devices can be ascertained by placing individual identifying characteristics, such as a bar code, other marks, or a radio frequency identification (RFID) device or marker, on each of the vessel holders 38-68 and matching up the identity of vessels measured at two or more different points about the endless conveyor shown in FIG. 1. Since the vessel holders can be reused, they can be registered in a computer database or other data storage structure as they reach the position of the vessel holder 40 in FIG. 1, just after a new vessel 80 has been seated on the vessel holder 40, and removed from the data register at or near the end of the process, for example as or after they reach the position of the vessel holder 66 in FIG. 1 and the processed vessel 80 is removed by the transfer mechanism 74.

VI. The processing station or device 32 can be configured to inspect a vessel, for example a barrier or other type of coating applied to the vessel, for defects.

VI. The vessel inspection method according to the invention can include carrying out the inspecting step (i.e. the measurement of the outgassed volatile species) within an elapsed time of 30 or fewer seconds per vessel, or 25 or fewer seconds per vessel, or 20 or fewer seconds per vessel, or 15 or fewer seconds per vessel, or 10 or fewer seconds per vessel, or 5 or fewer seconds per vessel, or 4 or fewer seconds per vessel, or 3 or fewer seconds per vessel, or 2 or fewer seconds per vessel, or 1 or fewer seconds per vessel. This can be made possible, for example, by measuring the efficacy of the barrier or other type of coated vessel wall.

VI. In any of the above embodiments, the inspecting step can be carried out at a sufficient number of positions throughout the vessel 80 interior surface 88 to determine that the barrier or other type of coating 90 will be effective to prevent the initial vacuum level (i.e. initial reduction of pressure versus ambient) within the vessel 80, when it is

initially evacuated and its wall 86 is exposed to the ambient atmosphere, from decreasing more than 20%, optionally more than 15%, optionally more than 10%, optionally more than 5%, optionally more than 2%, during a shelf life of at least 12 months, or at least 18 months, or at least two years.

VI. The initial vacuum level can be a high vacuum, i.e. a remaining pressure of less than 10 Torr, or a lesser vacuum such as less than 20 Torr of positive pressure (i.e. the excess pressure over a full vacuum), or less than 50 Torr, or less than 100 Torr, or less than 150 Torr, or less than 200 Torr, or less than 250 Torr, or less than 300 Torr, or less than 350 Torr, or less than 380 Torr of positive pressure. The initial vacuum level of evacuated blood collection tubes, for example, is in many instances determined by the type of test the tube is to be used for, and thus the type and appropriate amount of a reagent that is added to the tube at the time of manufacture. The initial vacuum level is commonly set to draw the correct volume of blood to combine with the reagent charge in the tube.

VI. Optionally, the barrier or other type of coating 90 inspecting step can be carried out at a sufficient number of positions throughout the vessel interior surface 88 to determine that the barrier or other type of coating 90 will be effective to prevent the pressure within the vessel 80, when it is initially evacuated and its wall is exposed to the ambient atmosphere, from increasing to more than 15%, or more than 10%, of the ambient atmospheric pressure of the ambient atmospheric pressure during a shelf life of at least one year.

FIG. 15 shows a processing system 20 according to an exemplary embodiment of the present invention, comprising apparatus (i.e. one or more processing stations) adapted for performing the above and below described inspection method. The processing system 20 may be a vessel processing system and comprises, inter alia, a first processing station 5501 and may or may not also comprise a second processing station 5502. Examples for such processing stations are for example depicted in Fig. 1, reference numerals 24, 26, 28, 30, 32 and 34.

The first vessel processing station 5501 contains a vessel holder 38 which holds a seated vessel 80. Although FIG. 15 depicts a blood tube 80, the vessel may also be a syringe body, a vial, a catheter or, for example, a pipette or any other object having a surface to be inspected. The vessel may, for example, be made of glass or plastic. In case of plastic vessels, the first processing station may also comprise a mould for moulding the plastic vessel.

After the first processing at the first processing station (which processing may comprise moulding of the vessel, a first inspection of the vessel for defects, coating of the interior surface of the vessel and a second inspection of the vessel for defects, in particular of the interior coating), the vessel holder 38 may be transported together with the vessel 82 to the second vessel processing station 5502. This transportation is performed by a conveyor arrangement 70, 72, 74. For example, a gripper or several grippers may be provided for gripping the vessel holder 38 and/or the vessel 80 in order to move the vessel/holder combination to the next processing station 5502. Alternatively, only the vessel may be moved without the holder. However, it may be advantageous to move the holder together with the vessel in which case the holder is adapted such that it can be transported by the conveyor arrangement.

One or more of the processing stations may comprise a tubing for supplying a volatile species to the vicinity of the surface to be inspected, e.g. into the interior volume of the vessel. Further, a gas detector may be provided for measuring the release of at least one volatile species from the inspection object into the gas space adjacent to the coated surface.

FIG. 16 shows a vessel processing system 20 according to another exemplary embodiment of the present invention. Again, two vessel processing stations 5501, 5502 are provided. Furthermore, additional vessel processing stations 5503, 5504 may be provided which are arranged in series and in which the vessel can be processed, i.e. inspected and/or coated.

A vessel can be moved from a stock to the left processing station 5504. Alternatively, the vessel can be moulded in the first processing station 5504. In any case, a first vessel processing is performed in the processing station 5504, such as a moulding, an inspection and/or a coating, which may be followed by a second inspection. Then, the vessel is moved to the next processing station 5501 via the conveyor arrangement 70, 72, 74. Typically, the vessel is moved together with the vessel holder. A second processing is performed in the second processing station 5501 after which the vessel and holder are moved to the next processing station 5502 in which a third processing is performed. The vessel is then moved (again together with the holder) to the fourth processing station 5503 for a fourth processing, after which it is conveyed to a storage.

Before and after each coating step or moulding step or any other step which manipulates the vessel an inspection of the whole vessel, of part of the vessel and in particular of an interior surface of the vessel may be performed. The result of each inspection can be transferred to a central processing unit 5505 via a data bus 5507. Each processing station is connected to the data bus 5507. The above described program element may run on the processor 5505, and the processor, which may be adapted in form of a central control and regulation unit, controls the system and may also be adapted to process the inspection data, to analyze the data and to determine whether the last processing step was successful.

If it is determined that the last processing step was not successful, because for example the coating comprises holes or because the surface of the coating is determined to be regular or not smooth enough, the vessel does not enter the next processing station but is either removed from the production process (see conveyor sections 7001, 7002, 7003, 7004) or conveyed back in order to become re-processed.

The processor 5505 may be connected to a user interface 5506 for inputting control or regulation parameters.

FIG. 17 shows a vessel processing station 5501 according to an exemplary embodiment of the present invention. The station comprises a PECVD apparatus 5701 for coating an interior surface of the vessel. Furthermore, several detectors 5702-5707 may be provided for vessel inspection. Such detectors may for example be electrodes for performing electric measurements, optical detectors, like CCD cameras, gas detectors or pressure detectors.

FIG. 18 shows a vessel holder 38 according to an exemplary embodiment of the present invention, together with several detectors 5702, 5703, 5704 and an electrode with gas inlet port 108, 110.

The electrode and the detector 5702 may be adapted to be moved into the interior space of the vessel 80 when the vessel is seated on the holder 38.

The optical inspection may be particularly performed during a coating step, for example with the help of optical detectors 5703, 5704 which are arranged outside the seated vessel 80 or even with the help of an optical detector 5705 arranged inside the interior space of the vessel 80.

The detectors may comprise colour filters such that different wavelengths can be detected during the coating process. The processing unit 5505 analyzes the optical data and determines whether the coating was successful or not to a predetermined level of certainty. If it is determined that the coating was most probably unsuccessful, the respective vessel is separated from the processing system or re-processed.

**VI.A. Vessel Processing Including Pre-Coating and Post-Coating Inspection**

VI.A. Even another embodiment is a vessel processing method for processing a molded plastic vessel having an opening and a wall defining an interior surface. The method is carried out by inspecting the interior surface of the vessel as molded or just before coating for defects; applying a coating to the interior surface of the vessel after inspecting the vessel as molded; and inspecting the coating for defects.

VI.A. Another embodiment is a vessel processing method in which a barrier coating is applied to the vessel after inspecting the vessel as molded, and the interior surface of the vessel is inspected for defects after applying the barrier coating.

VI.A. Optionally, the vessel inspection at the station or by the device 26 can be modified by providing an inspection gas, such as helium, on an upstream side with respect to the substrate, either within or outside the vessel 80, and detecting it on the downstream side. A low-molecular-weight gas, such as hydrogen, or a less expensive or more available gas, such as oxygen or nitrogen, can also be used as an inspection gas.

VI.A. Helium is contemplated as an inspection gas that can increase the rate of leak or permeation detection, as it will pass through an imperfect barrier or other type of coating, or past a leaking seal, much more quickly than the usual ambient gases such as nitrogen and oxygen in ordinary air. Helium has a high transfer rate through many solid substrates or small gaps because it: (1) is inert, so it is not adsorbed by the substrate to any great degree, (2) is not ionized easily, so its molecules are very compact due to the high level of attraction between its electrons and nucleus, and (3) has a molecular weight of 4, as opposed to nitrogen (molecular weight 28) and oxygen (molecular weight 32), again making the molecules more compact and easily passed through a porous substrate or gap. Due to these factors, helium will travel through a barrier having a given permeability much more quickly than many other gases. Also, the atmosphere contains an extremely small proportion of helium naturally, so the presence of additional helium can be relatively easy to detect, particularly if the helium is introduced within the vessel 80 and detected outside the vessel 80 to measure leakage and permeation. The helium can be detected by a pressure drop upstream of the substrate or by other means, such as spectroscopic analysis of the downstream gas that has passed through the substrate.

VI.A. After molding a device 80, as at the station 22, several potential issues can arise that will render any subsequent treatment or coating imperfect, and possibly ineffective. If the devices are inspected prior to coating for these issues, the devices can be coated with a highly optimized, optionally up to 6-sigma controlled process that will ensure a desired result (or results).

VI.A. Some of the potential problems that can interfere with treatment and coating include (depending on the nature of the coated article to be produced):

VI.A. 1. Large density of particulate contamination defects (for example, each more than 10 micrometers in its longest dimension), or a smaller density of large particulate contamination (for example, each more than 10 micrometers in its longest dimension).

VI.A. 2. Chemical or other surface contamination (for example silicone mold release or oil).

VI.A. 3. High surface roughness, characterized by either a high/large number of sharp peaks and/or valleys. This can also be characterized by quantifying the average roughness (Ra) which should be less than 100 nm.

VI.A. 4. Any defect in the device such as a hole that will not allow a vacuum to be created.

VI.A. 5. Any defect on the surface of the device that will be used to create a seal (for example the open end of a sample collection tube).

VI.A. 6. Large wall thickness non-uniformities which can impede or modify power coupling through the thickness during treatment or coating.

VI.A. 7. Other defects that will render the barrier or other type of coating ineffective.

VI.A. To assure that the treatment/coating operation is successful using the parameters in the treatment/coating operation, the device can be pre-inspected for one or more of the above potential issues or other issues. Previously, an apparatus was disclosed for holding a device (a puck or vessel holder such as 38-68) and moving it through a production process, including various tests and a treatment/coating operation. Several possible tests can be implemented to ensure that a device will have the appropriate surface for treatment/coating. These include outgassing of the vessel wall, which optionally can be measured as described below under post-coating inspection to determine an outgassing baseline.

VI.A. The above testing can be conducted in a station 28 as shown in FIG. 2. In this figure the device (for example a sample collection tube 80) can be held in place and an appropriate detector positioned to measure the desired result.

VI.A. In the case of vacuum leak detection, the vessel holder and device can be coupled to a vacuum pump and a measuring device inserted into the tube. The testing can also be conducted as detailed elsewhere in the specification.

VI.A. The above systems can be integrated into a manufacturing and inspection method comprising multiple steps.

VI.A. FIG. 1 as previously described shows a schematic layout of the steps of one possible method (although this invention is not limited to a single concept or approach). First the vessel 80 is visually inspected at the station or by the device 24, which can include dimensional measurement of the vessel 80. If there are any defects found, the device or vessel 80 is rejected and the puck or vessel holder such as 38 is inspected for defects, recycled or removed.

VI.A. Next the leak rate or other characteristics of the assembly of a vessel holder 38 and seated vessel 80 is tested, as at the station 26, and stored for comparison after coating. The puck or vessel holder 38 then moves, for example, into the coating step 28. The device or vessel 80 is coated with a $SiO_x$ or other barrier or other type of coating at a power supply frequency of, for example, 13.56 MHz. Once coated, the vessel holder is retested for its leak rate or other characteristics (this can be carried out as a second test at the testing station 26 or a duplicate or similar station such as 30 - the use of a duplicate station can increase the system throughput).

VI.A. The coated measurement can be compared to the uncoated measurement. If the ratio of these values exceeds a pre-set required level, indicating an acceptable overall coating performance, the vessel holder and device move on. The value can be required to exceed a pre-set limit at which the device is rejected or recycled for additional coating. Next (for devices that are not rejected), a second, optical testing station 34 can be used. In this case a point light source can be inserted inside of the tube or vessel 80 and pulled out slowly while measurements are taken with a tubular CCD detector array outside of the tube. The data is then computationally analyzed to determine the defect density distribution. Based on the measurements the device is either approved for final packaging or rejected.

VI.A. The above data optionally can be logged and plotted (for example, electronically) using statistical process control techniques to ensure up to 6-sigma quality.

### VI.B. Vessel Inspection By Detecting Outgassing Of Container Wall Through Barrier layer

A method for inspecting an object for a barrier layer by outgassing measurement, is described in the following. In the method, an object made at least in part of a first material (substrate) is provided. The object has a surface, and optionally has at least a partial barrier layer between the first material and the surface. In the broadest aspect of the disclosed technology, the barrier layer is optional because in a particular situation the vessel may be inspected to determine whether or not it has a barrier coating. Optionally, a charging material is provided that is soluble in, absorbed by, or adsorbed by the first material. The object is in this case contacted with the charging material. Outgassing is then measured from at least a portion of the surface. The method is carried out under conditions effective to determine the presence or absence of a barrier layer.

Discrimination between "coated" and "uncoated" can be made based on the initial slopes of the respective flow rates. These flow rate slopes have to be distinguishable from each other either by (a) a direct slope calculation (delta flow/delta time) or (b) algorithms which interpolate a calculated slope back from a flow rate vs. time curve. The difference in the slopes between a coated article and an uncoated article can be very small to be sufficient to perform the invention, as long as they are reproducible (see the Examples). But generally, the difference should be at least 0.1 sec, more preferably at least from 0.3 sec to 10 sec, even more preferably at least from 1 sec to 5 sec. The upper limit of the slope difference can be several minutes, e.g. 15 or 30 minutes. Typically, the slope difference ranges from 1 second to 15 minutes, more typically from 1 sec to 1 min, from 1 sec to 30 sec or from 1 sec to 10 sec.

The six sigma evaluation as explained with regard to FIG. 8 elswhere in this specification is a very helpful tool for distinguisiong pass or fail for the inspection. The sigma number applied by the inventors in the Examples was six, and this is also the most preferred number for performing the invention. Dependant on the desired reliability, the sigma number can, however, vary from 2 to 8, preferably from 3 to 7, more preferably from 4 to 7 or from 5 to 7. Reliability versus sigma number is a tradeoff: if longer inspection times are acceptable when performing the invention, one gets a higher sigma number.

The volatile species measured can be a volatile species released from the coating, a volatile species released from the substrate, or a combination of both. In one aspect, the volatile species is a volatile species released from the coating, optionally is a volatile coating component, and the inspection is performed to determine the presence, the properties and/or the composition of the coating. In another aspect, the volatile species is a volatile species released from the substrate and the inspection is performed to determine the presence of the coating and/or the barrier effect of the coating. In particular, the volatile species may be an atmospheric constituent, for example nitrogen, oxygen, water, carbon dioxide, argon, helium, neon, krypton, xenon, or ambient air.

Some examples of suitable objects which can be provided with barrier layers are films or vessels. Some specific contemplated vessels are a syringe or syringe barrel, a medical sample collection vessel, a vial, an ampoule, and/or a tube with one end closed and the other end open, for example a blood or other medical sample collection tube.

The object can be a vessel having a plastic, e.g. a thermoplastic wall. The first material, i.e. the material forming the plastic wall, can comprise, consist essentially of, or consist of, for example, a thermoplastic material, for example a polyester, for example polyethylene terephthalate (PET), polybutylene terephthalate (PBT), or polyethylene naphthalate or combinations or interpolymers thereof. The first material can comprise, consist essentially of, or consist of, for example, an olefin polymer, for example a cyclic olefin copolymer (COC), a cyclic olefin polymer (COP), polypropylene homopolymer, a polypropylene copolymer, or combinations or interpolymers thereof. Other contemplated first materials comprise polystyrene, polycarbonate, polyvinyl chloride, nylon, polyurethane, epoxy resin, polyacrylonitrile (PAN), polymethylpentene, an ionomeric resin, for example Surlyn® ionomeric resin.

In any embodiment, the first material can comprise cyclic olefin copolymer, consist essentially of cyclic olefin copolymer, or consist of a cyclic olefin copolymer resin composition. In this embodiment, "consisting of" does not exclude other materials blended with the pure cyclic olefin copolymer to make a complete molding composition. This definition of "consisting of" applies throughout this specification, to all materials. "Consisting of" also does not exclude laminar materials having at least one layer consisting of the indicated resin composition and other layers of unlike composition.

In any embodiment, the first material can comprise polyethylene terephthalate, consist essentially of polyethylene terephthalate, or consist of a polyethylene terephthalate resin composition.

A specifically preferred combination of first material (e.g. material of a tube coated with an $SiO_x$ coating) and volatile constituent is COC and carbon dioxide.

A specifically preferred combination (e.g. material of a tube coated with an $SiO_x$ coating) and volatile constituent is PET and water.

A further preferred combination of first material (e.g. material of a tube coated with an $SiO_x$ coating) and volatile constituent is COC and Argon.

In any embodiment, the barrier layer can comprise or consist of $SiO_x$, in which x is from about 1.5 to about 2.9, or any other suitable material as described elsewhere in this specification. The "barrier layer" can be a layer having some other primary function, such as conferring lubricity, hydrophobicity, or other surface properties, such as any layer described in this specification. Any method described in this specification for applying a barrier layer can be used.

The charging material can be any material that facilitates a measurement of outgassing by providing a gas to outgas from the material. Some non-limiting contemplated examples are an atmospheric constituent, for example nitrogen, oxygen, water, carbon dioxide, argon, helium, neon, krypton, xenon, or ambient air. Another type of charging material contemplated here comprises a process material used to form the barrier layer. For example, the charging material can include an organosilicon material, for example octamethylcyclotetrasiloxane, hexamethyldisiloxane, or any of the other gases disclosed in this specification as precursors or process materials. Another type of charging material contemplated is a carrier gas used, e.g. in the coating process.

The barrier coating optionally can be applied or present either before the charging material is contacted, after the charging material is contacted, while the charging material is contacted, or at two or more of those stages.

The contacting step can be carried out in various ways, such as by exposing the object to a volume containing the charging material. Contacting can be carried out by exposing the object to ambient air containing the charging material. One contemplated charging material is water, which can be provided in the form of humid air. Contacting can be carried, out, for example before measuring outgassing, by contacting the barrier layer with air at a relative humidity of 35% to 100%, optionally 40% to 100%, optionally 40% to 50%, optionally at least 50%, optionally at least 60%, optionally at least 70%, optionally at least 80%, optionally at least 90%, optionally at least 95%, optionally 100%.

Contacting the object with the charging material can be carried out by exposing the object to a gas comprising the charging material, or by exposing the object to a liquid comprising the charging material.

An exemplary contacting time is from 0.1 second to one hour, optionally from 1 second to 50 minutes, optionally from 10 seconds to 40 minutes, optionally from one minute to thirty minutes, optionally from 5 minutes to 25 minutes, optionally from 10 minutes to 20 minutes. However, the contacting time can also be considerably shorter. One option to achieve a shorter contacting time (shorter than, e.g. the 12 min of the Examples) is increasing the temperature during contacting the object with the charging material. This temperature increase can accelerate the diffusion of the contacting material through the inspected item, e.g. through a plastic substrate. Eg., the diffusion of $CO_2$ through the wall of PET bottles was considerably increased when the temperature was increased to a temperature between 30 and 40°C.

Typical parameters and conditions for charging with $CO_2$ are indicated in the Basic Protocol for Charging with $CO_2$. The parameters given therein may be varied by +/-50% or less when performing the charging. Similar conditions, if appropriate modified taking into account the different physicochemical properties of the other charging materials described herein, are suitable for performing the charging with one of said other charging materials.

The enhanced discrimination of uncoated versus coated plastic articles, e.g. of articles coated with a barrier layer, in an outgassing measurement including charging of the article with a charging material according to the invention relies on the capability of the plastic article to imbibe gases into the resin for subsequent degassing measurement (in micrograms/minute) during the This is demonstrated in the Examples using Ar, N2 or Co2 as charging material. The solubility of gases in plastic is a key determinant of the amount of gas which can reside in a plastic resin, and therefore a good estimate of the potential for a particular charging gas to provide good uncoated versus coated article discrimination. While experimental determination of various gas solubilities in plastic resins is very limited, a linear relationship (Equation 1) between gas solubility, S, and the Lennard-Jones gas temperature [= gas potential energy constant (*epsilon*) divided by the Boltzmann constant (k)] has been determined by Van Amerongen, Michaels, and Bixler (D. W. Van Krevelen, Properties of Polymers, Elsivier, 3rd Ed., 1990, pp 538-542, and references therein) for glassy polymers with an accuracy of +/- 0.6.

$$\log S\ (298) = -7.4 + 0.010 * (\textit{epsilon}/\mathrm{k})$$

(Equation 1)

In Table H is a listing of gases (including the gases carbon dioxide, argon, and nitrogen used in the Examples), their Lennard-Jones gas temperature (epsilon/k ratio), calculated log S parameter, and the average ratio of Uncoated to $SiO_x$-Coated ATC (micrograms/min) signal of maximum separation. Plotting calculated log S versus the Experimental ATC ratios of carbon dioxide, argon, and nitrogen (Figure 5) indicates gases with higher gas solubilities are preferred and gases having lower gas solubilities less preferred.

In other words, the gases with greater solubility (further down on Table H) provided better separation of barrier coated versus uncoated substrates based on an outgassing measurement.

**Table H. List of gases, Lennard-Jones temperatures, calculated log S (298) and Uncoated/SiOx-Coated ATC Response.**

| Gas | (epsilon/kappa) [Kelvin] | Log S (298 Kelvin) | Uncoated/$SiO_x$-coated COC ratio (0-14 [ug/min] ATC microflow)* |
|---|---|---|---|
| He | 10 | -7.30 | |
| Ne | 33 | -7.07 | |
| $H_2$ | 60 | -6.80 | |
| $N_2$ | 71 | -6.69 | 1.2 |
| CO | 92 | -6.48 | |
| Ar | 93 | -6.47 | 1.6 |
| $O_2$ | 107 | -6.33 | |
| $CH_4$ | 149 | -5.91 | |
| Kr | 179 | -5.61 | |
| $CO_2$ | 195 | -5.45 | 2.5 |
| $C_2H_6$ | 216 | -5.24 | |
| $C_2H_4$ | 225 | -5.15 | |
| Xe | 231 | -5.09 | |
| *ratios from Tables F and G and FIG. 19 | | | |

The same principle applies to other coatings or the discrimination of different materials shown different gas absorption and adsorption.

Hence, it is preferred in the context of present invention to use a charging gas with a good solubility in the inspected plastic material. In order to increase the sensitivity of a test, the charging material can be supplemented or replaced by a charging material with a higher solubility in the plastic.

In a particular aspect of the invention, the charging material is selected for the materials listed in Table H.

In another particular aspect, it is selected from the group of gases having a log S of more than - 7.5, preferably of more than -7, even more preferably of more than - 6.9. In a specific embodiment, the charging gas has a log S in the range of from -7.5 to - 4.5, preferably of from -6.9 to - 5.1.

Measuring outgassing from at least a portion of the surface can be carried out, for example, by drawing at least a partial vacuum on the surface and measuring the flow rate of outgassing from the surface. Any outgassing measurement described in this specification is contemplated.

Outgassing can be measured at a pressure from 0.1 Torr to 100 Torr, optionally from 0.2 Torr to 50 Torr, optionally from 0.5 Torr to 40 Torr, optionally from 1 Torr to 30 Torr, optionally from 5 Torr to 100 Torr, optionally from 10 Torr to 80 Torr, optionally from 15 Torr to 50 Torr, for example by drawing a vacuum on the coated surface during the outgassing measurement.

Outgassing can be measured at a temperature from -10°C to 150°C, optionally from 0°C to 100 °C, optionally from 0°C to 50°C, optionally from 0°C to 21°C, optionally from 5°C to 20°C. Depending on the physicochemical properties of the first material (substrate) and the measured outgassed gases, other temperatures can also be suitable. Some materials, like COC, have higher permeability at elevated temperatures, though it is also important not to heat them so much as to cause distortion. For example, for COC (which has a glass transition temperature in the range of from about 70°C to about 180°C) the temperature for outgassing measurement could be about 80°C or higher, providing it remains below the glass transition temperature and distortion is avoided.

In any of the embodiments described in this specification, the first material optionally can be provided in the form of a vessel having a wall having an outer surface and an inner surface, the inner surface enclosing a lumen. Optionally, the barrier layer can be disposed on the inner surface of the vessel wall.

Optionally, a pressure differential can be provided across the barrier layer by at least partially evacuating the lumen. This can be done, for example, by connecting the lumen via a duct to a vacuum source to at least partially evacuate the lumen.

An outgassing measurement cell can be provided, communicating between the lumen and the vacuum source.

Outgassing can be measured by determining the volume of outgassed material which passes through the barrier layer per interval of time.

Outgassing can be measured using micro-flow technology.

Outgassing can be measured as the mass flow rate of outgassed material.

Outgassing can be measured in a molecular flow mode of operation.

Outgassing can be measured using microcantilever technology as described in this specification.

Optionally, a pressure differential can be provided across the barrier layer, such that at least some of the material that outgasses is on the higher-pressure side of the barrier layer. In another option, the outgassed gas can be allowed to diffuse without providing a pressure difference. The outgassed gas is measured. If a pressure differential is provided across the barrier layer, the outgassing can be measured on the higher-pressure or lower-pressure side of the barrier layer.

VI.B. In addition a measurement of the efficacy of the interior coating (applied above) can be made by measuring the diffusion rate of a specific species or adsorbed materials in the wall of the device (prior to coating). When compared to an uncoated (untreated) tube, this type of measurement can provide a direct measurement of the barrier or other type of properties of the coating or treatment, or the presence or absence of the coating or treatment. The coating or treatment detected, in addition to or instead of being a barrier layer, can be a lubricity layer, a hydrophobic layer, a decorative coating, or other types of layers that modify the outgassing of the substrate, either by increasing or decreasing it.

VI.B. Distinctions are made in this disclosure among "permeation," "leakage," and "surface diffusion" or "outgassing." "Permeation" as used here in reference to a vessel is traverse of a material through a wall 346 or other obstruction, as from the outside of the vessel to the inside or vice versa along the path 350 in FIG. 6 or the reverse of that path. Outgassing refers to the movement of an absorbed or adsorbed material such as the gas molecule 354 or 357 or 359 outward from within the wall 346 or coating 348 in FIG. 6, for example through the coating 348 (if present) and into the vessel 358 (to the right in FIG. 6). Outgassing can also refer to movement of a material such as 354 or 357 out of the wall 346, to the left as shown in FIG. 6, thus to the outside of the vessel 357 as illustrated. Outgassing can also refer to the removal of adsorbed material from the surface of an article, for example the gas molecule 355 from the exposed surface of the barrier coating 90.
Leakage refers to the movement of a material around the obstruction represented by the wall 346 and coating 348 rather than through or off the surface of the obstruction, as by passing between a closure and the wall of a vessel closed with a closure.

VI.B. Permeation is indicative of the rate of gas movement through a material, devoid of gaps/defects and not relating to leaks or outgassing. Referring to FIG. 6, which shows a vessel wall or other substrate 346 having a barrier coating 348, permeation is traverse of a gas entirely through the substrate 346 and coating 348 along the path 350 through both layers. Permeation is regarded as a thermodynamic, thus relatively slow, process.

VI.B. Permeation measurements are very slow, as the permeating gas must past entirely through an unbroken wall of the plastic article. In the case of evacuated blood collection tubes, a measurement of permeation of gas through its wall is conventionally used as a direct indication of the propensity of the vessel to lose vacuum over time, but commonly is an extremely slow measurement, commonly requiring a test duration of six days, thus not fast enough to support on-line coating inspection. Such testing is ordinarily used for off-line testing of a sample of vessels.

VI.B. Permeation testing also is not a very sensitive measurement of the barrier efficacy of a thin coating on a thick substrate. Since all the gas flow is through both the coating and the substrate, variations in flow through the thick substrate will introduce variation that is not due to the barrier efficacy of the coating per se.

VI.B. The inventors have found a much quicker and potentially more sensitive way of measuring the barrier properties of a coating - measuring outgassing of quickly-separated air or other gaseous or volatile constituents in the vessel wall through the coating. The gaseous or volatile constituents can be any material that in fact outgasses, or can be selected from one or more specific materials to be detected. The constituents can include, but are not limited to, oxygen, nitrogen, air, carbon dioxide, water vapor, helium, volatile organic materials such as alcohols, ketones, hydrocarbons, halogenated hydrocarbons, ethers, coating precursors, substrate components, by-products of the preparation of the coating such as volatile organosilicons, by-products of the preparation of the coated substrate, other constituents that happen to be present or are introduced by spiking the substrate, or mixtures or combinations of any of these.
Outgassing can provide much quicker measurement (compared to permeation) of the presence and barrier properties

of a coating such as 348.Outgassing is a more rapid test because the gas does not need to pass through the entire thickness of the wall 346 in FIG. 6. It can be present within the wall 346 close to the barrier coating 348 or inner surface of the vessel (if there is no coating), so it can traverse a very small proportion of the thickness of the wall 346. Since the barrier coating is thousands of times thinner than the vessel wall, measuring outgassing removes practically all of the delay required for permeation testing by reducing the wall thickness the gas must get through before it is detectable within the vessel. Reducing the distance to be traveled reduces the length of the trip.

Outgassing can be made more sensitive and thus quicker by charging the surface to be tested with a gas that is readily taken up by the vessel wall but not by the barrier coating. The interior of the vessel to be tested is exposed to the charging gas, then tested for outgassing. If an effective barrier coating is present, very little gas will be charged because the barrier coating will block it from entering the vessel wall. If no barrier coating is present, the charging gas will be taken up in a substantial proportion by the vessel wall, even in a very short period of time. The amount of gas that enters the wall and the presence or absence of a barrier coating will determine the amount of outgassing to the interior of the vessel.

Finally, the method of measuring outgassing will determine how quickly it can be measured. The preferred measurement technique is known as microflow technology. Microflow technology used in the present method has allowed the presence and efficacy of the barrier coating to be verified in a few seconds, potentially in one second or less.

It has been found that the outgassing method can differentiate among coated and uncoated plastic tubes in a few seconds, and even in less than a second.

Surface diffusion and outgassing are synonyms. Each term refers to fluid initially adsorbed on or absorbed in a wall 346, such as the wall of a vessel, and caused to pass into the adjacent space by some motivating force, such as drawing a vacuum (creating air movement indicated by the large arrow of FIG. 6) within a vessel having a wall to force fluid out of the wall into the interior of the vessel. Outgassing or diffusion is regarded as a kinetic, relatively quick process. It is contemplated that, for a wall 346 having substantial resistance to permeation along the path 350, outgassing will quickly dislodge the molecules such as 354 that are closest to the interface 356 between the wall 346 and the barrier layer 348. This differential outgassing is suggested by the large number of molecules such as 354 near the interface 356 shown as outgassing, and by the large number of other molecules such as 358 that are further from the interface 356 and are not shown as outgassing.

VI.B. Accordingly, yet another method is contemplated for inspecting a barrier layer on a material that outgasses a vapor, including several steps. A sample of material is provided that outgasses a gas and has at least a partial barrier layer. A pressure differential is provided across the barrier layer, such that at least some of the material that outgasses initially is on the higher-pressure side of the barrier layer. The outgassed gas transported to the lower-pressure side of the barrier layer during a test is measured to determine such information as whether the barrier is present or how effective it is as a barrier.

VI.B. In this method, the material that outgasses a gas can include a polymeric compound, a thermoplastic compound, or one or more compounds having both properties. The material that outgasses a gas can include polyester, for example polyethylene terephthalate. The material that outgasses a gas can include a polyolefin, for two examples polypropylene, a cyclic olefin copolymer, or a combination of these. The material that outgasses a gas can be a composite of two different materials, at least one of which outgasses a vapor. One example is a two layer structure of polypropylene and polyethylene terephthalate. Another example is a two layer structure of cyclic olefin copolymer and polyethylene terephthalate. These materials and composites are exemplary; any suitable material or combination of materials can be used.

VI.B. Optionally, the material that outgasses a gas is provided in the form of a vessel having a wall having an outer surface and an inner surface, the inner surface enclosing a lumen. In this embodiment, the barrier layer optionally is disposed on the vessel wall, optionally on the inner surface of the vessel wall. The barrier layer could or also be disposed on the outer surface of the vessel wall. Optionally, the material that outgasses a gas can be provided in the form of a film.

VI.B. The barrier layer can be a full or partial coating of any of the presently described barrier layers. The barrier layer can be less than 500 nm thick, or less than 300 nm thick, or less than 100 nm thick, or less than 80 nm thick, or less than 60 nm thick, or less than 50 nm thick, or less than 40 nm thick, or less than 30 nm thick, or less than 20 nm thick, or less than 10 nm thick, or less than 5 nm thick. Typically, when it is an $SiO_x$ barrier layer, it may be about 20 to 30 nm thick.

VI.B. In the case of a coated wall, the inventors have found that diffusion/ outgassing can be used to determine the coating integrity. Optionally, a pressure differential can be provided across the barrier layer by at least partially

evacuating the lumen or interior space of the vessel. This can be done, for example, by connecting the lumen via a duct to a vacuum source to at least partially evacuate the lumen. For example, an uncoated PET wall 346 of a vessel that has been exposed to ambient air will outgas from its interior surface a certain number of oxygen and other gas molecules such as 354 for some time after a vacuum is drawn. If the same PET wall is coated on the interior with a barrier coating 348, the barrier coating will stop, slow down, or reduce this outgassing. This is true for example of an $SiO_x$ barrier coating 348, which outgasses less than a plastic surface. By measuring this differential of outgassing between coated and uncoated PET walls, the barrier effect of the coating 348 for the outgassed material can be rapidly determined.

VI.B. If the barrier coating 348 is imperfect, due to known or theoretical holes, cracks, gaps or areas of insufficient thickness or density or composition, the PET wall will outgas preferentially through the imperfections, thus increasing the total amount of outgassing. The primary source of the collected gas is from the dissolved gas or vaporizable constituents in the (sub)surface of the plastic article next to the coating, not from outside the article. The amount of outgassing beyond a basic level (for example the amount passed or released by a standard coating with no imperfections, or the least attainable degree of imperfection, or an average and acceptable degree of imperfection) can be measured in various ways to determine the integrity of the coating.

VI.B. The measurement can be carried out, for example, by providing an outgassing measurement cell communicating between the lumen and the vacuum source.

VI.B. The measurement cell can implement any of a variety of different measurement technologies. One example of a suitable measurement technology is micro-flow technology. For example, the mass flow rate of outgassed material can be measured. The measurement can be carried out in a molecular flow mode of operation. An exemplary measurement is a determination of the volume of gas outgassed through the barrier layer per interval of time.

VI.B. The outgassed gas on the lower-pressure side of the barrier layer can be measured under conditions effective to distinguish the presence or absence of the barrier layer. Optionally, the conditions effective to distinguish the presence or absence of the barrier layer include a test duration of less than one minute, or less than 50 seconds, or less than 40 seconds, or less than 30 seconds, or less than 20 seconds, or less than 15 seconds, or less than 10 seconds, or less than 8 seconds, or less than 6 seconds, or less than 4 seconds, or less than 3 seconds, or less than 2 seconds, or less than 1 second.

VI.B. Optionally, the measurement of the presence or absence of the barrier layer can be confirmed to at least a six-sigma level of certainty within any of the time intervals identified above.

VI.B. Optionally, the outgassed gas on the lower-pressure side of the barrier layer is measured under conditions effective to determine the barrier improvement factor (BIF) of the barrier layer, compared to the same material without a barrier layer. A BIF can be determined, for example, by providing two groups of identical containers, adding a barrier layer to one group of containers, testing a barrier property (such as the rate of outgassing in micrograms per minute or another suitable measure) on containers having a barrier, doing the same test on containers lacking a barrier, and taking a ratio of the properties of the materials with versus without a barrier. For example, if the rate of outgassing through the barrier is one-third the rate of outgassing without a barrier, the barrier has a BIF of 3.

VI.B. Optionally, outgassing of a plurality of different gases can be measured, in instances where more than one type of gas is present, such as both nitrogen and oxygen in the case of outgassed air. Optionally, outgassing of substantially all or all of the outgassed gases can be measured. Optionally, outgassing of substantially all of the outgassed gases can be measured simultaneously, as by using a physical measurement like the combined mass flow rate of all gases.

VI.B. Measuring the number or partial pressure of individual gas species (such as oxygen, helium, $CO_2$ or water vapor) outgassed from the sample can be done more quickly than barometric testing, but the rate of testing is reduced to the extent that only a fraction of the outgassing is of the measured species. For example, if nitrogen and oxygen are outgassed from the PET wall in the approximately 4:1 proportion of the atmosphere, but only oxygen outgassing is measured, the test would need to be run five times as long as an equally sensitive test (in terms of number of molecules detected to obtain results of sufficient statistical quality) that measures all the species outgassed from the vessel wall.

VI.B. For a given level of sensitivity, it is contemplated that a method that accounts for the volume of all species

outgassed from the surface will provide the desired level of confidence more quickly than a test that measures outgassing of a specific species, such as oxygen atoms. Consequently, outgassing data having practical utility for in-line measurements can be generated. Such in-line measurements can optionally be carried out on every vessel manufactured, thus reducing the number of idiosyncratic or isolated defects and potentially eliminating them (at least at the time of measurement).

VI.B. In a practical measurement, a factor changing the apparent amount of outgassing is leakage past an imperfect seal, such as the seal of the vessel seated on a vacuum receptacle as the vacuum is drawn in the outgassing test. Leakage means a fluid bypassing a solid wall of the article, for example fluid passing between a blood tube and its closure, between a syringe plunger and syringe barrel, between a container and its cap, or between a vessel mouth and a seal upon which the vessel mouth is seated (due to an imperfect or mis-seated seal). The word "leakage" is usually indicative of the movement of gas/gas through an opening in the plastic article.

VI.B. Leakage and (if necessary in a given situation) permeation can be factored into the basic level of outgassing, so an acceptable test result assures both that the vessel is adequately seated on the vacuum receptacle (thus its seated surfaces are intact and properly formed and positioned), the vessel wall does not support an unacceptable level of permeation (thus the vessel wall is intact and properly formed), and the coating has sufficient barrier integrity.

VI.B. Outgassing can be measured in various ways, as by barometric measurement (measuring the pressure change within the vessel in a given amount of time after the initial vacuum is drawn) or by measuring the partial pressure or flow rate of gas outgassed from the sample. Equipment is available that measures a mass flow rate in a molecular flow mode of operation. An example of commercially available equipment of this type employing Micro-Flow Technology is available from ATC, Inc., Indianapolis, IN. See U.S. Patent Nos. 5861546, 6308556, 6584828 and EP1356260, which are incorporated by reference here, for a further description of this known equipment. See also Example 8 in EP2251671 A2, showing an example of outgassing measurement to distinguish barrier coated polyethylene terephthalate (PET) tubes from uncoated tubes very rapidly and reliably.

VI.B. For a vessel made of polyethylene terephthalate (PET), the microflow rate is much different for the $SiO_x$ coated surface versus an uncoated surface. For example, in Working Example 8 in EP EP2251671 A2, the microflow rate for PET was 8 or more micrograms after the test had run for 30 seconds, as shown in FIG. 8. This rate for uncoated PET was much higher than the measured rate for $SiO_x$-coated PET, which was less than 6 micrograms after the test had run for 30 sec, again as shown in FIG. 8.

VI.B. One possible explanation for this difference in flow rate is that uncoated PET contains roughly 0.7 percent equilibrium moisture; this high moisture content is believed to cause the observed high microflow rate. With an $SiO_x$-coated PET plastic, the $SiO_x$ coating can have a higher level of surface moisture than an uncoated PET surface. Under the testing conditions, however, the barrier coating is believed to prevent additional desorption of moisture from the bulk PET plastic, resulting in a lower microflow rate. The microflow rates of oxygen or nitrogen from the uncoated PET plastic versus the $SiO_x$ coated PET would also be expected to be distinguishable.

VI.B. Modifications of the above test for a PET tube might be appropriate when using other materials. For example, polyolefin plastics tend to have little moisture content. An example of a polyolefin having low moisture content is TOPAS® cyclic olefin copolymer (COC), having an equilibrium moisture content (0.01 percent) and moisture permeation rate much lower than for PET. In the case of COC, uncoated COC plastic can have microflow rate similar to, or even less than, $SiO_x$-coated COC plastic. This is most likely due to the higher surface moisture content of the $SiO_x$-coating and the lower equilibrium bulk moisture content and lower permeation rate of an uncoated COC plastic surface. This makes differentiation of uncoated and coated COC articles more difficult.
The present invention shows that exposure of the to-be-tested surfaces of COC articles to moisture (uncoated and coated) results in improved and consistent microflow separation between uncoated and $SiO_x$-coated COC plastics. This is shown in Example 19 of EP2251671 A2 and FIG. 13. The moisture exposure can be simply exposure to relative humidity ranging from 35%-100%, either in a controlled relative humidity room or direct exposure to a warm (humidifier) or cold (vaporizer) moisture source, with the latter preferred.

VI.B. While the validity and scope of the invention are not limited according to the accuracy of this theory, it appears the moisture doping or spiking of the uncoated COC plastic increases its moisture or other outgassable content relative to the already saturated $SiO_x$-coated COC surface. This can also be accomplished by exposing the coated and uncoated tubes to other gases including carbon dioxide, oxygen, nitrogen, water vapor, or their mixtures, for example air. Carbon dioxide exposition (or "spiking") is especially effective in this regard when the tubes are made

of COC.

VI.B Thus, before measuring the outgassed gas, the barrier layer can be contacted with water, for example water vapor. Water vapor can be provided, for example, by contacting the barrier layer with air at a relative humidity of 35% to 100%, alternatively 40% to 100%, alternatively 40% to 50%. Instead of or in addition to water, the barrier layer can be contacted with oxygen, nitrogen or a mixture of oxygen and nitrogen, for example ambient air. Other materials can also be used to test outgassing, including carbon dioxide, nitrogen and noble gases (e.g. argon). The contacting time can be from 0.1 second to one hour, optionally from 1 second to 50 minutes, optionally from 10 seconds to 40 minutes, optionally from one minute to thirty minutes, optionally from 5 minutes to 25 minutes, optionally from 10 minutes to 20 minutes..
Alternatively, the wall 346 which will be outgassing can be spiked or supplemented from the side opposite a barrier layer 348, for example by exposing the left side of the wall 346 as shown in FIG. 11 to a material that will ingas into the wall 346, then outgas either to the left or to the right as shown in FIG. 6. Spiking a wall or other material such as 346 from the left by ingassing, then measuring outgassing of the spiked material from the right (or vice versa) is distinguished from permeation measurement because the material spiked is within the wall 346 at the time outgassing is measured, as opposed to material that travels the full path 350 through the wall at the time gas presented through the coating is being measured. The ingassing can take place over a long period of time, as one embodiment before the coating 348 is applied, and as another embodiment after the coating 348 is applied and before it is tested for outgassing.

VI.B. Another potential method to increase separation of microflow response between uncoated and $SiO_x$-coated plastics is to modify the measurement pressure and/or temperature. Increasing the pressure or decreasing the temperature when measuring outgassing can result in greater relative binding of water molecules in, e.g., $SiO_x$-coated COC than in uncoated COC. Thus, the outgassed gas can be measured at a pressure from 0.1 Torr to 100 Torr, alternatively from 0.2 Torr to 50 Torr, alternatively from 0.5 Torr to 40 Torr, alternatively from 1 Torr to 30 Torr, alternatively from 5 Torr to 100 Torr, alternatively from 10 Torr to 80 Torr, alternatively from 15 Torr to 50 Torr. The outgassed gas can be measured at a temperature from -10°C to 150°C, optionally from 0°C to 100 °C, optionally from 0°C to 50°C, optionally from 0°C to 21°C, optionally from 5°C to 20°C. For COC the temperature can be about 80°C or higher.
Another specific embodiment of is an improved system and an improved method for discrimination of plasma-coated COC plastic articles or surfaces, versus uncoated articles or surfaces, by measuring carbon dioxide infusion or charging of the COC articles or surfaces.
In contrast to PET resins which have substantial dissolved moisture (ca. 0.1-0.2 weight percent), cyclic olefin copolymer (COC) compositions, including TOPAZ-brand resins, have much lower equilibrium moisture (ca. 0.01%). Microflow analysis to distinguish outgassing rates between uncoated and plasma coated COCs using moisture outgassing is more difficult.
It has been discovered that infusion or charging of carbon dioxide ($CO_2$) into the COC article or surface provides improved microflow signal separation between uncoated and plasma-coated injection-molding COC articles. This increased discrimination will provide better determination of coating uniformity for performance optimization and faster discrimination of real-time, in-line assessment of acceptable or unacceptable coated articles. Thus, $CO_2$ is a preferred charging material for performing the outgassing method including charging the inspected item according to presention invention.
Preferred conditions for the use of the charging material, e.g. for the use of CO2 as charging material, are as follows:
Charging time: 0.1 second to one hour, optionally from 1 second to 50 minutes, optionally from 10 seconds to 40 minutes, optionally from one minute to thirty minutes, optionally from 5 minutes to 25 minutes, optionally from 10 minutes to 20 minutes. 12-14 min charging time are particularly considered.
Charging pressure: from 16 psi (1 atm) to 48 psi (3 atm), preferably from 20 psi to 30 psi, and 25 psi particularly preferred. Higher pressures than 3 atm when performing the charging are not desirable, as then the coated vessel and the coating might be stretched, resulting in defects like cracks and distensions in the coating and/or the substrate.
Typical parameters and conditions for charging with $CO_2$ are indicated in the Basic Protocol for Charging with $CO_2$. The parameters given therein may be varied by +/-50% or less when performing the charging. Similar conditions, if appropriate modified taking into account the different physicochemical properties of the other charging materials described herein, are suitable for performing the charging with one of said other charging materials.
The $CO_2$ or other charging material can be charged into the coated article immediately post-coating.
Other infusion or charging gases considered for improved uncoated vs plasma coated degassing discrimination using microflow methods would characteristically have low atmospheric natural abundance and high solubility in COC resins, including but not limited to helium, argon, neon, hydrogen, and acetylene. However, as the Examples show, $N_2$ can also be successfully used. Fluids which are liquids at standard temperature and pressure (760 Torr),

but vapors under analysis conditions (for example ca 1-5 Torr) may also be considered as alternative candidates for analysis, e.g. hexamethyldisiloxane and similar volatile organosilicon species, and halogenated hydrocarbons like methylene chloride.
$CO_2$ infusion or charging into plastic articles can be accomplished as a purge gas during the micro-flow measurement step, as part of a finishing step in the plasma-coating operation, or as a separate operation.

VI.B. A way contemplated for measuring outgassing, in any embodiment of the present disclosure, is to employ a microcantilever measurement technique. Such a technique is contemplated to allow measurement of smaller mass differences in outgassing, potentially on the order of $10^{-12}$ g (picograms) to $10^{-15}$ g (femtograms). This smaller mass detection permits differentiation of coated versus uncoated surfaces as well as different coatings in less than a second, optionally less than 0.1 sec, optionally a matter of microseconds.

VI.B. Microcantilever (MCL) sensors in some instances can respond to the presence of an outgassed or otherwise provided material by bending or otherwise moving or changing shape due to the absorption of molecules. Microcantilever (MCL) sensors in some instances can respond by shifting in resonance frequency. In other instances, the MCL sensors can change in both these ways or in other ways. They can be operated in different environments such as gaseous environments, liquids, or vacuum. In gas, microcantilever sensors can be operated as an artificial nose, whereby the bending pattern of a microfabricated array of eight polymer-coated silicon cantilevers is characteristic of the different vapors from solvents, flavors, and beverages. The use of any other type of electronic nose, operated by any technology, is also contemplated.
Several MCL electronic designs, including piezoresistive, piezoelectric, and capacitive approaches, have been applied and are contemplated to measure the movement, change of shape, or frequency change of the MCLs upon exposure to chemicals.

VI.B. One specific example of measuring outgassing can be carried out as follows. At least one microcantilever is provided that has the property, when in the presence of an outgassed material, of moving or changing to a different shape. The microcantilever is exposed to the outgassed material under conditions effective to cause the microcantilever to move or change to a different shape. The movement or different shape is then detected.

VI.B. As one example, the movement or different shape can be detected by reflecting an energetic incident beam from a portion of the microcantilever that moves or changes shape, before and after exposing the microcantilever to outgassing, and measuring the resulting deflection of the reflected beam at a point spaced from the cantilever. The shape is optionally measured at a point spaced from the cantilever because the amount of deflection of the beam under given conditions is proportional to the distance of the point of measurement from the point of reflection of the beam.

VI.B. Several suitable examples of an energetic incident beam are a beam of photons, a beam of electrons, or a combination of two or more of these. Alternatively, two or more different beams can be reflected from the MCL along different incident and/or reflected paths, to determine movement or shape change from more than one perspective. One specifically contemplated type of energetic incident beam is a beam of coherent photons, such as a laser beam. "Photons" as discussed in this specification are inclusively defined to include wave energy as well as particle or photon energy per se.

VI.B. An alternative example of measurement takes advantage of the property of certain MCLs of changing in resonant frequency when encountering an environmental material in an effective amount to accomplish a change in resonant frequency. This type of measurement can be carried out as follows. At least one microcantilever is provided that resonates at a different frequency when in the presence of an outgassed material. The microcantilever can be exposed to the outgassed material under conditions effective to cause the microcantilever to resonate at a different frequency. The different resonant frequency is then detected by any suitable means.

VI.B. As one example, the different resonant frequency can be detected by inputting energy to the microcantilever to induce it to resonate before and after exposing the microcantilever to outgassing. The differences between the resonant frequencies of the MCL before and after exposure to outgassing are determined. Alternatively, instead of determining the difference in resonant frequency, an MCL can be provided that is known to have a certain resonant frequency when in the presence of a sufficient concentration or quantity of an outgassed material. The different resonant frequency or the resonant frequency signaling the presence of a sufficient quantity of the outgassed material is detected using a harmonic vibration sensor.
As one example of using MCL technology for measuring outgassing, an MCL device can be incorporated into a

quartz vacuum tube linked to a vessel and vacuum pump. A harmonic vibration sensor using a commercially available piezoresistive cantilever, Wheatstone bridge circuits, a positive feedback controller, an exciting piezoactuator and a phase-locked loop (PLL) demodulator can be constructed. See, e.g.,

- Hayato Sone, Yoshinori Fujinuma and Sumio Hosaka Picogram Mass Sensor Using Resonance Frequency Shift of Cantilever, Jpn. J. Appl. Phys. 43 (2004) 3648;
- Hayato Sone, Ayumi Ikeuchi, Takashi Izumi, Haruki Okano and Sumio Hosaka Femtogram Mass Biosensor Using Self-Sensing Cantilever for Allergy Check, Jpn. J. Appl. Phys. 43 (2006) 2301).

To prepare the MCL for detection, one side of the microcantilever can be coated with gelatin. See, e.g., Hans Peter Lang, Christoph Gerber, STM and AFM Studies on (Bio)molecular Systems: Unravelling the Nanoworld, Topics in Current Chemistry, Volume 285/2008. Water vapor desorbing from the evacuated coated vessel surface binds with the gelatin, causing the cantilever to bend and its resonant frequency to change, as measured by laser deflection from a surface of the cantilever. The change in mass of an uncoated vs coated vessel is contemplated to be resolvable in fractions of seconds and be highly reproducible. The articles cited above in connection with cantilever technology are incorporated here by reference for their disclosures of specific MCLs and equipment arrangements that can be used for detecting and quantifying outgassed species.
Alternative coatings for moisture detection (phosphoric acid) or oxygen detection can be applied to MCLs in place of or in addition to the gelatin coating described above.

VI.B. It is further contemplated that any of the presently contemplated outgassing test set-ups can be combined with a coating station, e.g. a PECVD coating station which may provide an $SiO_x$ coating. In such an arrangement, the measurement cell 362 could be as illustrated above, using the main vacuum channel for PECVD as the bypass 386. In an embodiment, the measurement cell generally indicated as 362 of FIG. 7 can be incorporated in a vessel holder such as 50 in which the bypass channel 386 is configured as the main vacuum duct 94 and the measurement cell 362 is a side channel.

VI.B. This combination of the measurement cell 362 with the vessel holder 50 would optionally allow the outgassing measurement to be conducted without breaking the vacuum used for PECVD. Optionally, the vacuum pump for PECVD would be operated for a short, optionally standardized amount of time to pump out some or all of the residual reactant gases remaining after the coating step (a pump-down of less than one Torr, with a further option of admitting a small amount of air, nitrogen, oxygen, or other gas to flush out or dilute the process gases before pumping down). This would expedite the combined processes of coating the vessel and testing the coating for presence and barrier level.

VI.B. It will be further appreciated by those skilled in the art, after review of this specification, that outgassing measurements can be used for many purposes other than or in addition to determining the efficacy of a barrier layer. For one example, the test can be used on uncoated or coated vessels to determine the degree of outgassing of the vessel walls. This test can be used, for example, in cases in which an uncoated polymer is required to outgas less than a specified amount.

VI.B. For another example, these outgassing measurements can be used on barrier coated or uncoated films, either as a static test or as an in-line test to measure variations in outgassing of a film as it traverses the measurement cell. The test can be used for determining the continuity or barrier efficacy of other types of coatings, such as aluminum coatings or EVOH barrier coatings or layers of packaging films.

VI.B. These outgassing measurements can also be used to determine the efficacy of a barrier layer applied on the side of a vessel wall, film, or the like opposite the measurement cell, such as a barrier layer applied on the outside of a vessel wall and interrogated for outgassing to the interior of the vessel wall. In this instance, the flow differential would be for permeation through the barrier coating followed by permeation through the substrate film or wall. This measurement would be particularly useful in instances where the substrate film or wall is quite permeable, such as a very thin or porous film or wall.

VI.B. These outgassing measurements can also be used to determine the efficacy of a barrier layer which is an interior layer of a vessel wall, film, or the like, in which case the measurement cell would detect any outgassing through the layer adjacent to the measurement cell plus outgassing, through the barrier layer, of the layer or layers more remote from the measurement cell than the barrier layer.

VI.B. These outgassing measurements can also be used to determine the percentage of coverage of a pattern of barrier material over a material that outgasses, as by determining the degree of outgassing of the partially barrier coated material as a proportion of the amount of outgassing expected if no barrier were present over any part of the material.

VI.B. One test technique that can be used to increase the rate of testing for outgassing of a vessel, usable with any outgassing test embodiment in the specification, is to reduce the void volume of the vessel, as by inserting a plunger or closure into the vessel to reduce the void volume of the portion of the vessel tested. Decreasing the void volume allows the vessel to be pumped down more quickly to a given vacuum level, thus decreasing the test interval.

VI.B. Many other applications for the presently described outgassing measurements and all the other described barrier measurement techniques will be evident to the skilled person after reviewing this specification.

**VII. PECVD TREATED VESSELS**

VII. Vessels are contemplated having a barrier coating 90 (shown in FIG. 2, for example), which can be an $SiO_x$ coating applied to a thickness of at least 2 nm, or at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The coating can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Specific thickness ranges composed of any one of the minimum thicknesses expressed above, plus any equal or greater one of the maximum thicknesses expressed above, are expressly contemplated. The thickness of the $SiO_x$ or other coating can be measured, for example, by transmission electron microscopy (TEM), and its composition can be measured by X-ray photoelectron spectroscopy (XPS).

VII. It is contemplated that the choice of the material to be barred from permeating the coating and the nature of the $SiO_x$ coating applied can affect its barrier efficacy. For example, two examples of material commonly intended to be barred are oxygen and water/water vapor. Materials commonly are a better barrier to one than to the other. This is believed to be so at least in part because oxygen is transmitted through the coating by a different mechanism than water is transmitted.

VII. Oxygen transmission is affected by the physical features of the coating, such as its thickness, the presence of cracks, and other physical details of the coating. Water transmission, on the other hand, is believed to commonly be affected by chemical factors, i.e. the material of which the coating is made, more than physical factors. The inventors also believe that at least one of these chemical factors is a substantial concentration of OH moieties in the coating, which leads to a higher transmission rate of water through the barrier. An $SiO_x$ coating often contains OH moieties, and thus a physically sound coating containing a high proportion of OH moieties is a better barrier to oxygen than to water. A physically sound carbon-based barrier, such as amorphous carbon or diamond-like carbon (DLC) commonly is a better barrier to water than is a $SiO_x$ coating because the carbon-based barrier more commonly has a lower concentration of OH moieties.

VII. Other factors lead to a preference for an $SiO_x$ coating, however, such as its oxygen barrier efficacy and its close chemical resemblance to glass and quartz. Glass and quartz (when used as the base material of a vessel) are two materials long known to present a very high barrier to oxygen and water transmission as well as substantial inertness to many materials commonly carried in vessels. Thus, it is commonly desirable to optimize the water barrier properties such as the water vapor transmission rate (WVTR) of an $SiO_x$ coating, rather than choosing a different or additional type of coating to serve as a water transmission barrier.

VII. Several ways contemplated to improve the WVTR of an $SiO_x$ coating are as follow.

VII. The concentration ratio of organic moieties (carbon and hydrogen compounds) to OH moieties in the deposited coating can be increased. This can be done, for example, by increasing the proportion of oxygen in the feed gases (as by increasing the oxygen feed rate or by lowering the feed rate of one or more other constituents). The lowered

incidence of OH moieties is believed to result from increasing the degree of reaction of the oxygen feed with the hydrogen in the silicone source to yield more volatile water in the PECVD exhaust and a lower concentration of OH moieties trapped or incorporated in the coating.

VII. Higher energy can be applied in the PECVD process, either by raising the plasma generation power level, by applying the power for a longer period, or both. An increase in the applied energy must be employed with care when used to coat a plastic tube or other device, as it also has a tendency to distort the vessel being treated, to the extent the tube absorbs the plasma generation power. This is why RF power is contemplated in the context of present application. Distortion of the medical devices can be reduced or eliminated by employing the energy in a series of two or more pulses separated by cooling time, by cooling the vessels while applying energy, by applying the coating in a shorter time (commonly thus making it thinner), by selecting a frequency of the applied coating that is absorbed minimally by the base material selected for being coated, and/or by applying more than one coating, with time in between the respective energy application steps. For example, high power pulsing can be used with a duty cycle of 1 millisecond on, 99 milliseconds off, while continuing to feed the process gas. The process gas is then the coolant, as it keeps flowing between pulses. Another alternative is to reconfigure the power applicator, as by adding magnets to confine the plasma increase the effective power application (the power that actually results in incremental coating, as opposed to waste power that results in heating or unwanted coating). This expedient results in the application of more coating-formation energy per total Watt-hour of energy applied. See for example U.S. Patent 5,904,952.

VII. An oxygen post-treatment of the coating can be applied to remove OH moieties from the previously-deposited coating. This treatment is also contemplated to remove residual volatile organosilicon compounds or silicones or oxidize the coating to form additional $SiO_x$.

VII. The plastic base material tube can be preheated.

VII. A different volatile source of silicon, such as hexamethyldisilazane (HMDZ), can be used as part or all of the silicone feed. It is contemplated that changing the feed gas to HMDZ will address the problem because this compound has no oxygen moieties in it, as supplied. It is contemplated that one source of OH moieties in the HMDSO-sourced coating is hydrogenation of at least some of the oxygen atoms present in unreacted HMDSO.

VII. A composite coating can be used, such as a carbon-based coating combined with $SiO_x$. This can be done, for example, by changing the reaction conditions or by adding a substituted or unsubstituted hydrocarbon, such as an alkane, alkene, or alkyne, to the feed gas as well as an organosilicon-based compound. See for example U.S. Patent 5,904,952, which states in relevant part: "For example, inclusion of a lower hydrocarbon such as propylene provides carbon moieties and improves most properties of the deposited films (except for light transmission), and bonding analysis indicates the film to be silicon dioxide in nature. Use of methane, methanol, or acetylene, however, produces films that are silicone in nature. The inclusion of a minor amount of gaseous nitrogen to the gas stream provides nitrogen moieties in the deposited films and increases the deposition rate, improves the transmission and reflection optical properties on glass, and varies the index of refraction in response to varied amounts of $N_2$. The addition of nitrous oxide to the gas stream increases the deposition rate and improves the optical properties, but tends to decrease the film hardness." Suitable hydrocarbons include methane, ethane, ethylene, propane, acetylene, or a combination of two or more of these.

VII. A diamond-like carbon (DLC) coating can be formed as the primary or sole coating deposited. This can be done, for example, by changing the reaction conditions or by feeding methane, hydrogen, and helium to a PECVD process. These reaction feeds have no oxygen, so no OH moieties can be formed. For one example, an $SiO_x$ coating can be applied on the interior of a tube or syringe barrel and an outer DLC coating can be applied on the exterior surface of a tube or syringe barrel. Or, the $SiO_x$ and DLC coatings can both be applied as a single layer or plural layers of an interior tube or syringe barrel coating.

VII. Referring to FIG. 2, the barrier or other type of coating 90 reduces the transmission of atmospheric gases into the vessel 80 through its interior surface 88. Or, the barrier or other type of coating 90 reduces the contact of the contents of the vessel 80 with the interior surface 88. The barrier or other type of coating can comprise, for example, $SiO_x$, amorphous (for example, diamond-like) carbon, or a combination of these.

VII. Any coating described herein can be used for coating a surface, for example a plastic surface. It can further be used as a barrier layer, for example as a barrier against a gas or liquid, optionally against water vapor, oxygen and/or air. It can also be used for preventing or reducing mechanical and/or chemical effects which the coated

surface would have on a compound or composition if the surface were uncoated. For example, it can prevent or reduce the precipitation of a compound or composition, for example insulin precipitation or blood clotting or platelet activation.

**VII.A. Coated Vessels**

The coatings described herein can be applied to a variety of vessels made from plastic or glass, most prominently to plastic tubes and syringes. A process is contemplated for applying a lubricity layer or coating on a substrate, for example the interior of the barrel of a syringe, comprising applying one of the described precursors on or in the vicinity of a substrate at a thickness of 1 to 5000 nm, or 10 to 1000 nm, or 10 to 500 nm, or 10 to 200 nm, or 20 to 100 nm, or 30 to 1000 nm, or 30 to 500 nm thick, or 30 to 1000 nm, or 20 to 100 nm, or 80 to 150 nm, and crosslinking or polymerizing (or both) the coating, optionally in a PECVD process, to provide a lubricated surface. The coating applied by this process is also contemplated to be new.

A coating of $Si_wO_xC_yH_z$ as defined in the Definition Section can have utility as a hydrophobic layer. Coatings of this kind are contemplated to be hydrophobic, independent of whether they function as lubricity layers. A coating or treatment is defined as "hydrophobic" if it lowers the wetting tension of a surface, compared to the corresponding uncoated or untreated surface. Hydrophobicity is thus a function of both the untreated substrate and the treatment.

The degree of hydrophobicity of a coating can be varied by varying its composition, properties, or deposition method. For example, a coating of SiOx having little or no hydrocarbon content is more hydrophilic than a coating of $Si_wO_xC_yH_z$ as defined in the Definition Section. Generally speaking, the higher the C-$H_x$ (e.g. CH, $CH_2$, or $CH_3$) moiety content of the coating, either by weight, volume, or molarity, relative to its silicon content, the more hydrophobic the coating.

A hydrophobic layer or coating can be very thin, having a thickness of at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The coating can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Specific thickness ranges composed of any one of the minimum thicknesses expressed above, plus any equal or greater one of the maximum thicknesses expressed above, are expressly contemplated.

One utility for such a hydrophobic layer or coating is to isolate a thermoplastic tube wall, made for example of polyethylene terephthalate (PET), from blood collected within the tube. The hydrophobic layer or coating can be applied on top of a hydrophilic $SiO_x$ coating on the internal surface of the tube. The $SiO_x$ coating increases the barrier properties of the thermoplastic tube and the hydrophobic layer or coating changes the surface energy of blood contact surface with the tube wall. The hydrophobic layer or coating can be made by providing a precursor selected from those identified in this specification. For example, the hydrophobic layer or coating precursor can comprise hexamethyldisiloxane (HMDSO) or octamethylcyclotetrasiloxane (OMCTS).

Another use for a hydrophobic layer or coating is to prepare a glass cell preparation tube. The tube has a wall defining a lumen, a hydrophobic layer or coating in the internal surface of the glass wall, and contains a citrate reagent. The hydrophobic layer or coating can be made by providing a precursor selected from those identified elsewhere in this specification. For another example, the hydrophobic layer or coating precursor can comprise hexamethyldisiloxane (HMDSO) or octamethylcyclotetrasiloxane (OMCTS). Another source material for hydrophobic layers is an alkyl trimethoxysilane of the formula:

$$R\text{-}Si(OCH_3)_3$$

in which R is a hydrogen atom or an organic substituent, for example methyl, ethyl, propyl, isopropyl, butyl, isobutyl, t-butyl, vinyl, alkyne, epoxide, or others. Combinations of two or more of these are also contemplated.

Combinations of acid or base catalysis and heating, using an alkyl trimethoxysilane precursor as described above, can condense the precursor (removing ROH by-products) to form crosslinked polymers, which can optionally be further crosslinked via an alternative method. One specific example is by Shimojima et. al. J. Mater. Chem., 2007, 17, 658 - 663.

A lubricity layer can be applied as a subsequent coating after applying an $SiO_x$ barrier coating to the interior surface 88 of the vessel 80 to provide a lubricity layer, particularly if the lubricity layer or coating is a liquid organosiloxane compound at the end of the coating process.

Optionally, after the lubricity layer or coating is applied, it can be post-cured after the PECVD process. Radiation curing approaches, including UV-initiated (free radial or cationic), electron-beam (E-beam), and thermal as described in Development Of Novel Cycloaliphatic Siloxanes For Thermal And UV-Curable Applications (Ruby Chakraborty Dissertation, 2008) can be utilized.

Another approach for providing a lubricity layer or coating is to use a silicone demolding agent when injection-molding the thermoplastic vessel to be lubricated. For example, it is contemplated that any of the demolding agents and latent monomers causing in-situ thermal lubricity layer or coating formation during the molding process can be used. Or, the aforementioned monomers can be doped into traditional demolding agents to accomplish the same result.

A lubricity layer, is particularly contemplated for the internal surface of a syringe barrel as further described below. A lubricated internal surface of a syringe barrel can reduce the plunger sliding force needed to advance a plunger in the barrel during operation of a syringe, or the breakout force to start a plunger moving after the prefilled syringe plunger has pushed away the intervening lubricant or adhered to the barrel, for example due to decomposition of the lubricant between the plunger and the barrel. As explained elsewhere in this specification, a lubricity layer or coating also can be applied to the interior surface 88 of the vessel 80 to improve adhesion of a subsequent coating of $SiO_x$.

Thus, the coating 90 can comprise a layer or coating of $SiO_x$ and a lubricity layer or coating and/or hydrophobic layer, characterized as defined in the Definition Section. The lubricity layer or coating and/or hydrophobic layer or coating of $Si_wO_xC_yH_z$ can be deposited between the layer or coating of $SiO_x$ and the interior surface of the vessel. Or, the layer or coating of $SiO_x$ can be deposited between the lubricity layer or coating and/or hydrophobic layer or coating and the interior surface of the vessel. Or, three or more layers, either alternating or graduated between these two coating compositions: (1) a layer or coating of $SiO_x$ and (2) the lubricity layer or coating and/or hydrophobic layer; can also be used. The layer or coating of $SiO_x$ can be deposited adjacent to the lubricity layer or coating and/or hydrophobic layer or coating or remotely, with at least one intervening layer or coating of another material. The layer or coating of $SiO_x$ can be deposited adjacent to the interior surface of the vessel. Or, the lubricity layer or coating and/or hydrophobic layer or coating can be deposited adjacent to the interior surface of the vessel.

Another expedient contemplated here, for adjacent layers of $SiO_x$ and a lubricity layer or coating and/or hydrophobic layer, is a graded composite of $Si_wO_xC_yH_z$, as defined in the Definition Section. A graded composite can be separate layers of a lubricity layer or coating and/or hydrophobic layer or coating and $SiO_x$ with a transition or interface of intermediate composition between them, or separate layers of a lubricity layer or coating and/or hydrophobic layer or coating and $SiO_x$ with an intermediate distinct layer or coating of intermediate composition between them, or a single layer or coating that changes continuously or in steps from a composition of a lubricity layer or coating and/or hydrophobic layer or coating to a composition more like $SiO_x$, going through the coating in a normal direction.

The grade in the graded composite can go in either direction. For example, the a lubricity layer or coating and/or hydrophobic layer or coating can be applied directly to the substrate and graduate to a composition further from the surface of $SiO_x$. Or, the composition of $SiO_x$ can be applied directly to the substrate and graduate to a composition further from the surface of a lubricity layer or coating and/or hydrophobic layer. A graduated coating is particularly contemplated if a coating of one composition is better for adhering to the substrate than the other, in which case the better-adhering composition can, for example, be applied directly to the substrate. It is contemplated that the more distant portions of the graded coating can be less compatible with the substrate than the adjacent portions of the graded coating, since at any point the coating is changing gradually in properties, so adjacent portions at nearly the same depth of the coating have nearly identical composition, and more widely physically separated portions at substantially different depths can have more diverse properties. It is also contemplated that a coating portion that forms a better barrier against transfer of material to or from the substrate can be directly against the substrate, to prevent the more remote coating portion that forms a poorer barrier from being contaminated with the material intended to be barred or impeded by the barrier.

The coating, instead of being graded, optionally can have sharp transitions between one layer or coating and the next, without a substantial gradient of composition. Such coatings can be made, for example, by providing the gases to produce a layer or coating as a steady state flow in a non-plasma state, then energizing the system with a brief plasma discharge to form a coating on the substrate. If a subsequent coating is to be applied, the gases for the previous coating are cleared out and the gases for the next coating are applied in a steady-state fashion before energizing the plasma and again forming a distinct layer or coating on the surface of the substrate or its outermost previous coating, with little if any gradual transition at the interface.

**VII.A.1.a. Exemplary Vessels**

VII.A.1.a. Referring to FIG. 2, more details of the vessel such as 80 are shown. The illustrated vessel 80 can be generally tubular, having an opening 82 at one end of the vessel, opposed by a closed end 84. The vessel 80 also has a wall 86 defining an interior surface 88. One example of the vessel 80 is a medical sample tube, such as an evacuated blood collection tube, as commonly is used by a phlebotomist for receiving a venipuncture sample of a patient's blood for use in a medical laboratory.

VII.A.1.a. The vessel 80 can be made, for example, of thermoplastic material. Some examples of suitable thermoplastic material are polyethylene terephthalate or a polyolefin such as polypropylene or a cyclic polyolefin copolymer.
VII.A.1.a. The vessel 80 can be made by any suitable method, such as by injection molding, by blow molding, by machining, by fabrication from tubing stock, or by other suitable means. PECVD can be used to form a coating on the internal surface of $SiO_x$.
VII.A.1.a. If intended for use as an evacuated blood collection tube, the vessel 80 desirably can be strong enough to withstand a substantially total internal vacuum substantially without deformation when exposed to an external pressure of 760 Torr or atmospheric pressure and other coating processing conditions. This property can be provided, in a thermoplastic vessel 80, by providing a vessel 80 made of suitable materials having suitable dimensions and a glass transition temperature higher than the processing temperature of the coating process, for example a cylindrical wall 86 having sufficient wall thickness for its diameter and material.
VII.A.1.a. Medical vessels or containers like sample collection tubes and syringes are relatively small and are injection molded with relatively thick walls, which renders them able to be evacuated without being crushed by the ambient atmospheric pressure. They are thus stronger than carbonated soft drink bottles or other larger or thinner-walled plastic containers. Since sample collection tubes designed for use as evacuated vessels typically are constructed to withstand a full vacuum during storage, they can be used as vacuum chambers.
VII.A.1.a. Such adaptation of the vessels to be their own vacuum chambers might eliminate the need to place the vessels into a vacuum chamber for PECVD treatment, which typically is carried out at very low pressure. The use of a vessel as its own vacuum chamber can result in faster processing time (since loading and unloading of the parts from a separate vacuum chamber is not necessary) and can lead to simplified equipment configurations. Furthermore, a vessel holder is contemplated, for certain embodiments, that will hold the device (for alignment to gas tubes and other apparatus), seal the device (so that the vacuum can be created by attaching the vessel holder to a vacuum pump) and move the device between molding and subsequent processing steps.
VII.A.1.a. A vessel 80 used as an evacuated blood collection tube should be able to withstand external atmospheric pressure, while internally evacuated to a reduced pressure useful for the intended application, without a substantial volume of air or other atmospheric gas leaking into the tube (as by bypassing the closure) or permeating through the wall 86 during its shelf life. If the as-molded vessel 80 cannot meet this requirement, it can be processed by coating the interior surface 88 with a barrier or other type of coating 90. It is desirable to treat and/or coat the interior surfaces of these devices (such as sample collection tubes and syringe barrels) to impart various properties that will offer advantages over existing polymeric devices and/or to mimic existing glass products. It is also desirable to measure various properties of the devices before and/or after treatment or coating.

Further exemplary vessels are the syringes described herein.

### VII.A.1.b. Vessel Having Wall Coated With Hydrophobic coating

VII.A.1.b. Another embodiment is a vessel having a wall provided with a hydrophobic layer or coating on its inside surface and containing an aqueous sodium citrate reagent. The hydrophobic layer or coating can be also be applied on top of a hydrophilic $SiO_x$ coating on the internal surface of the vessel. The $SiO_x$ coating increases the barrier properties of the plastic vessel and the hydrophobic layer or coating changes the surface energy of the contact surface of the composition or compound inside the vessel with the vessel wall.
VII.A.1.b. The wall is made of thermoplastic material having an internal surface defining a lumen.
VII.A.1.b. A vessel according to the embodiment VII.A.1.b can have a first layer or coating of $SiO_x$ on the internal surface of the tube, applied as explained in this specification, to function as an oxygen barrier and extend the shelf life of an evacuated blood collection tube made of thermoplastic material. A second layer or coating of a hydrophobic layer, characterized as defined in the Definition Section, can then be applied over the barrier layer or coating on the internal surface of the vessel to provide a hydrophobic surface. In a blood collection tube or syringe, the coating optionally is effective to reduce the platelet activation of blood plasma treated with a sodium citrate additive and exposed to the inner surface, compared to the same type of wall uncoated.
VII.A.1.b. PECVD is used to form a hydrophobic layer or coating on the internal surface. Unlike conventional citrate blood collection tubes, a blood collection tube having a hydrophobic layer as defined herein does not require a coating of baked on silicone on the vessel wall, as is conventionally applied to make the surface of the tube hydrophobic.
VII.A.1.b. Both layers can be applied using the same precursor, for example HMDSO or OMCTS, and different PECVD reaction conditions.
VII.A.1.b. When preparing a blood collection tube or syringe, a sodium citrate anticoagulation reagent may then be placed within the tube and it is evacuated and sealed with a closure to produce an evacuated blood collection tube.

The components and formulation of the reagent are known to those skilled in the art. The aqueous sodium citrate reagent is disposed in the lumen of the tube in an amount effective to inhibit coagulation of blood introduced into the tube.

**VII.A.1.c. $SiO_x$ Barrier Coated Double Wall Plastic Vessel- COC, PET, $SiO_x$ layers**

VII.A.1.c. Another embodiment is a vessel having a wall at least partially enclosing a lumen. The wall has an interior polymer layer or coating enclosed by an exterior polymer layer. One of the polymer layers is a layer or coating at least 0.1 mm thick of a cyclic olefin copolymer (COC) resin defining a water vapor barrier. Another of the polymer layers is a layer or coating at least 0.1 mm thick of a polyester resin.
VII.A.1.c. The wall includes an oxygen barrier layer or coating of $SiO_x$ having a thickness of from about 10 to about 500 angstroms.
VII.A.1.c. In an embodiment, illustrated in FIG. 10, the vessel 80 can be a double-walled vessel having an inner wall 408 and an outer wall 410, respectively made of the same or different materials. One particular embodiment of this type can be made with one wall molded from a cyclic olefin copolymer (COC) and the other wall molded from a polyester such as polyethylene terephthalate (PET), with an $SiO_x$ coating as previously described on the interior surface. As needed, a tie coating or layer or coating can be inserted between the inner and outer walls to promote adhesion between them. An advantage of this wall construction is that walls having different properties can be combined to form a composite having the respective properties of each wall.
VII.A.1.c. As one example, the inner wall 408 can be made of PET coated on the interior surface with an $SiO_x$ barrier layer, and the outer wall 410 can be made of COC. PET coated with $SiO_x$, as shown elsewhere in this specification, is an excellent oxygen barrier, while COC is an excellent barrier for water vapor, providing a low water vapor transition rate (WVTR). This composite vessel can have superior barrier properties for both oxygen and water vapor. This construction is contemplated, for example, for an evacuated medical sample collection tube that contains an aqueous reagent as manufactured, and has a substantial shelf life, so it should have a barrier preventing transfer of water vapor outward or transfer of oxygen or other gases inward through its composite wall during its shelf life.
VII.A.1.c. As another example, the inner wall 408 can be made of COC coated on the interior surface with an $SiO_x$ barrier layer, and the outer wall 410 can be made of PET. This construction is contemplated, for example, for a prefilled syringe that contains an aqueous sterile fluid as manufactured. The $SiO_x$ barrier will prevent oxygen from entering the syringe through its wall. The COC inner wall will prevent ingress or egress of other materials such as water, thus preventing the water in the aqueous sterile fluid from leaching materials from the wall material into the syringe. The COC inner wall is also contemplated to prevent water derived from the aqueous sterile fluid from passing out of the syringe (thus undesirably concentrating the aqueous sterile fluid), and will prevent non-sterile water or other fluids outside the syringe from entering through the syringe wall and causing the contents to become non-sterile. The COC inner wall is also contemplated to be useful for decreasing the breakout force or friction of the plunger against the inner wall of a syringe.

**VII.A.1.d. Method of Making Double Wall Plastic Vessel- COC, PET, $SiO_x$ Layers**

VII.A.1.d. Another embodiment is a method of making a vessel having a wall having an interior polymer layer or coating enclosed by an exterior polymer layer, one layer or coating made of COC and the other made of polyester. The vessel is made by a process including introducing COC and polyester resin layers into an injection mold through concentric injection nozzles.
VII.A.1.d. An optional additional step is applying an amorphous carbon coating to the vessel by PECVD, as an inside coating, an outside coating, or as an interlayer or coating coating located between the layers.
VII.A.1.d. An optional additional step is applying an $SiO_x$ barrier layer or coating to the inside of the vessel wall, where $SiO_x$ is defined as before. Another optional additional step is post-treating the $SiO_x$ layer or coating with a gaseous reactant or process gas consisting essentially of oxygen and essentially free of a volatile silicon compound.
VII.A.1.d. Optionally, the $SiO_x$ coating can be formed at least partially from a silazane feed gas.
VII.A.1.d. The vessel 80 shown in FIG. 10 can be made from the inside out, for one example, by injection molding the inner wall in a first mold cavity, then removing the core and molded inner wall from the first mold cavity to a second, larger mold cavity, then injection molding the outer wall against the inner wall in the second mold cavity. Optionally, a tie layer or coating can be provided to the exterior surface of the molded inner wall before over-molding the outer wall onto the tie layer.

VII.A.1.d. Or, the vessel 80 shown in FIG. 10 can be made from the outside in, for one example, by inserting a first core in the mold cavity, injection molding the outer wall in the mold cavity, then removing the first core from the molded first wall and inserting a second, smaller core, then injection molding the inner wall against the outer wall still residing in the mold cavity. Optionally, a tie layer or coating can be provided to the interior surface of the molded outer wall before over-molding the inner wall onto the tie layer.

VII.A.1.d. Or, the vessel 80 shown in FIG. 10 can be made in a two shot mold. This can be done, for one example, by injection molding material for the inner wall from an inner nozzle and the material for the outer wall from a concentric outer nozzle. Optionally, a tie layer or coating can be provided from a third, concentric nozzle disposed between the inner and outer nozzles. The nozzles can feed the respective wall materials simultaneously. One useful expedient is to begin feeding the outer wall material through the outer nozzle slightly before feeding the inner wall material through the inner nozzle. If there is an intermediate concentric nozzle, the order of flow can begin with the outer nozzle and continue in sequence from the intermediate nozzle and then from the inner nozzle. Or, the order of beginning feeding can start from the inside nozzle and work outward, in reverse order compared to the preceding description.

**VII.A.1.e. Vessel or Coating Made Of Glass**

VII.A.1.e. Another embodiment is a vessel including a vessel, a barrier coating, and a closure. The vessel is generally tubular and made of thermoplastic material. The vessel has a mouth and a lumen bounded at least in part by a wall having an inner surface interfacing with the lumen. There is an at least essentially continuous barrier coating made of glass on the inner surface of the wall. A closure covers the mouth and isolates the lumen of the vessel from ambient air.

VII.A.1.e. The vessel 80 can also be made, for example of glass of any type used in medical or laboratory applications, such as soda-lime glass, borosilicate glass, or other glass formulations. Other vessels having any shape or size, made of any material, are also contemplated for use in the system 20. One function of coating a glass vessel can be to reduce the ingress of ions in the glass, either intentionally or as impurities, for example sodium, calcium, or others, from the glass to the contents of the vessel, such as a reagent or blood in an evacuated blood collection tube. Another function of coating a glass vessel in whole or in part, such as selectively at surfaces contacted in sliding relation to other parts, is to provide lubricity to the coating, for example to ease the insertion or removal of a stopper or passage of a sliding element such as a piston in a syringe. Still another reason to coat a glass vessel is to prevent a reagent or intended sample for the vessel, such as blood, from sticking to the wall of the vessel or an increase in the rate of coagulation of the blood in contact with the wall of the vessel.

VII.A.1.e.i. A related embodiment is a vessel as described in the previous paragraph, in which the barrier coating is made of soda lime glass, borosilicate glass, or another type of glass.

**VII.B. Syringes**

VII.B. The foregoing description has largely addressed applying a barrier coating to a tube with one permanently closed end, such as a blood collection tube or, more generally, a specimen receiving tube 80. The apparatus is not limited to such a device.

VII.B. Another example of a suitable vessel, shown in FIG. 3, is a syringe barrel 250 for a medical syringe 252. Such syringes 252 are sometimes supplied prefilled with saline solution, a pharmaceutical preparation, or the like for use in medical techniques. Pre-filled syringes 252 are also contemplated to benefit from an $SiO_x$ barrier or other type of coating on the interior surface 254 to keep the contents of the prefilled syringe 252 out of contact with the plastic of the syringe, for example of the syringe barrel 250 during storage. The barrier or other type of coating can be used to avoid leaching components of the plastic into the contents of the barrel through the interior surface 254.

VII.B. A syringe barrel 250 as molded commonly can be open at both the back end 256, to receive a plunger 258, and at the front end 260, to receive a hypodermic needle, a nozzle, or tubing for dispensing the contents of the syringe 252 or for receiving material into the syringe 252. But the front end 260 can optionally be capped and the plunger 258 optionally can be fitted in place before the prefilled syringe 252 is used, closing the barrel 250 at both ends. A cap 262 can be installed either for the purpose of processing the syringe barrel 250 or assembled syringe, or to remain in place during storage of the prefilled syringe 252, up to the time the cap 262 is removed and (optionally)

a hypodermic needle or other delivery conduit is fitted on the front end 260 to prepare the syringe 252 for use.

Another suitable vessel is the "staked needle syringe" described in PCT/US11/36097 filed on 11 May 2011 and in US61/359,434, filed June 29, 2010, i.e. a syringe barrel with an affixed ("staked") hollow needle.

**VII.B. Syringes**

VII.B. The foregoing description has largely addressed applying a barrier coating to a tube with one permanently closed end, such as a blood collection tube or, more generally, a specimen receiving tube 80. The apparatus is not limited to such a device.

VII.B. Another example of a suitable vessel, shown in FIG. 3, is a syringe barrel 250 for a medical syringe 252. Such syringes 252 are sometimes supplied prefilled with saline solution, a pharmaceutical preparation, or the like for use in medical techniques. Pre-filled syringes 252 are also contemplated to benefit from an $SiO_x$ barrier or other type of coating on the interior surface 254 to keep the contents of the prefilled syringe 252 out of contact with the plastic of the syringe, for example of the syringe barrel 250 during storage. The barrier or other type of coating can be used to avoid leaching components of the plastic into the contents of the barrel through the interior surface 254.

VII.B. A syringe barrel 250 as molded commonly can be open at both the back end 256, to receive a plunger 258, and at the front end 260, to receive a hypodermic needle, a nozzle, or tubing for dispensing the contents of the syringe 252 or for receiving material into the syringe 252. But the front end 260 can optionally be capped and the plunger 258 optionally can be fitted in place before the prefilled syringe 252 is used, closing the barrel 250 at both ends. A cap 262 can be installed either for the purpose of processing the syringe barrel 250 or assembled syringe, or to remain in place during storage of the prefilled syringe 252, up to the time the cap 262 is removed and (optionally) a hypodermic needle or other delivery conduit is fitted on the front end 260 to prepare the syringe 252 for use.

Another suitable syringe is the "staked needle syringe" described in PCT/US11/36097 filed on 11 May 2011 and in US 61/359,434 filed on June 29, 2010, i.e. a syringe barrel with an affixed ("staked") hollow needle.

Typically, when the syringe barrel is coated, the PECVD coating methods described herein are performed such that the coated substrate surface is part or all of the inner surface of the barrel, the gas for the PECVD reaction fills the interior lumen of the barrel, and the plasma is generated within part or all of the interior lumen of the barrel.

**VII.B.1.a. Syringe Having Barrel Coated With Lubricity Layer**

VII.B.1.a. A syringe having a lubricity layer of the type can be made by the following process.

VII.B.1.a. A precursor is provided as defined above.

VII.B.1.a. The precursor is applied to a substrate under conditions effective to form a coating. The coating is polymerized or crosslinked, or both, to form a lubricated surface having a lower plunger sliding force or breakout force than the untreated substrate.

VII.B.1.a. Respecting any of the Embodiments VII and sub-parts, optionally the applying step is carried out by vaporizing the precursor and providing it in the vicinity of the substrate.

VII.B.1.a. A plasma, is formed in the vicinity of the substrate. Optionally, the precursor is provided in the substantial absence of nitrogen. Optionally, the precursor is provided at less than 1 Torr absolute pressure. Optionally, the precursor is provided to the vicinity of a plasma emission. Optionally, the precursor its reaction product is applied to the substrate at an average thickness of 1 to 5000 nm, or 10 to 1000 nm, or 10 to 500 nm, or 10 to 200 nm, or 20 to 100 nm, or 30 to 1000 nm, or 30 to 500 nm, or 30 to 1000 nm, or 20 to 100 nm, or 80 to 150 nm thick. Optionally, the substrate comprises glass. Optionally, the substrate comprises a polymer, optionally a polycarbonate polymer, optionally an olefin polymer, optionally a cyclic olefin copolymer, optionally a polypropylene polymer, optionally a polyester polymer, optionally a polyethylene terephthalate polymer. COC is particularly considered for syringes and syringe barrels.

VII.B.1.a. Optionally, the plasma is generated by energizing the gaseous reactant containing the precursor with electrodes powered, for example, at a RF frequency as defined above, for example a frequency of from 10 kHz to less than 300 MHz, optionally from 1 to 50 MHz, even optionally from 10 to 15 MHz, optionally a frequency of 13.56 MHz.

VII.B.1.a. Optionally, the plasma is generated by energizing the gaseous reactant containing the precursor with electrodes supplied with an electric power of from 0.1 to 25 W, optionally from 1 to 22 W, optionally from 3 to 17 W,

even optionally from 5 to 14 W, optionally from 7 to 11 W, optionally 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally is from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity layers to syringes and sample tubes and vessels of similar geometry having a void volume of 1 to 3 mL in which PECVD plasma is generated. It is contemplated that for larger or smaller objects the power applied should be increased or reduced accordingly to scale the process to the size of the substrate.

VII.B.1.a. Another embodiment is a lubricity coating of the present invention on the inner wall of a syringe barrel. The coating is produced from a PECVD process using the following materials and conditions. A cyclic precursor is optionally employed, selected from a monocyclic siloxane, a polycyclic siloxane, or a combination of two or more of these, as defined elsewhere in this specification for lubricity layers. One example of a suitable cyclic precursor comprises octamethylcyclotetrasiloxane (OMCTS), optionally mixed with other precursor materials in any proportion. Optionally, the cyclic precursor consists essentially of octamethycyclotetrasiloxane (OMCTS), meaning that other precursors can be present in amounts which do not change the basic and novel properties of the resulting lubricity layer, i.e. its reduction of the plunger sliding force or breakout force of the coated surface.

VII.B.1.a. A sufficient plasma generation power input, for example any power level successfully used in one or more working examples of this specification or described in the specification, is provided to induce coating formation.

VII.B.1.a. The materials and conditions employed are effective to reduce the syringe plunger sliding force or breakout force moving through the syringe barrel at least 25 percent, alternatively at least 45 percent, alternatively at least 60 percent, alternatively greater than 60 percent, relative to an uncoated syringe barrel. Ranges of plunger sliding force or breakout force reduction of from 20 to 95 percent, alternatively from 30 to 80 percent, alternatively from 40 to 75 percent, alternatively from 60 to 70 percent, are contemplated.

VII.B.1.a. Another embodiment is a vessel having a hydrophobic layer, characterized as defined in the Definition Section, on the inside wall. The coating is made as explained for the lubricant coating of similar composition, but under conditions effective to form a hydrophobic surface having a higher contact angle than the untreated substrate.

VII.B.1.a. Optionally the substrate comprises glass or a polymer. The glass optionally is borosilicate glass. The polymer is optionally a polycarbonate polymer, optionally an olefin polymer, optionally a cyclic olefin copolymer, optionally a polypropylene polymer, optionally a polyester polymer, optionally a polyethylene terephthalate polymer.

VII.B.1.a. Another embodiment is a syringe including a plunger, a syringe barrel, and a lubricity layer. The syringe barrel includes an interior surface receiving the plunger for sliding. The lubricity layer or coating is disposed on part or all of the interior surface of the syringe barrel. The lubricity layer or coating optionally can be less than 1000 nm thick and effective to reduce the breakout force or the plunger sliding force necessary to move the plunger within the barrel. Reducing the plunger sliding force is alternatively expressed as reducing the coefficient of sliding friction of the plunger within the barrel or reducing the plunger force; these terms are regarded as having the same meaning in this specification.

VII.B.1.a. The syringe comprises a plunger and a syringe barrel. The syringe barrel has an interior surface receiving the plunger for sliding. The interior surface of the syringe barrel further comprises a lubricity layer or coating. The lubricity layer or coating is less than 1000 nm thick, optionally less than 500 nm thick, optionally less than 200 nm thick, optionally less than 100 nm thick, optionally less than 50 nm thick, and is effective to reduce the breakout force necessary to overcome adhesion of the plunger after storage or the plunger sliding force necessary to move the plunger within the barrel after it has broken away. The lubricity layer or coating is characterized by having a plunger sliding force or breakout force lower than that of the uncoated surface.

VII.B.1.a. Any of the above precursors of any type can be used alone or in combinations of two or more of them to provide a lubricity layer.

VII.B.1.a. In addition to utilizing vacuum processes, low temperature atmospheric (non-vacuum) plasma processes can also be utilized to induce molecular ionization and deposition through precursor monomer vapor delivery optionally in a non-oxidizing atmosphere such as helium or argon. Separately, thermal CVD can be considered via flash thermolysis deposition.

VII.B.1.a. The approaches above are similar to vacuum PECVD in that the surface coating and crosslinking mechanisms can occur simultaneously.

VII.B.1.a. Yet another expedient contemplated for any coating or coatings described here is a coating that is not uniformly applied over the entire interior 88 of a vessel. For example, a different or additional coating can be applied selectively to the cylindrical portion of the vessel interior, compared to the hemispherical portion of the vessel interior at its closed end 84, or vice versa. This expedient is particularly contemplated for a syringe barrel or a sample collection tube as described below, in which a lubricity layer or coating might be provided on part or all of the cylindrical portion of the barrel, where the plunger or piston or closure slides, and not elsewhere.

VII.B.1.a. Optionally, the precursor can be provided in the presence, substantial absence, or absence of nitrogen.

In one contemplated embodiment, the precursor alone is delivered to the substrate and subjected to PECVD to apply and cure the coating.

VII.B.1.a. Optionally, the precursor can be provided at less than 1 Torr absolute pressure.

VII.B.1.a. Optionally, the precursor can be provided to the vicinity of a plasma emission.

VII.B.1.a. In any of the above embodiments, the substrate can comprise glass, or a polymer, for example one or more of a polycarbonate polymer, an olefin polymer (for example a cyclic olefin copolymer or a polypropylene polymer), or a polyester polymer (for example, a polyethylene terephthalate polymer).

VII.B.1.a. In any of the above embodiments, the plasma is generated by energizing the gaseous reactant containing the precursor with electrodes powered at a RF frequency as defined in this description.

VII.B.1.a. In any of the above embodiments, the plasma is generated by energizing the gaseous reactant containing the precursor with electrodes supplied with sufficient electric power to generate a lubricity layer. Optionally, the plasma is generated by energizing the gaseous reactant containing the precursor with electrodes supplied with an electric power of from 0.1 to 25 W, optionally from 1 to 22 W, optionally from 3 to 17 W, even optionally from 5 to 14 W, optionally from 7 to 11 W, optionally 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity layers to syringes and sample tubes and vessels of similar geometry having a void volume of 1 to 3 mL in which PECVD plasma is generated. It is contemplated that for larger or smaller objects the power applied should be increased or reduced accordingly to scale the process to the size of the substrate.

VII.B.1.a. The coating can be cured, as by polymerizing or crosslinking the coating, or both, to form a lubricated surface having a lower plunger sliding force or breakout force than the untreated substrate. Curing can occur during the application process such as PECVD, or can be carried out or at least completed by separate processing.

VII.B.1.a. Although plasma deposition has been used herein to demonstrate the coating characteristics, alternate deposition methods can be used as long as the chemical composition of the starting material is preserved as much as possible while still depositing a solid film that is adhered to the base substrate.

VII.B.1.a. For example, the coating material can be applied onto the syringe barrel (from the liquid state) by spraying the coating or dipping the substrate into the coating, where the coating is either the neat precursor a solvent-diluted precursor (allowing the mechanical deposition of a thinner coating). The coating optionally can be crosslinked using thermal energy, UV energy, electron beam energy, plasma energy, or any combination of these.

VII.B.1.a. Application of a silicone precursor as described above onto a surface followed by a separate curing step is also contemplated. The conditions of application and curing can be analogous to those used for the atmospheric plasma curing of pre-coated polyfluoroalkyl ethers, a process practiced under the trademark TriboGlide®. More details of this process can be found at http://www.triboglide.com/process.htm.

VII.B.1.a. In such a process, the area of the part to be coated can optionally be pre-treated with an atmospheric plasma. This pretreatment cleans and activates the surface so that it is receptive to the lubricant that is sprayed in the next step.

VII.B.1.a. The lubrication fluid, in this case one of the above precursors or a polymerized precursor, is then sprayed on to the surface to be treated. For example, IVEK precision dispensing technology can be used to accurately atomize the fluid and create a uniform coating.

VII.B.1.a. The coating is then bonded or crosslinked to the part, again using an atmospheric plasma field. This both immobilizes the coating and improves the lubricant's performance.

VII.B.1.a. Optionally, the atmospheric plasma can be generated from ambient air in the vessel, in which case no gas feed and no vacuum drawing equipment is needed. Optionally, however, the vessel is at least substantially closed while plasma is generated, to minimize the power requirement and prevent contact of the plasma with surfaces or materials outside the vessel.

**VII.B.1.a.i. Lubricity layer: $SiO_x$ Barrier, Lubricity Layer, Surface Treatment**

**Surface treatment**

VII.B.1.a.i. Another embodiment is a syringe comprising a barrel defining a lumen and having an interior surface slidably receiving a plunger, i.e. receiving a plunger for sliding contact to the interior surface.

VII.B.1.a.i. The syringe barrel is made of thermoplastic base material.

VII.B.1.a.i. Optionally, the interior surface of the barrel is coated with an $SiO_x$ barrier layer or coating as described

elsewhere in this specification.

VII.B.1.a.i. A lubricity layer or coating is applied to part or all the barrel interior surface, the plunger, or both, or to the previously applied $SiO_x$ barrier layer. The lubricity layer or coating can be provided, applied, and cured as set out in embodiment VII.B.1.a or elsewhere in this specification.

VII.B.1.a.i. For example, the lubricity layer or coating can be applied, in any embodiment, by PECVD. The lubricity layer or coating is deposited from an organosilicon precursor, and is less than 1000 nm thick.

VII.B.1.a.i. A surface treatment is carried out on the lubricity layer or coating in an amount effective to reduce the leaching or extractables of the lubricity layer, the thermoplastic base material, or both. The treated surface can thus act as a solute retainer. This surface treatment can result in a skin coating, e.g. a skin coating which is at least 1 nm thick and less than 100 nm thick, or less than 50 nm thick, or less than 40 nm thick, or less than 30 nm thick, or less than 20 nm thick, or less than 10 nm thick, or less than 5 nm thick, or less than 3 nm thick, or less than 2 nm thick, or less than 1 nm thick, or less than 0.5 nm thick.

VII.B.1.a.i. As used herein, "leaching" refers to material transferred out of a substrate, such as a vessel wall, into the contents of a vessel, for example a syringe. Commonly, leachables are measured by storing the vessel filled with intended contents, then analyzing the contents to determine what material leached from the vessel wall into the intended contents. "Extraction" refers to material removed from a substrate by introducing a solvent or dispersion medium other than the intended contents of the vessel, to determine what material can be removed from the substrate into the extraction medium under the conditions of the test.

VII.B.1.a.i. The surface treatment resulting in a solute retainer optionally can be a $SiO_x$ layer or coating as previously defined in this specification or a hydrophobic layer, characterized as defined in the Definition Section. In one embodiment, the surface treatment can be applied by PECVD deposit of $SiO_x$ or a hydrophobic layer. Optionally, the surface treatment can be applied using higher power or stronger oxidation conditions than used for creating the lubricity layer, or both, thus providing a harder, thinner, continuous solute retainer. Surface treatment can be less than 100 nm deep, optionally less than 50 nm deep, optionally less than 40 nm deep, optionally less than 30 nm deep, optionally less than 20 nm deep, optionally less than 10 nm deep, optionally less than 5 nm deep, optionally less than 3 nm deep, optionally less than 1 nm deep, optionally less than 0.5 nm deep, optionally between 0.1 and 50 nm deep in the lubricity layer.

VII.B.1.a.i. The solute retainer is contemplated to provide low solute leaching performance to the underlying lubricity and other layers, including the substrate, as required. This retainer would only need to be a solute retainer to large solute molecules and oligomers (for example siloxane monomers such as HMDSO, OMCTS, their fragments and mobile oligomers derived from lubricants, for example a "leachables retainer") and not a gas ($O_2$/$N_2$/$CO_2$/water vapor) barrier layer. A solute retainer can, however, also be a gas barrier (e.g. the SiOx coating according to present invention. One can create a good leachable retainer without gas barrier performance, either by vacuum or atmospheric-based PECVD processes. It is desirable that the "leachables barrier" will be sufficiently thin that, upon syringe plunger movement, the plunger will readily penetrate the "solute retainer" exposing the sliding plunger nipple to the lubricity layer or coating immediately below to form a lubricated surface having a lower plunger sliding force or breakout force than the untreated substrate.

VII.B.1.a.i. In another embodiment, the surface treatment can be performed by oxidizing the surface of a previously applied lubricity layer, as by exposing the surface to oxygen in a plasma environment. The plasma environment described in this specification for forming $SiO_x$ coatings can be used. Or, atmospheric plasma conditions can be employed in an oxygen-rich environment.

VII.B.1.a.i. The lubricity layer or coating and solute retainer, however formed, optionally can be cured at the same time. In another embodiment, the lubricity layer or coating can be at least partially cured, optionally fully cured, after which the surface treatment can be provided, applied, and the solute retainer can be cured.

VII.B.1.a.i. The lubricity layer or coating and solute retainer are composed, and present in relative amounts, effective to provide a breakout force, plunger sliding force, or both that is less than the corresponding force required in the absence of the lubricity layer or coating and surface treatment. In other words, the thickness and composition of the solute retainer are such as to reduce the leaching of material from the lubricity layer or coating into the contents of the syringe, while allowing the underlying lubricity layer or coating to lubricate the plunger. It is contemplated that the solute retainer will break away easily and be thin enough that the lubricity layer or coating will still function to lubricate the plunger when it is moved.

VII.B.1.a.i. In one contemplated embodiment, the lubricity and surface treatments can be applied on the barrel interior surface. In another contemplated embodiment, the lubricity and surface treatments can be applied on the plunger. In still another contemplated embodiment, the lubricity and surface treatments can be applied both on the barrel interior surface and on the plunger. In any of these embodiments, the optional $SiO_x$ barrier layer or coating on the interior of the syringe barrel can either be present or absent.

VII.B.1.a.i. One embodiment contemplated is a plural-layer, e.g. a 3-layer, configuration applied to the inside surface of a syringe barrel. Layer or coating 1 can be an $SiO_x$ gas barrier made by PECVD of HMDSO, OMCTS, or both,

in an oxidizing atmosphere. Such an atmosphere can be provided, for example, by feeding HMDSO and oxygen gas to a PECVD coating apparatus as described in this specification. Layer or coating 2 can be a lubricity layer or coating using OMCTS applied in a non-oxidizing atmosphere. Such a non-oxidizing atmosphere can be provided, for example, by feeding OMCTS to a PECVD coating apparatus as described in this specification, optionally in the substantial or complete absence of oxygen. A subsequent solute retainer can be formed by a treatment forming a thin skin layer or coating of $SiO_x$ or a hydrophobic layer or coating as a solute retainer using higher power and oxygen using OMCTS and/or HMDSO.

VII.B.1.a.i. Certain of these plural-layer or coating coatings are contemplated to have one or more of the following optional advantages, at least to some degree. They can address the reported difficulty of handling silicone, since the solute retainer can confine the interior silicone and prevent if from migrating into the contents of the syringe or elsewhere, resulting in fewer silicone particles in the deliverable contents of the syringe and less opportunity for interaction between the lubricity layer or coating and the contents of the syringe. They can also address the issue of migration of the lubricity layer or coating away from the point of lubrication, improving the lubricity of the interface between the syringe barrel and the plunger. For example, the break-free force can be reduced and the drag on the moving plunger can be reduced, or optionally both.

VII.B.1.a.i. It is contemplated that when the solute retainer is broken, the solute retainer will continue to adhere to the lubricity layer or coating and the syringe barrel, which can inhibit any particles from being entrained in the deliverable contents of the syringe.

VII.B.1.a.i. Certain of these coatings will also provide manufacturing advantages, particularly if the barrier coating, lubricity layer or coating and surface treatment are applied in the same apparatus, for example the illustrated PECVD apparatus. Optionally, the $SiO_x$ barrier coating, lubricity layer, and surface treatment can all be applied in one PECVD apparatus, thus greatly reducing the amount of handling necessary.

Further advantages can be obtained by forming the barrier coating, lubricity layer, and solute retainer using the same precursors and varying the process. For example, an $SiO_x$ gas barrier layer or coating can be applied using an OMCTS precursor under high power/high $O_2$ conditions, followed by applying a lubricity layer or coating applied using an OMCTS precursor under low power and/or in the substantial or complete absence of oxygen, finishing with a surface treatment using an OMCTS precursor under intermediate power and oxygen.

### VII.B.1.b Syringe having barrel with $SiO_x$ coated interior and barrier coated exterior

VII.B.1.b. Still another embodiment is a syringe including a plunger, a barrel, and interior and exterior barrier coatings. The barrel can be made of thermoplastic base material defining a lumen. The barrel can have an interior surface receiving the plunger for sliding and an exterior surface. A barrier coating of $SiO_x$, in which x is from about 1.5 to about 2.9, can be provided on the interior surface of the barrel. A barrier coating of a resin can be provided on the exterior surface of the barrel.

VII.B.1.b. In any embodiment, the thermoplastic base material optionally can include a polyolefin, for example polypropylene or a cyclic olefin copolymer (for example the material sold under the trademark TOPAS®), a polyester, for example polyethylene terephthalate, a polycarbonate, for example a bisphenol A polycarbonate thermoplastic, or other materials. Composite syringe barrels are contemplated having any one of these materials as an outer layer and the same or a different one of these materials as an inner layer. Any of the material combinations of the composite syringe barrels or sample tubes described elsewhere in this specification can also be used.

VII.B.1.b. In any embodiment, the resin optionally can include polyvinylidene chloride in homopolymer or copolymer form. For example, the PVdC homopolymers (trivial name: Saran) or copolymers described in US Patent 6,165,566, incorporated here by reference, can be employed. The resin optionally can be applied onto the exterior surface of the barrel in the form of a latex or other dispersion.

VII.B.1.b. In any embodiment, the syringe barrel optionally can include a lubricity layer disposed between the plunger and the barrier coating of $SiO_x$. Suitable lubricity layers are described elsewhere in this specification.

VII.B.1.b. In any embodiment, the lubricity layer optionally can be applied by PECVD and optionally can include material characterized as defined in the Definition Section.

VII.B.1.b. In any embodiment, the syringe barrel optionally can include a surface treatment covering the lubricity

layer in an amount effective to reduce the leaching of the lubricity layer, constituents of the thermoplastic base material, or both into the lumen.

**VII.B.1.c Method of Making Syringe having barrel with $SiO_x$ coated interior and barrier coated exterior**

VII.B.1.c. Even another embodiment is a method of making a syringe as described in any of the embodiments of part VII.B.1.b, including a plunger, a barrel, and interior and exterior barrier coatings. A barrel is provided having an interior surface for receiving the plunger for sliding and an exterior surface. A barrier coating of $SiO_x$ is provided on the interior surface of the barrel by PECVD. A barrier coating of a resin is provided on the exterior surface of the barrel. The plunger and barrel are assembled to provide a syringe.

VII.B.1.c. For effective coating (uniform wetting) of the plastic article with the aqueous latex, it is contemplated to be useful to match the surface tension of the latex to the plastic substrate. This can be accomplished by several approaches, independently or combined, for example, reducing the surface tension of the latex (with surfactants or solvents), and/or corona pretreatment of the plastic article, and/or chemical priming of the plastic article.

VII.B.1.c. The resin optionally can be applied via dip coating of the latex onto the exterior surface of the barrel, spray coating of the latex onto the exterior surface of the barrel, or both, providing plastic-based articles offering improved gas and vapor barrier performance. Polyvinylidene chloride plastic laminate articles can be made that provide significantly improved gas barrier performance versus the non-laminated plastic article.

VII.B.1.c. In any embodiment, the resin optionally can be heat cured. The resin optionally can be cured by removing water. Water can be removed by heat curing the resin, exposing the resin to a partial vacuum or low-humidity environment, catalytically curing the resin, or other expedients.

VII.B.1.c. An effective thermal cure schedule is contemplated to provide final drying to permit PVdC crystallization, offering barrier performance. Primary curing can be carried out at an elevated temperature, for example between 180-310°F (82-154°C), of course depending on the heat tolerance of the thermoplastic base material. Barrier performance after the primary cure optionally can be about 85% of the ultimate barrier performance achieved after a final cure.

VII.B.1.c. A final cure can be carried out at temperatures ranging from ambient temperature, such as about 65-75°F (18-24°C) for a long time (such as 2 weeks) to an elevated temperature, such as 122°F (50°C), for a short time, such as four hours.

VII.B.1.c. The PVdC-plastic laminate articles, in addition to superior barrier performance, are optionally contemplated to provide one or more desirable properties such as colorless transparency, good gloss, abrasion resistance, printability, and mechanical strain resistance.

**VII.B.2. Plungers**

**VII.B.2.a. With Barrier Coated Piston Front Face**

VII.B.2.a. Another embodiment is a plunger for a syringe, including a piston and a push rod. The piston has a front face, a generally cylindrical side face, and a back portion, the side face being configured to movably seat within a syringe barrel. The front face has a barrier coating. The push rod engages the back portion and is configured for advancing the piston in a syringe barrel.

**VII.B.2.b. With Lubricity layer Interfacing With Side Face**

VII.B.2.b. Yet another embodiment is a plunger for a syringe, including a piston, a lubricity layer, and a push rod. The piston has a front face, a generally cylindrical side face, and a back portion. The side face is configured to movably seat within a syringe barrel. The lubricity layer interfaces with the side face. The push rod engages the back portion of the piston and is configured for advancing the piston in a syringe barrel.

**VII.B.3.a Two Piece Syringe and Luer Fitting**

VII.B.3.a Another embodiment is a syringe including a plunger, a syringe barrel, and a Luer fitting. The syringe includes a barrel having an interior surface receiving the plunger for sliding. The Luer fitting includes a Luer taper having an internal passage defined by an internal surface. The Luer fitting is formed as a separate piece from the syringe barrel and joined to the syringe barrel by a coupling. The internal passage of the Luer taper optionally has a barrier coating of $SiO_x$.

**VII.B.3.b Staked Needle Syringe**

VII.B.3.b Another embodiment is a syringe including a plunger, a syringe barrel, and a staked needle (a "staked needle syringe") as described for example in PCT/US11/36097 filed on 11 May 2011 and in US 61/359,434 filed on June 29, 2010. The needle is hollow with a typical size ranging from 18-29 gauge. The syringe barrel has an interior surface slidably receiving the plunger. The staked needle may be affixed to the syringe during the injection molding of the syringe or may be assembled to the formed syringe using an adhesive. A cover is placed over the staked needle to seal the syringe assembly. The syringe assembly must be sealed so that a vacuum can be maintained within the syringe to enable the PECVD coating process.

**VII.B.4. Lubricity layer or coating In general**

**VII.B.4.a. Product By Process and Lubricity**

VII.B.4.a. Still another embodiment is a lubricity layer. This coating can be of the type made by the process for preparing a lubricity coating as described herein.

VII.B.4.a. Any of the precursors for lubricity coatings mentioned elsewhere in this specification can be used, alone or in combination. The precursor is applied to a substrate under conditions effective to form a coating. The coating is polymerized or crosslinked, or both, to form a lubricated surface having a lower plunger sliding force or breakout force than the untreated substrate.

VII.B.4.a. Another embodiment is a method of applying a lubricity layer. An organosilicon precursor is applied to a substrate under conditions effective to form a coating. The coating is polymerized or crosslinked, or both, to form a lubricated surface having a lower plunger sliding force or breakout force than the untreated substrate.

**VII.B.4.b. Product by Process and Analytical Properties**

VII.B.4.b. Even another aspect of the invention is a lubricity layer or coating deposited by PECVD from a feed gas comprising an organometallic precursor, optionally an organosilicon precursor, optionally a linear siloxane, a linear silazane, a monocyclic siloxane, a monocyclic silazane, a polycyclic siloxane, a polycyclic silazane, or any combination of two or more of these. The coating can have a density between 1.25 and 1.65 g/cm$^3$ optionally between 1.35 and 1.55 g/cm$^3$, optionally between 1.4 and 1.5 g/cm$^3$, optionally between 1.44 and 1.48 g/cm$^3$ as determined by X-ray reflectivity (XRR).

VII.B.4.b. Still another aspect of the invention is a lubricity layer or coating deposited by PECVD from a feed gas comprising an organometallic precursor, optionally an organosilicon precursor, optionally a linear siloxane, a linear silazane, a monocyclic siloxane, a monocyclic silazane, a polycyclic siloxane, a polycyclic silazane, or any combination of two or more of these. The coating has as an outgas component one or more oligomers containing repeating $-(Me)_2SiO-$ moieties, as determined by gas chromatography / mass spectrometry. Optionally, the coating meets the limitations of any of embodiments VII.B.4.a. Optionally, the coating outgas component as determined by gas chromatography / mass spectrometry is substantially free of trimethylsilanol.

VII.B.4.b. Optionally, the coating outgas component can be at least 10 ng/test of oligomers containing repeating $-(Me)_2SiO-$ moieties, as determined by gas chromatography / mass spectrometry using the following test conditions:

- GC Column: 30m X 0.25mm DB-5MS (J&W Scientific), 0.25μm film thickness
- Flow rate: 1.0 ml/min, constant flow mode
- Detector: Mass Selective Detector (MSD)
- Injection Mode: Split injection (10:1 split ratio)
- Outgassing Conditions: 1½" (37mm) Chamber, purge for three hour at 85°C, flow 60 ml/min
- Oven temperature: 40°C (5 min.) to 300°C at 10°C/min.; hold for 5 min. at 300°C.

VII.B.4.b. Optionally, the outgas component can include at least 20 ng/test of oligomers containing repeating -$(Me)_2SiO$- moieties.

VII.B.4.b. Optionally, the feed gas comprises a monocyclic siloxane, a monocyclic silazane, a polycyclic siloxane, a polycyclic silazane, or any combination of two or more of these, for example a monocyclic siloxane, a monocyclic silazane, or any combination of two or more of these, for example octamethylcyclotetrasiloxane.

VII.B.4.b. The lubricity layer or coating of any embodiment can have an average thickness measured by transmission electron microscopy (TEM) of from 1 to 5000 nm, or 10 to 1000 nm, or 10 to 200 nm, or 20 to 100 nm, or 30 to 1000 nm, or 30 to 500 nm thick. Preferred ranges are from 30 to 1000 nm and from 20 to 100 nm, and a particularly preferred range is from 80 to 150 nm. The absolute thickness of the coating at single measurement points can be higher or lower than the range limits of the average thickness. However, it typically varies within the thickness ranges given for the average thickness.

VII.B.4.b. Another aspect of the invention is a lubricity layer or coating deposited by PECVD from a feed gas comprising a monocyclic siloxane, a monocyclic silazane, a polycyclic siloxane, a polycyclic silazane, or any combination of two or more of these. The coating has an atomic concentration of carbon, normalized to 100% of carbon, oxygen, and silicon, as determined by X-ray photoelectron spectroscopy (XPS), greater than the atomic concentration of carbon in the atomic formula for the feed gas. Optionally, the coating meets the limitations of embodiments VII.B.4.a or VII.B.4.b.A.

VII.B.4.b. Optionally, the atomic concentration of carbon increases by from 1 to 80 atomic percent (as calculated and based on the XPS conditions in Example 15 of EP 2 251 455), alternatively from 10 to 70 atomic percent, alternatively from 20 to 60 atomic percent, alternatively from 30 to 50 atomic percent, alternatively from 35 to 45 atomic percent, alternatively from 37 to 41 atomic percent in relation to the atomic concentration of carbon in the organosilicon precursor when a lubricity coating is made.

VII.B.4.b. An additional aspect of the invention is a lubricity layer or coating deposited by PECVD from a feed gas comprising a monocyclic siloxane, a monocyclic silazane, a polycyclic siloxane, a polycyclic silazane, or any combination of two or more of these. The coating has an atomic concentration of silicon, normalized to 100% of carbon, oxygen, and silicon, as determined by X-ray photoelectron spectroscopy (XPS), less than the atomic concentration of silicon in the atomic formula for the feed gas. See Example 15 of EP 2 251 455.

VII.B.4.b. Optionally, the atomic concentration of silicon decreases by from 1 to 80 atomic percent (as calculated and based on the XPS conditions in Example 15 of EP 2251 455), alternatively from 10 to 70 atomic percent, alternatively from 20 to 60 atomic percent, alternatively from 30 to 55 atomic percent, alternatively from 40 to 50 atomic percent, alternatively from 42 to 46 atomic percent.

VII.B.4.b. Lubricity layers having combinations of any two or more properties recited in Section VII.B.4 are also expressly contemplated.

### VII.C. Vessels Generally

VII.C. A coated vessel or container as described herein and/or prepared according to a method described herein can be used for reception and/or storage and/or delivery of a compound or composition. The compound or composition can be sensitive, for example air-sensitive, oxygen-sensitive, sensitive to humidity and/or sensitive to mechanical influences. It can be a biologically active compound or composition, for example a medicament like insulin or a composition comprising insulin. In another aspect, it can be a biological fluid, optionally a bodily fluid, for example blood or a blood fraction. In certain aspects of the present invention, the compound or composition is a product to be administrated to a subject in need thereof, for example a product to be injected, like blood (as in transfusion of blood from a donor to a recipient or reintroduction of blood from a patient back to the patient) or insulin.

VII.C. A coated vessel or container as described herein and/or prepared according to a method described herein can further be used for protecting a compound or composition contained in its interior space against mechanical and/or chemical effects of the surface of the uncoated vessel material. For example, it can be used for preventing or reducing precipitation and/or clotting or platelet activation of the compound or a component of the composition, for example insulin precipitation or blood clotting or platelet activation.

VII.C. It can further be used for protecting a compound or composition contained in its interior against the environment outside of the vessel, for example by preventing or reducing the entry of one or more compounds from the environment surrounding the vessel into the interior space of the vessel. Such environmental compound can be a gas or liquid, for example an atmospheric gas or liquid containing oxygen, air, and/or water vapor.

VII.C. A coated vessel as described herein can also be evacuated and stored in an evacuated state. For example, the coating allows better maintenance of the vacuum in comparison to a corresponding uncoated vessel. In one aspect of this embodiment, the coated vessel is a blood collection tube. The tube can also contain an agent for preventing blood clotting or platelet activation, for example EDTA or heparin.

VII.C. Any of the above-described embodiments can be made, for example, by providing as the vessel a length of tubing from about 1 cm to about 200 cm, optionally from about 1 cm to about 150 cm, optionally from about 1 cm to about 120 cm, optionally from about 1 cm to about 100 cm, optionally from about 1 cm to about 80 cm, optionally from about 1 cm to about 60 cm, optionally from about 1 cm to about 40 cm, optionally from about 1 cm to about 30 cm long, and processing it with a probe electrode as described below. Particularly for the longer lengths in the above ranges, it is contemplated that relative motion between the probe and the vessel can be useful during coating formation. This can be done, for example, by moving the vessel with respect to the probe or moving the probe with respect to the vessel.

VII.C. In these embodiments, it is contemplated that the coating can be thinner or less complete than can be preferred for a barrier coating, as the vessel in some embodiments will not require the high barrier integrity of an evacuated blood collection tube.

VII.C. As an optional feature of any of the foregoing embodiments the vessel has a central axis.

VII.C. As an optional feature of any of the foregoing embodiments the vessel wall is sufficiently flexible to be flexed at least once at 20°C, without breaking the wall, over a range from at least substantially straight to a bending radius at the central axis of not more than 100 times as great as the outer diameter of the vessel.

VII.C. As an optional feature of any of the foregoing embodiments the bending radius at the central axis is not more than 90 times as great as, or not more than 80 times as great as, or not more than 70 times as great as, or not more than 60 times as great as, or not more than 50 times as great as, or not more than 40 times as great as, or not more than 30 times as great as, or not more than 20 times as great as, or not more than 10 times as great as, or not more than 9 times as great as, or not more than 8 times as great as, or not more than 7 times as great as, or not more than 6 times as great as, or not more than 5 times as great as, or not more than 4 times as great as, or not more than 3 times as great as, or not more than 2 times as great as, or not more than, the outer diameter of the vessel.

VII.C. As an optional feature of any of the foregoing embodiments the vessel wall can be a fluid-contacting surface made of flexible material.

VII.C. As an optional feature of any of the foregoing embodiments the vessel lumen can be the fluid flow passage of a pump.

VII.C. As an optional feature of any of the foregoing embodiments the vessel can be a blood bag adapted to maintain blood in good condition for medical use.

VII.C., VII.D. As an optional feature of any of the foregoing embodiments the polymeric material can be a silicone elastomer or a thermoplastic polyurethane, as two examples, or any material suitable for contact with blood, or with insulin.

VII.C., VII.D. In an optional embodiment, the vessel has an inner diameter of at least 2 mm, or at least 4 mm.

VII.C. As an optional feature of any of the foregoing embodiments the vessel is a tube.

VII.C. As an optional feature of any of the foregoing embodiments the lumen has at least two open ends.

## COMMON CONDITIONS FOR ALL EMBODIMENTS

In any embodiment contemplated here, many common conditions can be used, for example any of the following, in any combination. Alternatively, any different conditions described elsewhere in this specification or claims can be employed.

## I. SUBSTRATE OF ANY EMBODIMENT

### I.A. Vessel of any embodiment

The vessel can be a sample collection tube, for example a blood collection tube, or a syringe, or a syringe part, for example a barrel or piston or plunger; a vial; a conduit; or a cuvette. The substrate can be a closed-ended tube, for example a medical sample collection tube. The substrate can be the inside wall of a vessel having a lumen, the lumen having a void volume of from 0.5 to 50 mL, optionally from 1 to 10 mL, optionally from 0.5 to 5 mL, optionally from 1 to 3 mL. The substrate surface can be part or all of the inner surface of a vessel having at least one opening and an inner surface, and wherein the gaseous reactant fills the interior lumen of the vessel and the plasma can be generated in part or all of the interior lumen of the vessel.

### I.B. Syringe and parts

The substrate can be a syringe barrel. The syringe barrel can have a plunger sliding surface and the coating can be disposed on at least a portion of the plunger sliding surface. The coating can be a lubricity layer. The lubricity layer or coating can be on the barrel interior surface. The lubricity layer or coating can be on the plunger. In a particular

aspect, the substrate is a staked needle syringe or part of a staked needle syringe.

**I.C. Vessel to receive Stopper**

The substrate can be a stopper receiving surface in the mouth of a vessel. The substrate can be a generally conical or cylindrical inner surface of an opening of a vessel adapted to receive a stopper.

**I.D. Stopper**

The substrate can be a sliding surface of a stopper. The substrates can be coated by providing a multiplicity of the stoppers located in a single substantially evacuated vessel. The chemical vapor deposition can be plasma-enhanced chemical vapor deposition and the stopper can be contacted with the plasma. The chemical vapor deposition can be plasma-enhanced chemical vapor deposition. The plasma can be formed upstream of the stopper, producing plasma product, and the plasma product can be contacted with the stopper.

A closure can define a substrate coated with a coating, optionally a stopper coated with a lubricity layer. The substrate can be a closure seated in a vessel defining a lumen and a surface of the closure facing the lumen can be coated with the coating.

The coating can be effective to reduce the transmission of a metal ion constituent of the stopper into the lumen of the vessel.

**I.E. The substrate of any embodiment**

The substrate can be a vessel wall. A portion of the vessel wall in contact with a wall-contacting surface of a closure can be coated with the coating. The coating can be a composite of material having first and second layers. The first layer or coating can interface with the elastomeric stopper. The first layer of the coating can be effective to reduce the transmission of one or more constituents of the stopper into the vessel lumen. The second layer or coating can interface with the inner wall of the vessel. The second layer can be effective to reduce friction between the stopper and the inner wall of the vessel when the stopper can be seated on the vessel.

Alternatively, the first and second layers of any embodiment can be defined by a coating of graduated properties containing carbon and hydrogen, in which the proportions of carbon and hydrogen are greater in the first layer or coating than in the second layer.

The coating of any embodiment can be applied by plasma enhanced chemical vapor deposition.

The substrate of any embodiment can comprise glass, alternatively a polymer, alternatively a polycarbonate polymer, alternatively an olefin polymer, alternatively a cyclic olefin copolymer, alternatively a polypropylene polymer, alternatively a polyester polymer, alternatively a polyethylene terephthalate polymer, alternatively a polyethylene naphthalate polymer, alternatively a combination, composite or blend of any two or more of the above materials.

**II. GASEOUS REACTANT OR PROCESS GAS LIMITATIONS OF ANY EMBODIMENT**

**II.A Deposition conditions of any embodiment**

The plasma for PECVD, if used, can be generated at reduced pressure and the reduced pressure can be less than 300 mTorr, optionally less than 200 mTorr, even optionally less than 100 mTorr. The physical and chemical properties of the coating can be set by setting the ratio of $O_2$ to the organosilicon precursor in the gaseous reactant, and/or by setting the electric power used for generating the plasma.

**II.C. Precursor of any embodiment**

The organosilicon precursor has been described elsewhere in this description.

The organosilicon compound can in certain aspects, particularly when a lubricity coating is formed, comprise octamethylcyclotetrasiloxane (OMCTS). The organosilicon compound for any embodiment of said certain aspects can consist essentially of octamethycyclotetrasiloxane (OMCTS). The organosilicon compound can in certain aspects, particularly when a barrier coating is formed, be or comprise hexamethyldisiloxane.

The reaction gas can also include a hydrocarbon. The hydrocarbon can comprise methane, ethane, ethylene, propane, acetylene, or a combination of two or more of these.

The organosilicon precursor can be delivered at a rate of equal to or less than 6 sccm, optionally equal to or less than 2.5 sccm, optionally equal to or less than 1.5 sccm, optionally equal to or less than 1.25 sccm. Larger vessels or other changes in conditions or scale may require more or less of the precursor. The precursor can be provided at

less than 1 Torr absolute pressure.

### II.D. Carrier gas of any embodiment

The carrier gas can comprise or consist of an inert gas, for example argon, helium, xenon, neon, another gas that is inert to the other constituents of the process gas under the deposition conditions, or any combination of two or more of these.

### II.E. Oxidizing gas of any embodiment

The oxidizing gas can comprise or consist of oxygen ($O_2$ and/or $O_3$ (commonly known as ozone)), nitrous oxide, or any other gas that oxidizes the precursor during PECVD at the conditions employed. The oxidizing gas comprises about 1 standard volume of oxygen. The gaseous reactant or process gas can be at least substantially free of nitrogen.

## III. PLASMA OF ANY EMBODIMENT

The plasma of any PECVD embodiment can be formed in the vicinity of the substrate. The plasma can in certain cases, especially when preparing an SiOx coating, be a non-hollow-cathode plasma. In other certain cases, especially when preparing a lubricity coating, a non-hollow-cathode plasma is not desired. The plasma can be formed from the gaseous reactant at reduced pressure. Sufficient plasma generation power input can be provided to induce coating formation on the substrate.

## IV. RF POWER OF ANY EMBODIMENT

The precursor can be contacted with a plasma made by energizing the vicinity of the precursor with electrodes powered at a frequency of 10 kHz to 2.45 GHz, alternatively from about 13 to about 14 MHz.

The precursor can be contacted with a plasma made by energizing the vicinity of the precursor with electrodes powered at radio frequency, optionally at a frequency of from 10 kHz to less than 300 MHz, optionally from 1 to 50 MHz, even optionally from 10 to 15 MHz, optionally at 13.56 MHz.

The precursor can be contacted with a plasma made by energizing the vicinity of the precursor with electrodes supplied with electric power at from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W.

The precursor can be contacted with a plasma made by energizing the vicinity of the precursor with electrodes supplied with electric power density at less than 10 W/ml of plasma volume, alternatively from 6 W/ml to 0.1 W/ml of plasma volume, alternatively from 5 W/ml to 0.1 W/ml of plasma volume, alternatively from 4 W/ml to 0.1 W/ml of plasma volume, alternatively from 2 W/ml to 0.2 W/ml of plasma volume.

The plasma can be formed by exciting the reaction mixture with electromagnetic energy, alternatively microwave energy.

## V. OTHER PROCESS OPTIONS OF ANY EMBODIMENT

The applying step for applying a coating to the substrate can be carried out by vaporizing the precursor and providing it in the vicinity of the substrate.

The chemical vapor deposition employed can be PECVD and the deposition time can be from 1 to 30 sec, alternatively from 2 to 10 sec, alternatively from 3 to 9 sec. The purposes for optionally limiting deposition time can be to avoid overheating the substrate, to increase the rate of production, and to reduce the use of process gas and its constituents. The purposes for optionally extending deposition time can be to provide a thicker coating for particular deposition conditions.

## VI. COATING PROPERTIES OF ANY EMBODIMENT

### VI.A. Lubricity properties of any embodiment

The vessels (e.g. syringe barrels and/or plungers) coated with a lubricity coating according to present invention have a higher lubricity (determined, e.g. by measuring the Fi and/or Fm) than the uncoated vessels. They also have a higher lubricity than vessels coated with a $SiO_x$ coating as described herein. An embodiment can be carried out under conditions effective to form a lubricated surface of the substrate having a lower sliding force or breakout force (or optionally

both) than the untreated substrate. Optionally, the materials and conditions can be effective to reduce the sliding force or breakout force at least at least 25 percent, alternatively at least 45 percent, alternatively at least 60 percent, alternatively more than 60 percent relative to an uncoated syringe barrel. Expressed otherwise, the coating can have a lower frictional resistance than the uncoated surface, wherein optionally the frictional resistance can be reduced by at least 25%, optionally by at least 45%, even optionally by at least 60% in comparison to the uncoated surface.

The break loose force (Fi) and the glide force (Fm) are important performance measures for the effectiveness of a lubricity coating. For Fi and Fm, it is desired to have a low, but not too low value. With too low Fi, which means a too low level of resistance (the extreme being zero), premature/unintended flow may occur, which might e.g. lead to an unintentional premature or uncontrolled discharge of the content of a prefilled syringe.

In order to achieve a sufficient lubricity (e.g. to ensure that a syringe plunger can be moved in the syringe, but to avoid uncontrolled movement of the plunger), the following ranges of Fi and Fm should be advantageously maintained:

Fi: 2.5 to 5 lbs, preferably 2.7 to 4.9 lbs, and in particular 2.9 to 4.7 lbs;
Fm: 2.5 to 8.0 lbs, preferably 3.3 to 7.6 lbs, and in particular 3.3 to 4 lbs.

Further advantageous Fi and Fm values can be found in the Tables of the Examples.

The lubricity coating optionally provides a consistent plunger force that reduces the difference between the break loose force (Fi) and the glide force (Fm).

### VI.B. Hydrophobicity properties of any embodiment

An embodiment can be carried out under conditions effective to form a hydrophobic layer or coating on the substrate. Optionally, the hydrophobic characteristics of the coating can be set by setting the ratio of the $O_2$ to the organosilicon precursor in the gaseous reactant, and/or by setting the electric power used for generating the plasma. Optionally, the coating can have a lower wetting tension than the uncoated surface, optionally a wetting tension of from 20 to 72 dyne/cm, optionally from 30 to 60 dynes/cm, optionally from 30 to 40 dynes/cm, optionally 34 dyne/cm. Optionally, the coating can be more hydrophobic than the uncoated surface.

### VI.C. Thickness of any embodiment

Optionally, the coating can have a thickness determined by transmission electron microscopy (TEM), of any amount stated in this disclosure.

For the lubricity coatings described herein, the indicated thickness ranges are representing average thickness, as a certain roughness may enhance the lubricious properties of the lubricity coating. Thus the thickness of the lubricity coating is advantageously not uniform throughout the coating (see above). However, a uniformly thick lubricity coating is also considered. The absolute thickness of the lubricity coating at single measurement points can be higher or lower than the range limits of the average thickness, with maximum deviations of preferably +/- 50%, more preferably +/- 25% and even more preferably +/- 15% from the average thickness. However, it typically varies within the thickness ranges given for the average thickness in this description.

### VI.D. Composition of any embodiment

Optionally, the lubricity coating can be composed of SiwOxCyHz or SiwNxCyHz. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

Alternatively, w can be 1, x can be from about 0.5 to 1.5 y can be from about 2 to about 3, and z can be from 6 to about 9. Alternatively, the coating can have atomic concentrations normalized to 100% carbon, oxygen, and silicon, as determined by X-ray photoelectron spectroscopy (XPS) of less than 50% carbon and more than 25% silicon. Alternatively, the atomic concentrations are from 25 to 45% carbon, 25 to 65% silicon, and 10 to 35% oxygen. Alternatively, the atomic concentrations are from 30 to 40% carbon, 32 to 52% silicon, and 20 to 27% oxygen. Alternatively, the atomic concentrations are from 33 to 37% carbon, 37 to 47% silicon, and 22 to 26% oxygen.

Optionally, the atomic concentration of carbon, normalized to 100% of carbon, oxygen, and silicon, as determined by X-ray photoelectron spectroscopy (XPS), can be greater than the atomic concentration of carbon in the atomic

formula for the organosilicon precursor. For example, embodiments are contemplated in which the atomic concentration of carbon increases by from 1 to 80 atomic percent, alternatively from 10 to 70 atomic percent, alternatively from 20 to 60 atomic percent, alternatively from 30 to 50 atomic percent, alternatively from 35 to 45 atomic percent, alternatively from 37 to 41 atomic percent.

Optionally, the atomic ratio of carbon to oxygen in the coating can be increased in comparison to the organosilicon precursor, and/or the atomic ratio of oxygen to silicon can be decreased in comparison to the organosilicon precursor.

Optionally, the coating can have an atomic concentration of silicon, normalized to 100% of carbon, oxygen, and silicon, as determined by X-ray photoelectron spectroscopy (XPS), less than the atomic concentration of silicon in the atomic formula for the feed gas. For example, embodiments are contemplated in which the atomic concentration of silicon decreases by from 1 to 80 atomic percent, alternatively by from 10 to 70 atomic percent, alternatively by from 20 to 60 atomic percent, alternatively by from 30 to 55 atomic percent, alternatively by from 40 to 50 atomic percent, alternatively by from 42 to 46 atomic percent.

As another option, a coating is contemplated that can be characterized by a sum formula wherein the atomic ratio C : O can be increased and/or the atomic ratio Si : O can be decreased in comparison to the sum formula of the organosilicon precursor.

**VI.E. Outgassing species of any embodiment**

The coating made from an organosilicon precursor, eg. The lubricity or SiOx barrier coating described herein, can have as an outgas component one or more oligomers containing repeating -$(Me)_2SiO$- moieties, as determined by gas chromatography / mass spectrometry. The coating outgas component can be determined by gas chromatography / mass spectrometry. For example, the coating outgas component can have at least 10 ng/test of oligomers containing repeating -(Me)2SiO- moieties, alternatively at least 20 ng/test of oligomers containing repeating -(Me)2SiO- moieties, as determined using the following test conditions:

- GC Column: 30m X 0.25mm DB-5MS (J&W Scientific), 0.25μm film thickness
- Flow rate 1.0 ml/min, constant flow mode
- Detector: Mass Selective Detector (MSD)
- Injection Mode: Split injection (10:1 split ratio)
- Outgassing Conditions: 1½" (37mm) Chamber, purge for three hour at 85°C, flow 60 ml/min
- Oven temperature: 40°C (5 min.) to 300°C @10°C/min.; hold for 5 min. at 300°C.

Optionally, the lubricity coating can have an outgas component at least substantially free of trimethylsilanol.

**VI.E. Other Coating Properties of any embodiment**

The coating can have a density between 1.25 and 1.65 g/cm$^3$, alternatively between 1.35 and 1.55 g/cm$^3$, alternatively between 1.4 and 1.5 g/cm$^3$, alternatively between 1.4 and 1.5 g/cm$^3$, alternatively between 1.44 and 1.48 g/cm$^3$, as determined by X-ray reflectivity (XRR). Optionally, the organosilicon compound can be octamethylcyclotetrasiloxane and the coating can have a density which can be higher than the density of a coating made from HMDSO as the organosilicon compound under the same PECVD reaction conditions.

The coating optionally can prevent or reduce the precipitation of a compound or component of a composition in contact with the coating, in particular can prevent or reduce insulin precipitation or blood clotting, in comparison to the uncoated surface and/or to a barrier coated surface using HMDSO as precursor.

The substrate can be a vessel, for protecting a compound or composition contained or received in the coated vessel against mechanical and/or chemical effects of the surface of the uncoated substrate.

The substrate can be a vessel, for preventing or reducing precipitation and/or clotting of a compound or a component of the composition in contact with the interior surface of the vessel. The compound or composition can be a biologically active compound or composition, for example a medicament, for example the compound or composition can comprise insulin, wherein insulin precipitation can be reduced or prevented. Alternatively, the compound or composition can be a biological fluid, for example a bodily fluid, for example blood or a blood fraction wherein blood clotting can be reduced or prevented.

## BASIC PROTOCOLS FOR FORMING, COATING, AND TESTING TUBES

The vessels tested in the subsequent working examples were formed and coated according to the following exemplary protocols, except as otherwise indicated in individual examples. Particular parameter values given in the following basic protocols, e.g. the electric power and process gas flow, are typical values. Whenever parameter values were changed in comparison to these typical values, this will be indicated in the subsequent working examples. The same applies to the type and composition of the process gas.

### Protocol for Forming COC Tube

Cyclic olefin copolymer (COC) tubes of the shape and size commonly used as evacuated blood collection tubes ("COC tubes") were injection molded from Topas® 8007-04 cyclic olefin copolymer (COC) resin, available from Hoechst AG, Frankfurt am Main, Germany, having these dimensions: 75 mm length, 13 mm outer diameter, and 0.85 mm wall thickness, each having a volume of about 7.25 $cm^3$ and a closed, rounded end.

### Protocol for Coating Tube Interior with $SiO_x$

The vessel holder 50 was made from Delrin® acetal resin, available from E.I. du Pont de Nemours and Co., Wilmington Delaware, USA, with an outside diameter of 1.75 inches (44 mm) and a height of 1.75 inches (44 mm). The vessel holder 50 was housed in a Delrin® structure that allowed the device to move in and out of the electrode (160).

The electrode 160 was made from copper with a Delrin® shield. The Delrin® shield was conformal around the outside of the copper electrode 160. The electrode 160 measured approximately 3 inches (76 mm) high (inside) and was approximately 0.75 inches (19 mm) wide.

The tube used as the vessel 80 was inserted into the vessel holder 50 base sealing with Viton® O-rings 490, 504 (Viton® is a trademark of DuPont Performance Elastomers LLC, Wilmington Delaware, USA) around the exterior of the tube (FIG. 11). The tube 80 was carefully moved into the sealing position over the extended (stationary) 1/8-inch (3-mm) diameter brass probe or counter electrode 108 and pushed against a copper plasma screen.

The copper plasma screen 610 was a perforated copper foil material (K&S Engineering, Chicago Illinois, USA, Part #LXMUW5 copper mesh) cut to fit the outside diameter of the tube, and was held in place by a radially extending abutment surface 494 that acted as a stop for the tube insertion (see FIG. 11). Two pieces of the copper mesh were fit snugly around the brass probe or counter electrode 108, insuring good electrical contact.

The brass probe or counter electrode 108 extended approximately 70 mm into the interior of the tube and had an array of #80 wire (diameter = 0.0135 inch or 0.343 mm). The brass probe or counter electrode 108 extended through a Swagelok® fitting (available from Swagelok Co., Solon Ohio, USA) located at the bottom of the vessel holder 50, extending through the vessel holder 50 base structure. The brass probe or counter electrode 108 was grounded to the casing of the RF matching network.

The gas delivery port 110 was 12 holes in the probe or counter electrode 108 along the length of the tube (three on each of four sides oriented 90 degrees from each other) and two holes in the aluminum cap that plugged the end of the gas delivery port 110. The gas delivery port 110 was connected to a stainless steel assembly comprised of Swagelok® fittings incorporating a manual ball valve for venting, a thermocouple pressure gauge and a bypass valve connected to the vacuum pumping line. In addition, the gas system was connected to the gas delivery port 110 allowing the process gases, oxygen and hexamethyldisiloxane (HMDSO) to be flowed through the gas delivery port 110 (under process pressures) into the interior of the tube.

The gas system was comprised of a Aalborg® GFC17 mass flow meter (Part # EW-32661-34, Cole-Parmer Instrument Co., Barrington Illinois USA) for controllably flowing oxygen at 70 sccm (or at the specific flow reported for a particular example) into the process and a polyether ether ketone ("PEEK") capillary (outside diameter, "OD" 1/16-inch (1.5-mm.), inside diameter, "ID" 0.004 inch (0.1 mm)) of length 52.5 inches (1.33 m). The PEEK capillary end was inserted into liquid hexamethyldisiloxane ("HMDSO," Alfa Aesar® Part Number L16970, NMR Grade, available from Johnson Matthey PLC, London). The liquid HMDSO was pulled through the capillary due to the lower pressure in the tube during processing. The HMDSO was then vaporized into a vapor at the exit of the capillary as it entered the low pressure region. The gas inlet pressure was 7.2 Torr.

To ensure no condensation of the liquid HMDSO past this point, the gas stream (including the oxygen) was diverted to the pumping line when it was not flowing into the interior of the tube for processing via a Swagelok® 3-way valve. Once the tube was installed, the vacuum pump valve was opened to the vessel holder 50 and the interior of the tube.

An Alcatel rotary vane vacuum pump and blower comprised the vacuum pump system. The pumping system allowed the interior of the tube to be reduced to pressure(s) of less than 200 mTorr while the process gases were flowing at the indicated rates.

Once the base vacuum level was achieved, the vessel holder 50 assembly was moved into the electrode 160

assembly. The gas stream (oxygen and HMDSO vapor) was flowed into the brass gas delivery port 110 (by adjusting the 3-way valve from the pumping line to the gas delivery port 110). Pressure inside the tube was approximately 300 mTorr as measured by a capacitance manometer (MKS) installed on the pumping line near the valve that controlled the vacuum. In addition to the tube pressure, the pressure inside the gas delivery port 110 and gas system was also measured with the thermocouple vacuum gauge that was connected to the gas system. This pressure was typically less than 8 Torr.

Once the gas was flowing to the interior of the tube, the RF power supply was turned on to its fixed power level. A ENI ACG-6 600 Watt RF power supply was used (at 13.56 MHz) at a fixed power level of approximately 70 Watts. The output power was calibrated in this and all following Protocols and Examples using a Bird Corporation Model 43 RF Watt meter connected to the RF output of the power supply during operation of the coating apparatus. The RF power supply was connected to a COMDEL CPMX1000 auto match which matched the complex impedance of the plasma (to be created in the tube) to the 50 ohm output impedance of the ENI ACG-6 RF power supply. The forward power was 70 Watts (or the specific amount reported for a particular example) and the reflected power was 0 Watts so that the applied power was delivered to the interior of the tube. The RF power supply was controlled by a laboratory timer and the power on time set to 6 seconds (or the specific time period reported for a particular example). Upon initiation of the RF power, a uniform plasma was established inside the interior of the tube. The plasma was maintained for the entire 6 seconds until the RF power was terminated by the timer. The plasma produced a silicon oxide coating of approximately 20 nm thickness (or the specific thickness reported in a particular example) on the interior of the tube surface.

After coating, the gas flow was diverted back to the vacuum line and the vacuum valve was closed. The vent valve was then opened, returning the interior of the tube to atmospheric pressure (approximately 760 Torr). The tube was then carefully removed from the vessel holder 50 assembly (after moving the vessel holder 50 assembly out of the electrode 160 assembly).

**Protocol For Charging Blood Tubes with $CO_2$**

Tubes to be charged with $CO_2$ were mounted in the vessel holder 50 described above and connected to the previously described Alcatel rotary vane vacuum pump and blower (comprising the vacuum pump system) and a source of $CO_2$. In some cases as indicated below the $CO_2$ charging was carried out in the coating apparatus immediately after applying the $SiO_x$ coating and clearing the process gases. In other cases the $SiO_x$ coating was applied, the tube was removed from the coating apparatus, and later installed on the $CO_2$ charging apparatus.

In either type of $CO_2$ charging as explained above, the vacuum pump system was first used to evacuate the tube to a pressure of 0.3 to 1 Torr. The vacuum was maintained for 3-30 sec. to evacuate process gases. The evacuated tube was then filled with $CO_2$ pressurized to 25 psi (1230 Torr) absolute pressure and maintained at that pressure for 1-14 minutes, or for the amount of time otherwise indicated in a working example. The charged tube was then transferred to the outgassing measurement apparatus described in the protocol below.

**Protocol for Measuring Outgassing**

VI.B. Present FIG. 7, adapted from FIG. 15 of U.S. Patent 6,584,828, is a schematic view of a test set-up that was used in a working example for measuring outgassing through an $SiO_x$ barrier coating 348 applied according to the Protocol for Coating Tube Interior with $SiO_x$ on the interior of the wall 346 of a COC tube 358 made according to the Protocol for Forming COC Tube seated with a seal 360 on the upstream end of a MicroFlow Technology measurement cell generally indicated at 362.

VI.B. A vacuum pump 364 was connected to the downstream end of a commercially available measurement cell 362 (an Intelligent Gas Leak System with Leak Test Instrument Model ME2, with a second generation IMFS sensor (either 2 or 14$\mu$/min full range, as indicated in a particular example), Absolute Pressure Sensor range: 0-10 Torr, Flow measurement uncertainty: +/- 5% of reading, at calibrated range, employing the Leak-Tek Program for automatic data acquisition (with PC) and signatures/plots of leak flow vs. time. This equipment is supplied by ATC Inc.), and was configured to draw gas from the interior of the vessel 358 in the direction of the arrows through the measurement cell 362 for determination of the mass flow rate outgassed vapor into the vessel 358 from its walls.

VI.B. The measurement cell 362 shown and described schematically here was understood to work substantially as follows, though this information might deviate somewhat from the operation of the equipment actually used and identified more specifically in the protocol by model number. The cell 362 has a conical passage 368 through which the outgassed flow is directed. The pressure is tapped at two longitudinally spaced lateral bores 370 and 372 along the passage 368 and fed respectively to the chambers 374 and 376 formed in part by the diaphragms 378 and 380.

The pressures accumulated in the respective chambers 374 and 376 deflect the respective diaphragms 378 and 380. These deflections are measured in a suitable manner, as by measuring the change in capacitance between conductive surfaces of the diaphragms 378 and 380 and nearby conductive surfaces such as 382 and 384. A bypass 386 can optionally be provided to speed up the initial pump-down by bypassing the measurement cell 362 until the desired vacuum level for carrying out the test is reached.

VI.B. The COC walls 350 of the vessels used in this test were 0.85 mm thick, and the coating 348 was on the order of 20 nm (nanometers) thick. Thus, the wall 350 to coating 348 thickness ratio was on the order of 50,000 : 1.

VI.B. To determine the flow rate through the measurement cell 362, including the vessel seal 360, a glass vessel substantially identical in size and construction to the vessel 358 was seated on the vessel seal 360, pumped down to an internal pressure of 1 Torr, then capacitance data was collected with the measurement cell 362 and converted to an "outgassing" flow rate. The test was carried out two times on each vessel. After the first run, the vacuum was released with nitrogen and the vessels were allowed recovery time to reach equilibrium before proceeding with another run, if any. Since a glass vessel is believed to have very little outgassing, and is essentially impermeable through its wall, this measurement is understood to be at least predominantly an indication of the amount of leakage of the vessel and connections within the measurement cell 362, and reflects little if any true outgassing or permeation.

VI.B. The family of plots or data table provided to show the outgassing results of certain examples shows the "outgas" flow rate, also in micrograms per minute, of individual tubes. Some of the flow rate is attributed to leakage. Numerical flow rate data points in tables are reported as of a certain test time (minutes per test on the ATC).

**WORKING EXAMPLES**

The working examples are to be understood to encompass the Examples referring to outgassing of EP2251671 A2, in particular Example 8, Example 16, Example 19, and the Figures and Tables to which these Examples of EP2251671 A2 refer.

**Example 1**

**Outgassing Measurement on $CO_2$ Charged, Uncoated COC Tubes**

VI.B. This initial test was carried out to determine whether $CO_2$ can be charged to COC to substantially increase its ATC outgas flow rate. Thirty uncoated COC tubes were made according to the Protocol for Forming COC Tube. Before $CO_2$ charging, the tubes were tested for outgassing following the Protocol For Testing Outgassing. The flow rate was measured using ATC with a 14 $\mu$g/min sensor. The ATC flow rate before $CO_2$ charging is shown in Table A. After this measurement was carried out on each tube, the same tube was then charged with $CO_2$ according to the Protocol For Charging Blood Tubes with $CO_2$, using a $CO_2$ charge time of 10 minutes. The flow rate was remeasured in the same manner, and the result is again reported in Table A.

Referring to Table A, the average outgassing flow rate of the COC tubes before $CO_2$ charging was 1.43 micrograms per minute. The average outgassing flow rate of the COC tubes after $CO_2$ charging was 3.48 micrograms per minute. This result demonstrated that $CO_2$ can be adsorbed in sufficient quantity on the uncoated tubes to substantially facilitate ATC outgassing measurement.

**Table A: Effect of $CO_2$ Charging COC on Outgassing**

| $CO_2$ Charging Status | Outgassing: Average ATC Flow Rate ($\mu$g/min) | Standard Deviation ($\mu$g/min) |
|---|---|---|
| Before $CO_2$ Charging | 1.43 | 0.107 |
| After $CO_2$ Charging | 3.48 | 0.133 |

**Example 2**

**$CO_2$ Charging Time vs. Outgassing Rate**

In this Example, the length of $CO_2$ charging time was varied to show its effect on outgassing. The ATC test time was also increased to five minutes, so the results of this test are not directly comparable with those of Example 1. The test was otherwise carried out similarly to the "After $CO_2$ Charging" test of Example 1.

The results are presented in Table B, which shows that the outgassing flow rate increases as the charge time increases, though the increase in the flow rate per minute of charging is less as the charging time is extended.

**Table B: Effect of $CO_2$ Charge Time On ATC Flow Rate**

| $CO_2$ Charge Time - Uncoated COC Tubes (Minutes) | Outgassing: Average ATC Flow Rate ($\mu$g/min) |
|---|---|
| 10 | 8.84 |
| 12 | 9.33 |
| 14 | 9.57 |

## Example 3

### Enhanced Microflow Separation of Uncoated and $SiO_x$-Coated Cyclic Olefin Copolymers with Carbon Dioxide ($CO_2$) Purging.

Ten uncoated and $SiO_x$-coated cyclic olefin copolymer (COC) 13x75 mm (0.85mm thick) injection-molded tubes were individually pressurized on the plasma coating apparatus under a 25 psi carbon dioxide ($CO_2$) pressure for 12 minutes. $SiO_x$ coating was performed on the plasma coating apparatus described in the protocol. $SiO_x$-coated tubes were allowed to cool to ambient temperature after coating prior to $CO_2$ pressurization; the standard plasma coater apparatus was modified with valving to permit $CO_2$ pressurization with a $CO_2$ pressure cylinder using the standard tube holder; tubes were evaluated immediately after $CO_2$ pressurization.

After $CO_2$ pressurization, the tubes were analyzed with a 0-14 microgram/liter (ug/L) microflow range ATC instrument, under analysis conditions previously described. The results of ten averaged readings (Table C) indicate excellent separation of greater than 4 ug/L units between uncoated and $SiO_x$-coated COC tubes. Comparatively, in the absence of $CO_2$ pressurization, (a) the microflow values for both uncoated and coated tubes are lower (1.25-2.00 ug/L) and (b) no separation between uncoated and $SiO_x$-coated COC tubes is achieved.

**Table C. Plasma Coating Parameters.**

| | | | |
|---|---|---|---|
| Power (Watts): | 70 | Capillary Length (in.): | 52.5 |
| Coating Time (Seconds): | 6 | | |
| $O_2$ Flow (sccm): | 70 | Inlet Pressure (torr.): | 7.2 |

**Table D. Average Microflow between Uncoated and $SiO_x$-Coated COC Tubes with $CO_2$ Purge.**

| Time (sec) | Uncoated (ug/L) | Coated (ug/L) |
|---|---|---|
| 300 | 9.33* | 5.29* |
| * average of ten samples | | |

## Example 4

### Fast, Six-Sigma Microflow Separation of Uncoated and $SiO_x$-Coated Cyclic Olefin Copolymers with Carbon Dioxide ($CO_2$) Purging.

Ten uncoated and $SiO_x$-coated cyclic olefin copolymer (COC) 13x75 mm (0.85mm thick) injection-molded tubes were individually pressurized on the plasma coating apparatus under a 25 psi carbon dioxide ($CO_2$) pressure for 12 minutes. $SiO_x$ coating was performed on a plasma coating apparatus described previously. $SiO_x$-coated tubes were allowed to cool to ambient temperature after coating prior to $CO_2$ pressurization; the standard plasma coater apparatus was modified with valving to permit $CO_2$ pressurization with a $CO_2$ pressure cylinder using the standard tube holder; tubes were evaluated immediately after $CO_2$ pressurization.

After $CO_2$ pressurization, the tubes were analyzed with a 0-14 microgram/liter (ug/L) microflow range ATC instrument, under analysis conditions previously described. Microflow data for each tube was collected at approximately every 0.3 seconds.

Data Analysis:

To achieve statistically significant, six-sigma separation between uncoated and $SiO_x$-coated microflow rates, the Average Uncoated Tube Microflow (minus three standard deviations) must be greater than the Average $SiO_x$-Coated Tube Microflow (plus three standard deviations). This condition is achieved between 0.96 and 1.29 seconds after start of measurement, as shown in Table E.

**Table E. Time of Uncoated Tube Microflow (minus 3 Std. Dev.) and $SiO_x$-Coated Tube Microflow (plus 3 Std. Dev.)**

| Time (sec) | Uncoated (ug/L) | Standard Deviation | UC Ave - 3Standard Deviations | Coated (ug/L) | Standard Deviation | C Ave + 3standard Deviations |
|---|---|---|---|---|---|---|
| 0.96 | 0.7495* | 0.02 | 0.68 | 0.63803* | 0.02 | 0.71 |
| 1.29 | 0.82274* | 0.02 | 0.76 | 0.68757* | 0.02 | 0.75 |
| * average of ten samples | | | | | | |

**Example 5**

**Carbon dioxide infusion or charging via evacuation/pressurization process**

Into a pressure vessel are placed several uncoated and $SiO_x$-plasma coated COC 13x75 mm tubes. The vessel is evacuated to 1 Torr for 30 minutes; followed by pressurization 3-10 psig from a carbon dioxide cylinder for 30 minutes. The tubes are then evaluated with the ATC microflow instrument under conditions described in Example 1. The microflow discrimination observed between uncoated and plasma coated COC tubes is comparable to that observed for uncoated and $SiO_x$-coated PET tubes (Figures 31 & 32), and much better than using nitrogen as the purge gas.

**Example 6**

**Carbon dioxide infusion or charging via pressurization process**

Tubes were mounted in a pressurization chamber and pressurized with $CO_2$ gas at 75 psi and 23°C for 1.5 hours. The tubes were measured using the above conditions on the ATC unit. The tubes showed good separation between the coated and uncoated 8007 COC 13 x 75mm tubes.

**Example 7**

**Enhanced Microflow Separation of Uncoated and $SiO_x$-Coated Cyclic Olefin Copolymers with Argon (Ar) Purging.**

Ten uncoated and $SiO_x$-coated cyclic olefin copolymer (COC) 13x75(0.85mm thick) injection-molded tubes were individually pressurized on the plasma coating apparatus under a 25 psi argon (Ar) pressure for 12 minutes. SiOx coating was performed on a plasma coating apparatus described previously; $SiO_x$-coated tubes were allowed to cool to ambient temperature after coating prior to Ar pressurization; the standard plasma coater apparatus was modified with valving to permit Ar pressurization with an Ar pressure cylinder using the standard tube holder; tubes were evaluated immediately after Ar pressurization.

After Ar pressurization, the tubes were analyzed with a 0-14 microgram/liter (ug/L) microflow range ATC instrument, under analysis conditions previously described. The results of ten averaged readings (Table F) indicate good separation of greater than 1 ug/L units between uncoated and $SiO_x$-coated COC tubes.

**Table F. Average Microflow between Uncoated and $SiO_x$-Coated COC Tubes with Argon Purge.**

| Time (sec) | Uncoated (ug/L) | Coated (ug/L) |
|---|---|---|
| 205 | 3.26* | 2.07 |
| * average of nine samples | | |

**Example 8**

**Enhanced Microflow Separation of Uncoated and $SiO_x$-Coated Cyclic Olefin Copolymers with Nitrogen ($N_2$) Purging.**

Ten uncoated and SiOx-coated cyclic olefin copolymer (COC) 13x75 (0.85 mm thick) injection-molded tubes were individually pressurized on the plasma coating apparatus under a 25 psi nitrogen ($N_2$) pressure for 12 minutes. SiOx coating was performed on a plasma coating apparatus described previously; $SiO_x$-coated tubes were allowed to cool to ambient temperature after coating prior to $N_2$ pressurization; the standard plasma coater apparatus was modified with valving to permit $N_2$ pressurization with a $N_2$ pressure cylinder using the standard tube holder; tubes were evaluated immediately after $N_2$ pressurization.

After $N_2$ pressurization, the tubes were analyzed with a 0-14 microgram/liter (ug/L) microflow range ATC instrument, under analysis conditions previously described. The results of ten averaged readings (Table G) indicate good separation between uncoated and $SiO_x$-coated COC tubes.

**Table G. Average Microflow between Uncoated and $SiO_x$-Coated COC Tubes with Nitrogen Purge.**

| Time (sec) | Uncoated (ug/L) | Coated (ug/L) |
|---|---|---|
| 143 | 2.38* | 1.96 |
| * average of nine samples | | |

**Example 9**

**$CO_2$ Purge Gas in Microflow Measurement**

This Example shows that a $CO_2$ charge time as short as one second can be used to improve the outgassing detection result. Uncoated COC vessels respectively made of COC 8007 and COC 6015 were evacuated in apparatus similar to that of Fig. 2, then purged with nitrogen gas or carbon dioxide for one second to break the vacuum. Outgassing of the tubes was then measured using the equipment shown in FIG. 7. A purge time of 1 second with carbon dioxide replacing nitrogen resulted in an increased microflow amplitude signal for uncoated COC injection molded 13x75 tubes (Table I).

**Table I. Microflow Amplitude (after 90 sec) Comparison of Uncoated TOPAS 8007 & 6015 Tubes**

| **Tube material** | **microflow (μg/min)** | |
|---|---|---|
| | **$N_2$** | **$CO_2$** |
| COC 8007 | 0.45-0.55 | 1.0 |
| COC 6015 | 1.1 | 2.2 |

**Example 10**

**Additional Microflow Measurement**

Into a pressure vessel are placed several uncoated and $SiO_x$-plasma coated COC 13x75 mm tubes. The vessel is evacuated to 1 Torr for 30 minutes, followed by pressurization 3-10 psig from a carbon dioxide cylinder for 30 minutes. The tubes are then evaluated with the equipment shown in FIG. 7. As can be seen in FIG. 19, the microflow discrimination observed between uncoated and plasma coated COC tubes is comparable to that observed for uncoated and $SiO_x$-coated PET tubes (FIGS. 8 and 9 of the present application which are identical to FIGS. 31 and 32 of EP2251671 A2), and much better than using nitrogen as purge gas.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims

should not be construed as limiting the scope.

FURTHER EMBODIMENTS OF THE INVENTION

1. A method for inspecting the product of a coating process wherein a coating has been applied to the surface of a substrate to form a coated surface, the method comprising the steps:

   (a) providing the product as inspection object;
   (c) measuring the release of at least one volatile species from the inspection object into the gas space adjacent to the coated surface; and
   (d) comparing the result of step (c) with the result of step (c) for at least one reference object measured under the same test conditions, thus determining the presence or absence of the coating, and/or a physical and/or chemical property of the coating.

2. The method of embodiment 1, wherein a physical and/or chemical property of the coating is determined which is selected from the group consisting of its barrier effect, its wetting tension, and its composition, and preferably is its barrier effect.

3. The method according to embodiment 1 or 2, wherein step (c) is performed by measuring the mass flow rate or volume flow rate of the at least one volatile species in the gas space adjacent to the coated surface.

4. The method according to any of the preceding embodiments, comprising as an additional step between steps (a) and (c) the step of (b) changing the atmospheric pressure in the gas space adjacent to the coated surface such that a pressure differential through the coated surface is provided and a higher mass flow rate or volume flow rate of the volatile species may be realized than without the pressure differential.

5. The method according to any of the preceding embodiments, wherein the inspection object is contacted with a volatile species in step (a), preferably a volatile species selected from the group consisting of air, nitrogen, oxygen, water vapor, carbon dioxide, argon, and a combination thereof, in order to allow the adsorption or absorption of said volatile species onto or into the material of the inspection object; and wherein the subsequent release of said volatile species from the inspection object is measured in step (c) as part of the measured total release of volatile species.

6. The method according to any of the preceding embodiments, wherein a plurality of different volatile species is measured in step (c), and wherein preferably substantially all the volatile species released from the inspection object are measured in step (c).

7. The method according to any of the preceding embodiments, wherein the substrate is a polymeric compound, preferably is a polyester, a polyolefin, a cyclic olefin copolymer, a polycarbonate, or a combination of these.

8. The method according to embodiment 7, wherein the substrate is COC.

9. The method according to embodiment 8, wherein the volatile species is carbon dioxide or Argon.

10. The method according to embodiment 7, wherein the substrate is PET.

11. The method according to embodiment 10, wherein the volatile species is water.

12. The method according to any of the preceding embodiments, wherein the coating is a coating prepared by PECVD from an organosilicon precursor.

13. The method according to any of the preceding embodiments, wherein

   (i) the coating is a barrier coating, preferably is a $SiO_x$ layer wherein x is from about 1.5 to about 2.9; and/or
   (ii) the coating is a coating modifying the lubricity and/or surface tension of the coated substrate, preferably is a layer of $Si_wO_xC_yH_z$, where w is 1, x is from about 0.5 to 2.4, y is from about 0.6 to about 3, and z is from 2 to about 9.

14. The method according to any of the preceding embodiments, wherein the volatile species is (i) a volatile species released from the coating, preferably is a volatile coating component, and wherein the inspection is performed to determine the presence, the properties and/or the composition of the coating; or

(ii)a volatile species released from the substrate and wherein the inspection is performed to determine the presence of the coating and/or the barrier effect of the coating; or
(iii) a combination of (i) and (ii).

15. The method according to any of the preceding embodiments, wherein the conditions effective to distinguish the presence or absence of the coating, and/or to determine a physical and/or chemical property of the coating include a test duration of less than one hour, or less than one minute, or less than 50 seconds, or less than 40 seconds, or less than 30 seconds, or less than 20 seconds, or less than 15 seconds, or less than 10 seconds, or less than 8 seconds, or less than 6 seconds, or less than 4 seconds, or less than 3 seconds, or less than 2 seconds, or less than 1 second.

16. The method according to any of the preceding embodiments, wherein the coating is a PECVD coating performed under vacuum conditions and wherein the subsequent outgassing measurement is conducted

(i) without breaking the vacuum used for PECVD; or
(ii)by charging the coated product with a volatile species after the coating and subsequently performing the outgassing measurement.

17. An apparatus for performing the method according to any of embodiments 1 to 16.

18. A program element for inspecting the product of a coating process, which, when being executed by a processor of a vessel processing system (20), is adapted to instruct the processor to control the vessel processing system such that it carries out the method of one of embodiments 1 to 17.

## Claims

**1.** A syringe having a barrel and a plunger, the barrel having an inner surface and the plunger having a side surface engaging the barrel inner surface, wherein at least one of the barrel inner surface and the plunger side surface serves as substrate which is coated with a lubricity layer;
wherein the lubricity layer comprises a layer of $Si_wO_xC_yH_z$ or $Si_wN_xC_yH_z$, where w is 1, x is from about 0.5 to 2.4, y is from about 0.6 to about 3, and z is from 2 to about 9;
wherein the lubricity layer has a thickness by transmission electron microscopy (TEM) between 10 and 500 nm;
wherein the lubricity layer has been deposited by plasma enhanced chemical vapor deposition (PECVD) from a precursor selected from a linear siloxane, a monocyclic siloxane, a polycyclic siloxane, a polysilsesquioxane, a linear silazane, a monocyclic silazane, a polycyclic silazane, a polysilsesquiazane, a silatrane, a silquasilatrane, a silproatrane, an azasilatrane, an azasilquasiatrane, an azasilproatrane, or a combination of any two or more of these precursors;
wherein the surface of the lubricity layer has a lower plunger sliding force or breakout force than the untreated substrate.

**2.** The syringe according to Claim 1, in which the precursor comprises hexamethyldisiloxane, octamethyltrisiloxane (OMCTS), decamethyltetrasiloxane, hexamethyl-cyclotrisiloxane, octamethylcyclotetrasiloxane, decamethylcyclopentasiloxane dodecamethylcyclohexasiloxane, SST-eM01 poly(methylsilsesquioxane), in which each R is methyl, SST-3MH1.1 poly(Methyl-Hydridosilsesquioxane), in which 90% of the R groups are methyl and 10% are hydrogen atoms, methyltrimethoxysilane, or a combination of any two or more of these.

**3.** The syringe of claim 1, in which the precursor comprises octamethylcyclotetrasiloxane (OMCTS).

**4.** The syringe of any one of claims 1 to 3, in which the plasma has been generated by a radio frequency (RF) power source.

**5.** The syringe of any one of claims 1 to 4, in which the substrate comprises glass.

**6.** The syringe of any one of claims 1 to 5, in which the substrate comprises a polymer.

**7.** The syringe of any one of claims 1 to 6, in which the lubricity layer is effective to reduce the plunger sliding force or breakout force by at least 45 percent relative to an untreated syringe.

**8.** The syringe of any one of claims 1 to 7, in which the lubricity layer is coated onto the barrel inner surface.

**9.** The syringe of any one of claims 1 to 7, in which the lubricity layer is coated onto the plunger side surface.

**10.** The syringe of any one of claims 1 to 7, in which the lubricity layer is coated onto the barrel inner surface and the plunger side surface.

**11.** The syringe of any one of claims 1 to 10, further comprising a barrier coating of $SiO_x$, in which x is from about 1.5 to about 2.9, on the inner surface of the barrel.

**12.** The syringe of claim 11, in which the lubricity layer is coated onto the barrier coating of SiOx.

**13.** The syringe of any one of claims 1 to 12, in which the plunger comprises a piston having a front face, a generally cylindrical side face defining the side surface, and a back portion, the side surface being configured to movably seat within the syringe barrel, a lubricity layer interfacing with the side surface, and a push rod engaging the back portion and configured for advancing the piston in the syringe barrel.

**14.** The syringe of any one of claims 1 to 13, in which the substrate is the inside wall of the syringe barrel defining a lumen, the lumen having a void volume of from 0.5 to 50 mL.

**15.** The syringe of any one of claims 1 to 14, in which the lubricity layer has a lower wetting tension than the uncoated surface.

**16.** The syringe of any one of claims 1 to 15, which contains a compound or composition in its lumen which is a biologically active compound or composition, a biological fluid, a citrate or a citrate containing composition, a medicament, blood or blood cells.

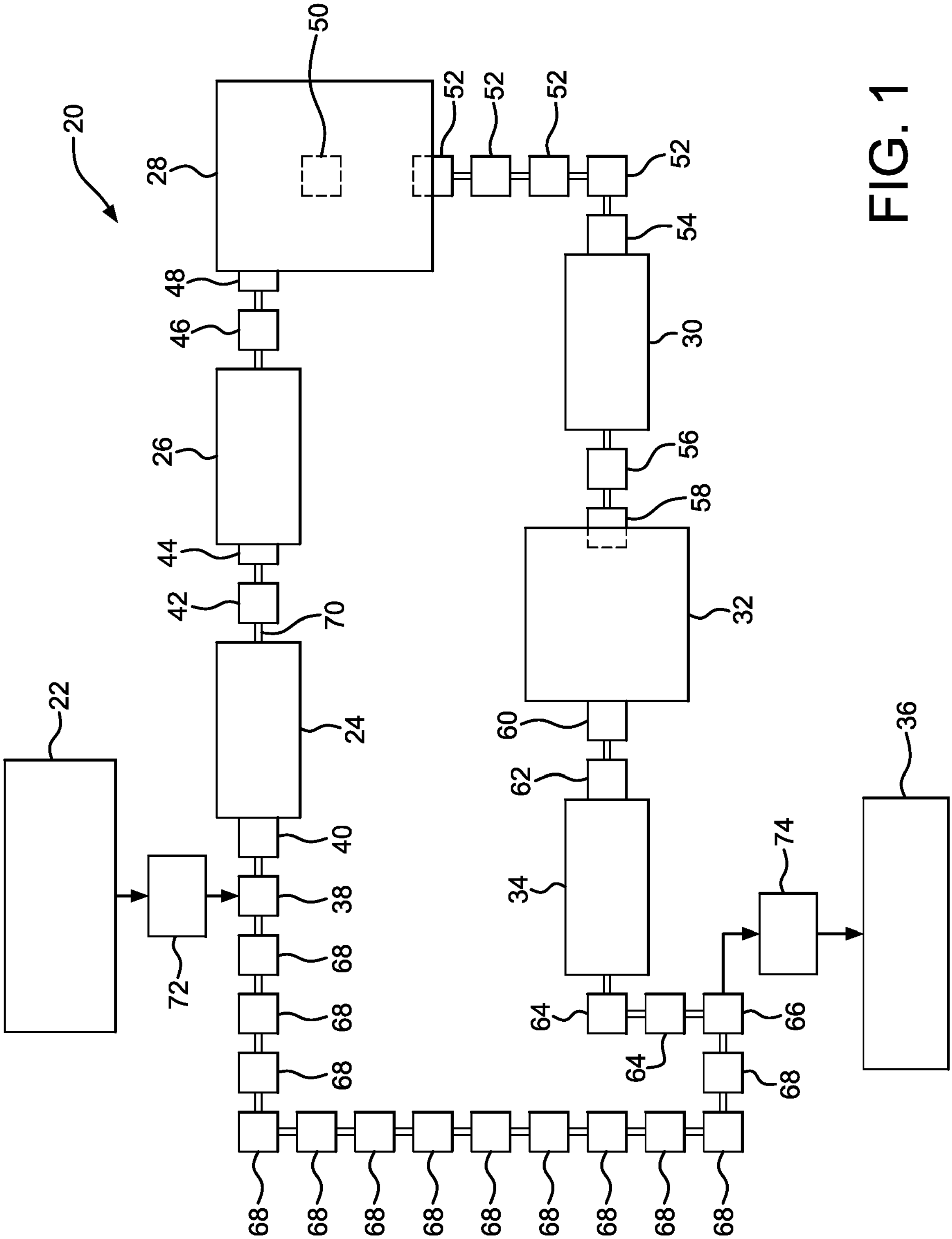
20
22
24
26
28
30
32
34
36
38
40
42
44
46
48
50
52
54
56
58
60
62
64
66
68
70
72
74

FIG. 1

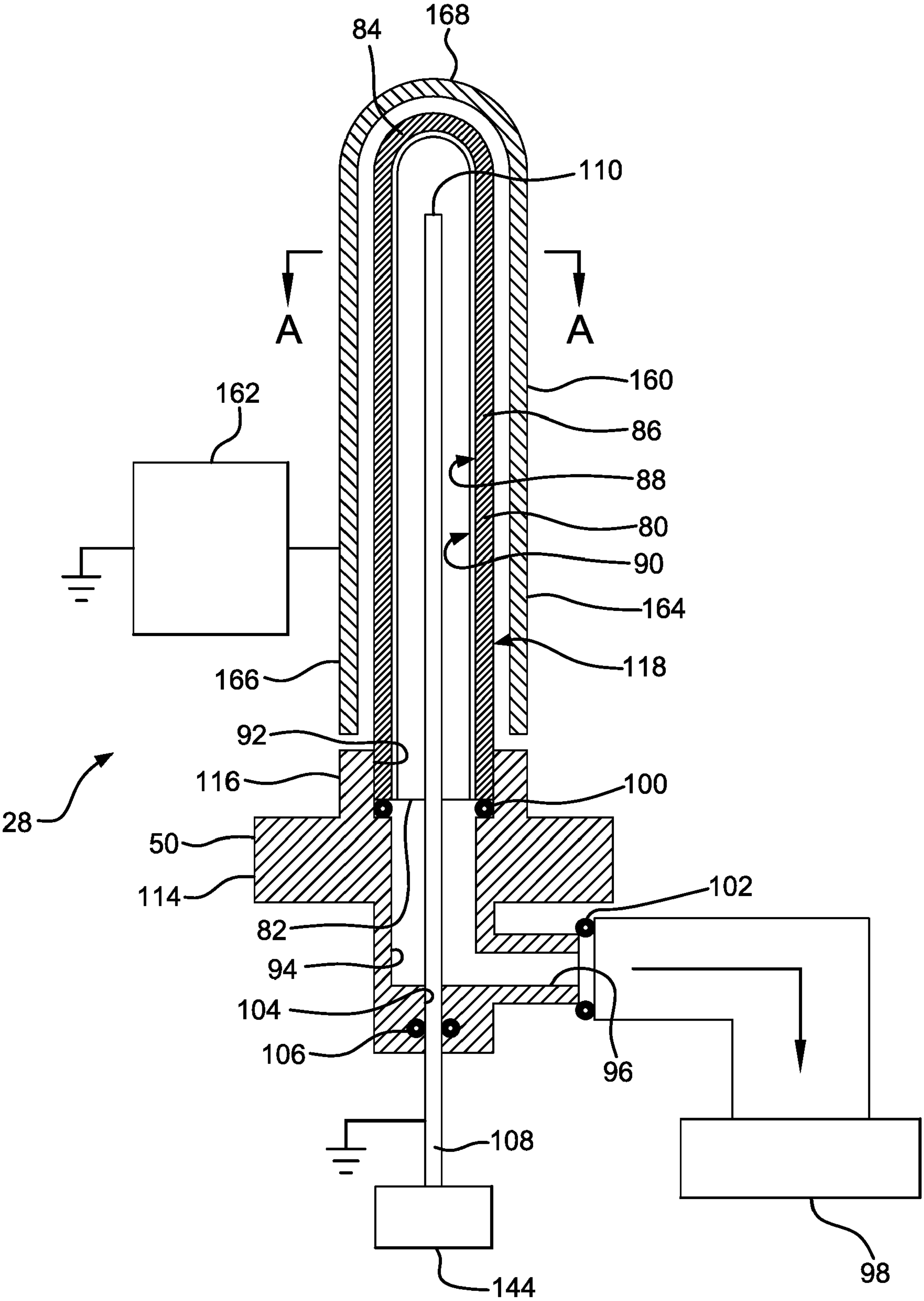
168
84
110
A
A
160
162
86
88
80
90
164
118
166
92
116
100
28
50
114
102
82
94
104
96
106
108
144
98

FIG. 2

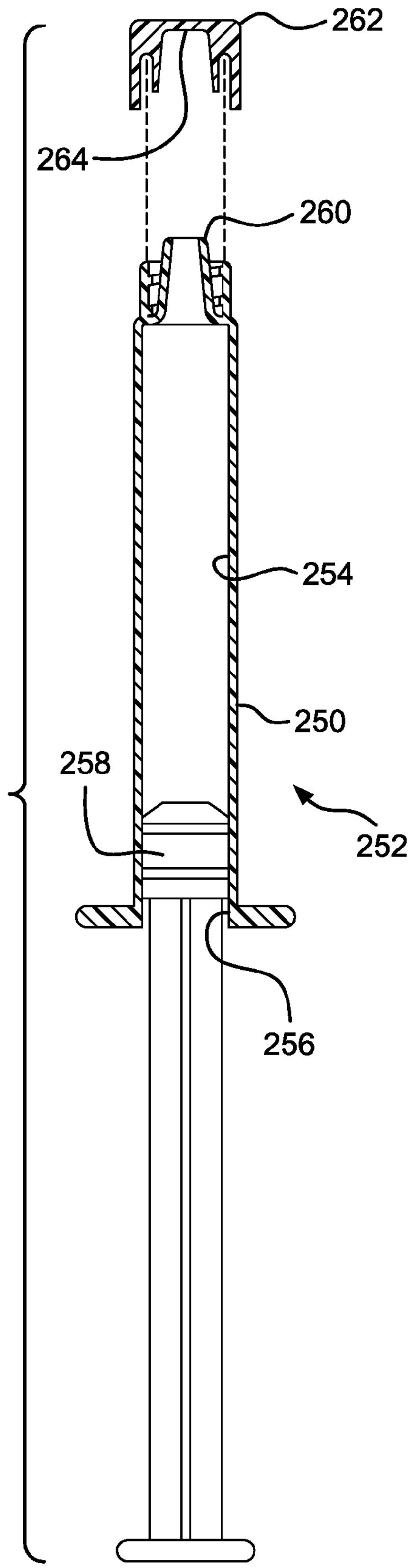


FIG. 3

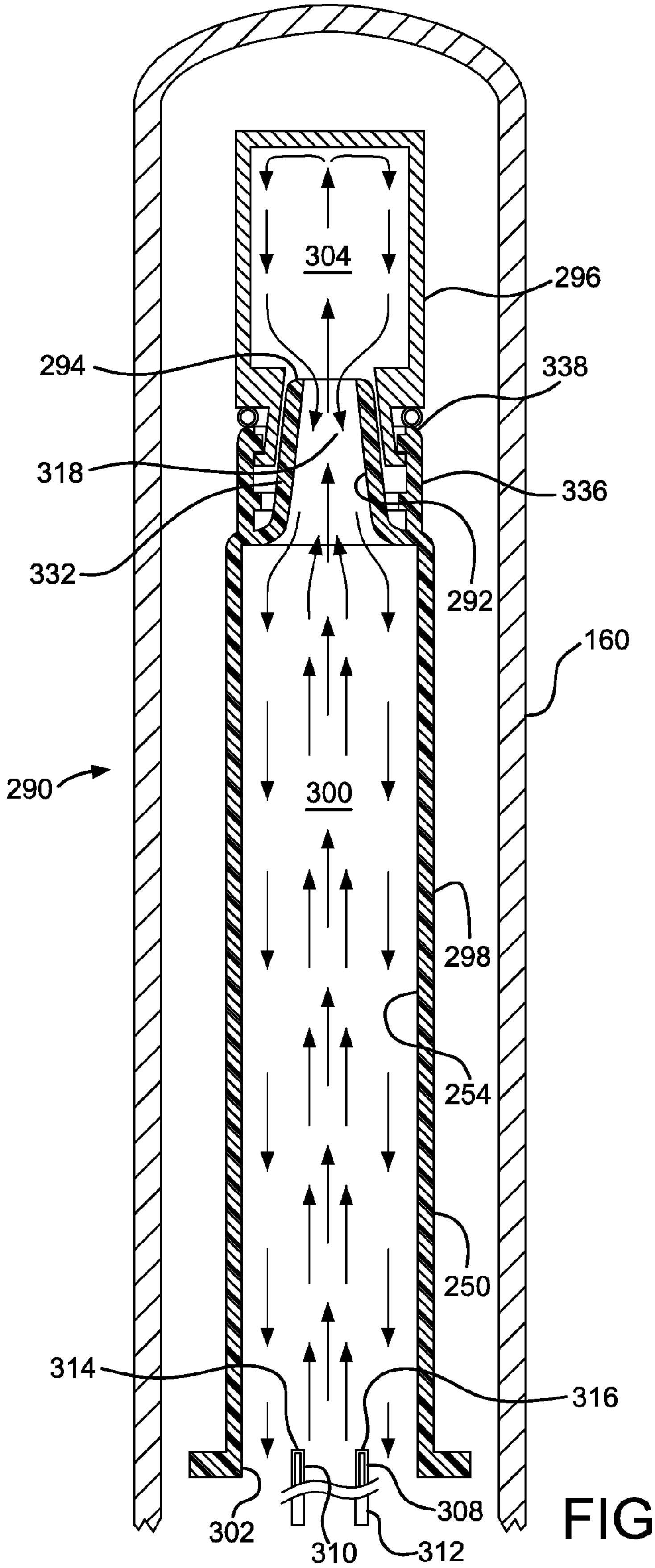
304
296
294
338
318
336
332
292
160
290
300
298
254
250
314
316
302
308
310
312

FIG. 4

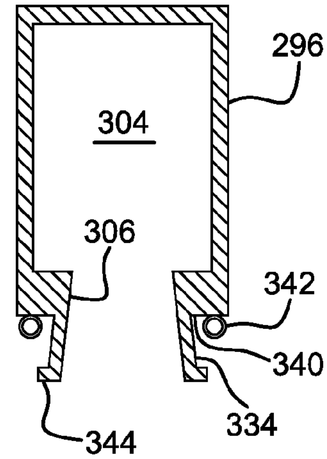
296
304
306
342
340
334
344

FIG. 5

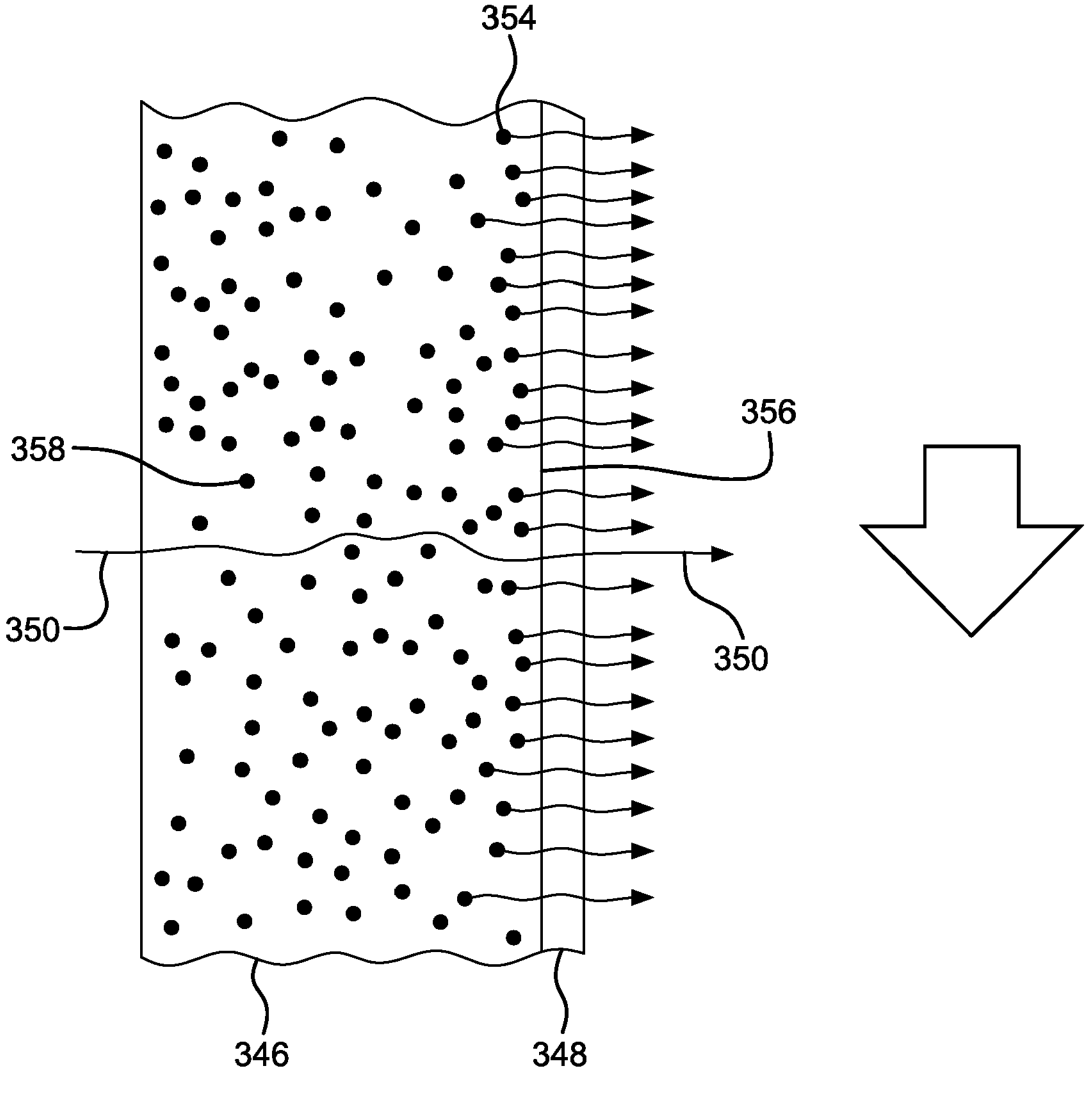


FIG. 6

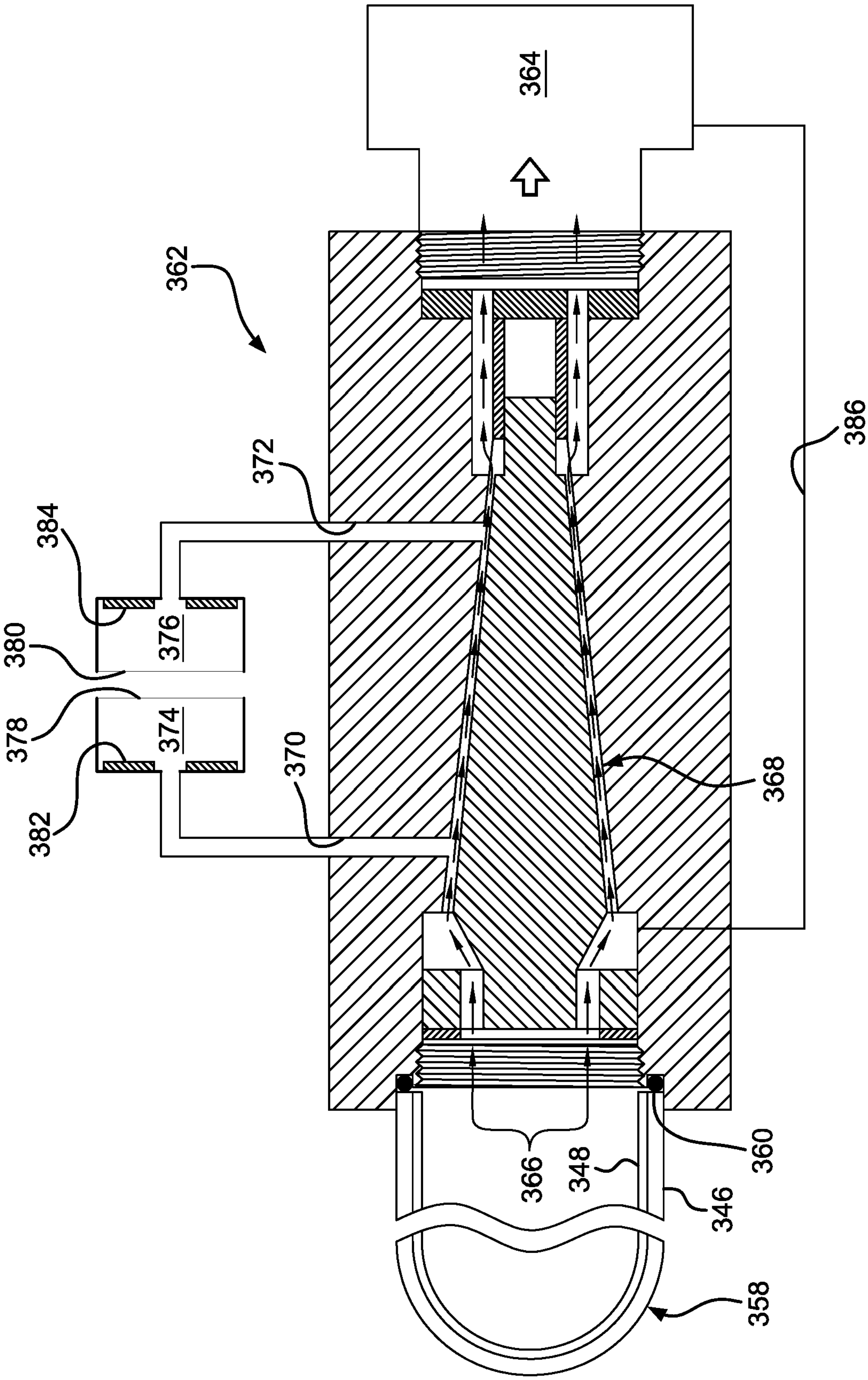
364
362
372
384
380
376
378
374
382
370
368
386
366
348
360
346
358

FIG. 7

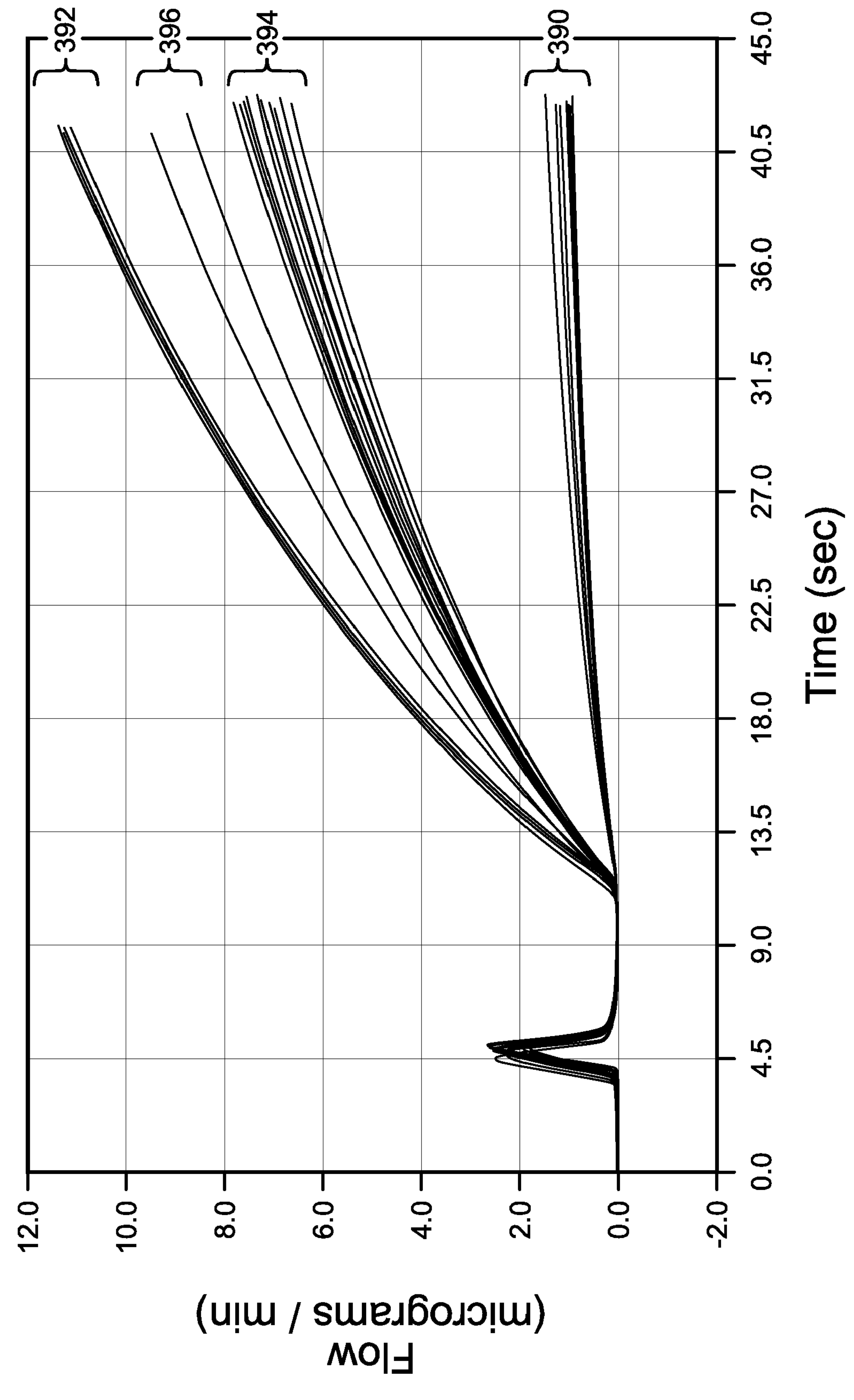
Flow (micrograms / min)
12.0
10.0
8.0
6.0
4.0
2.0
0.0
-2.0
0.0
4.5
9.0
13.5
18.0
22.5
27.0
31.5
36.0
40.5
45.0
Time (sec)
392
396
394
390

FIG. 8

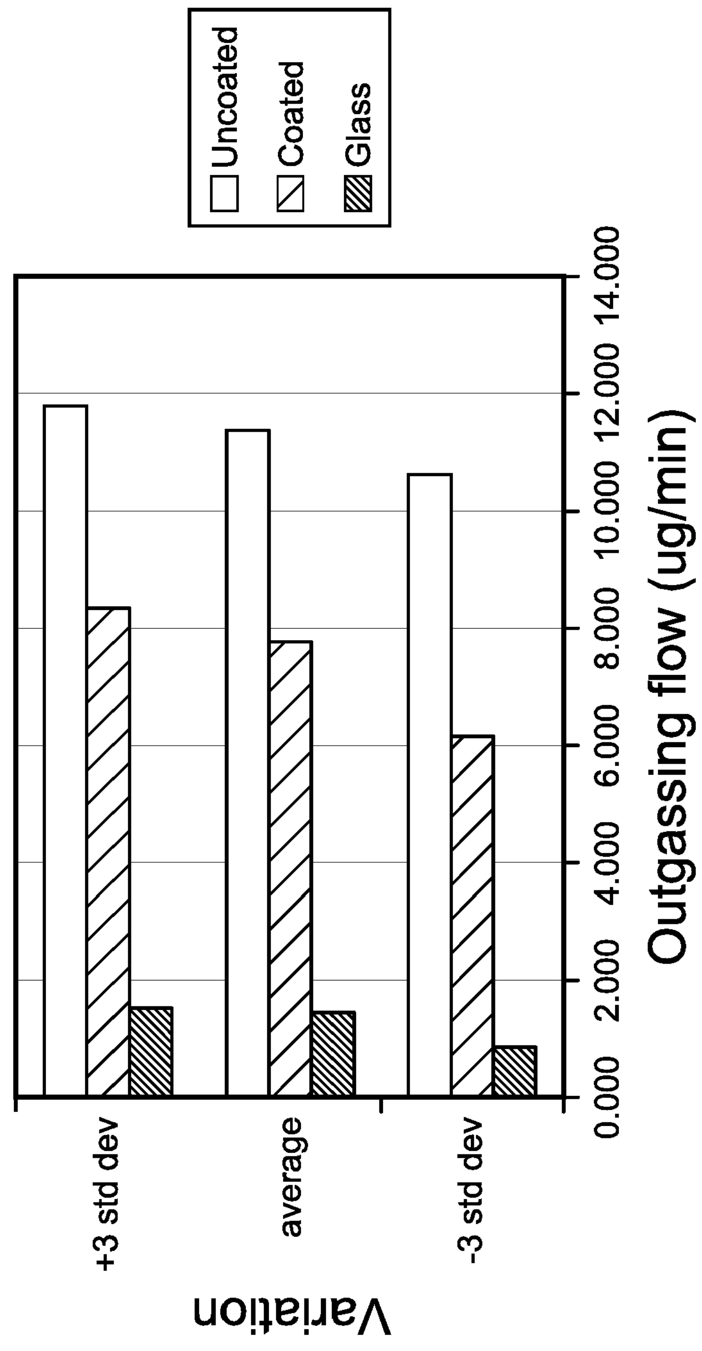
Uncoated
Coated
Glass
+3 std dev
average
-3 std dev
Variation
0.000
2.000
4.000
6.000
8.000
10.000
12.000
14.000
Outgassing flow (ug/min)

FIG. 9

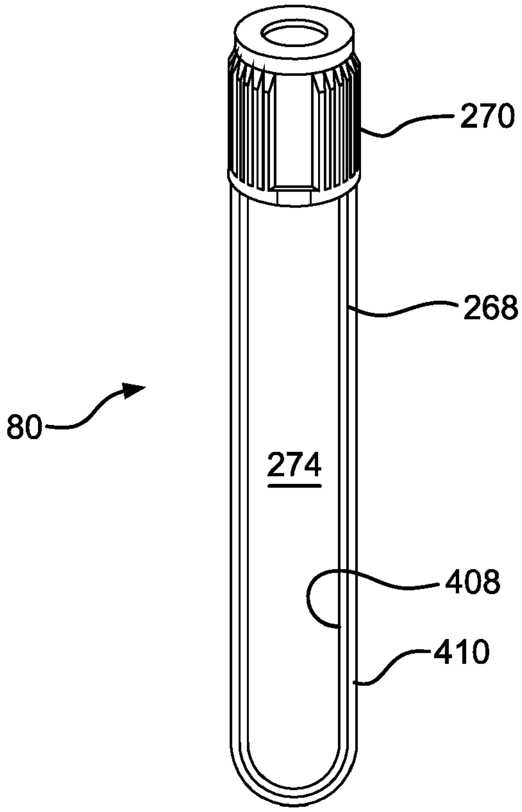


FIG. 10

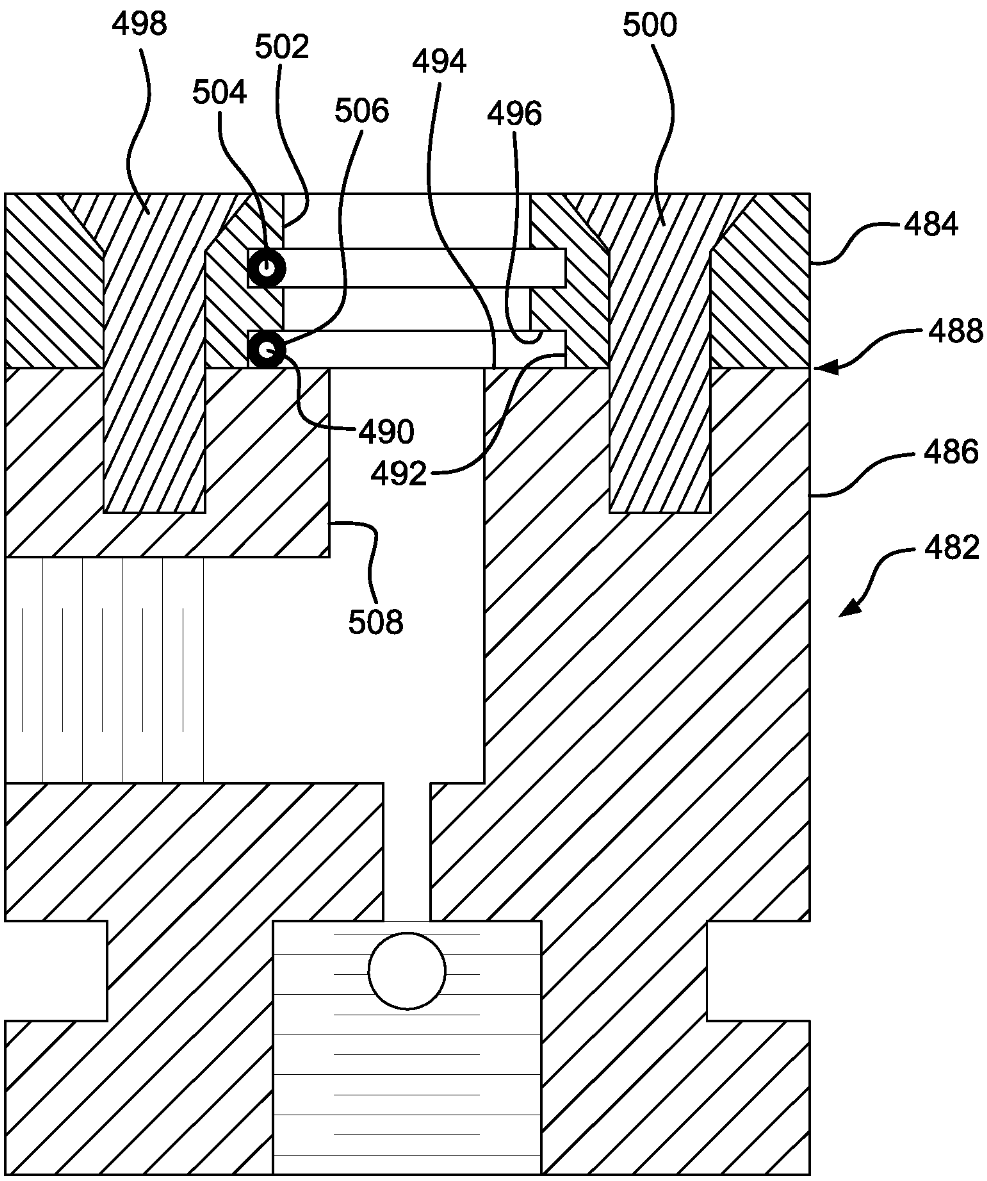


FIG. 11

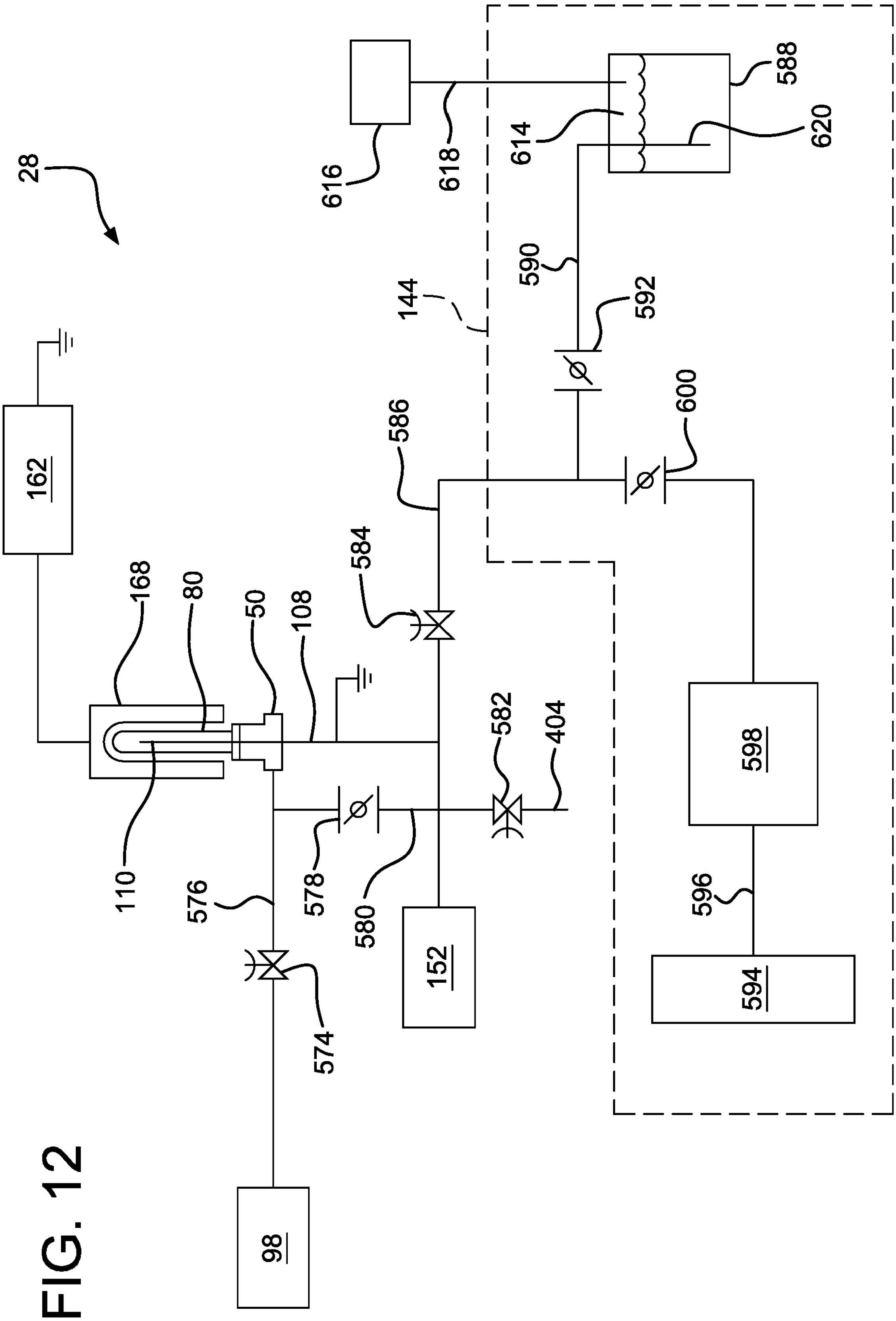
28
162
168
80
50
108
110
576
578
580
574
584
582
404
586
152
98
144
616
618
614
588
620
590
592
600
598
596
594

FIG. 12

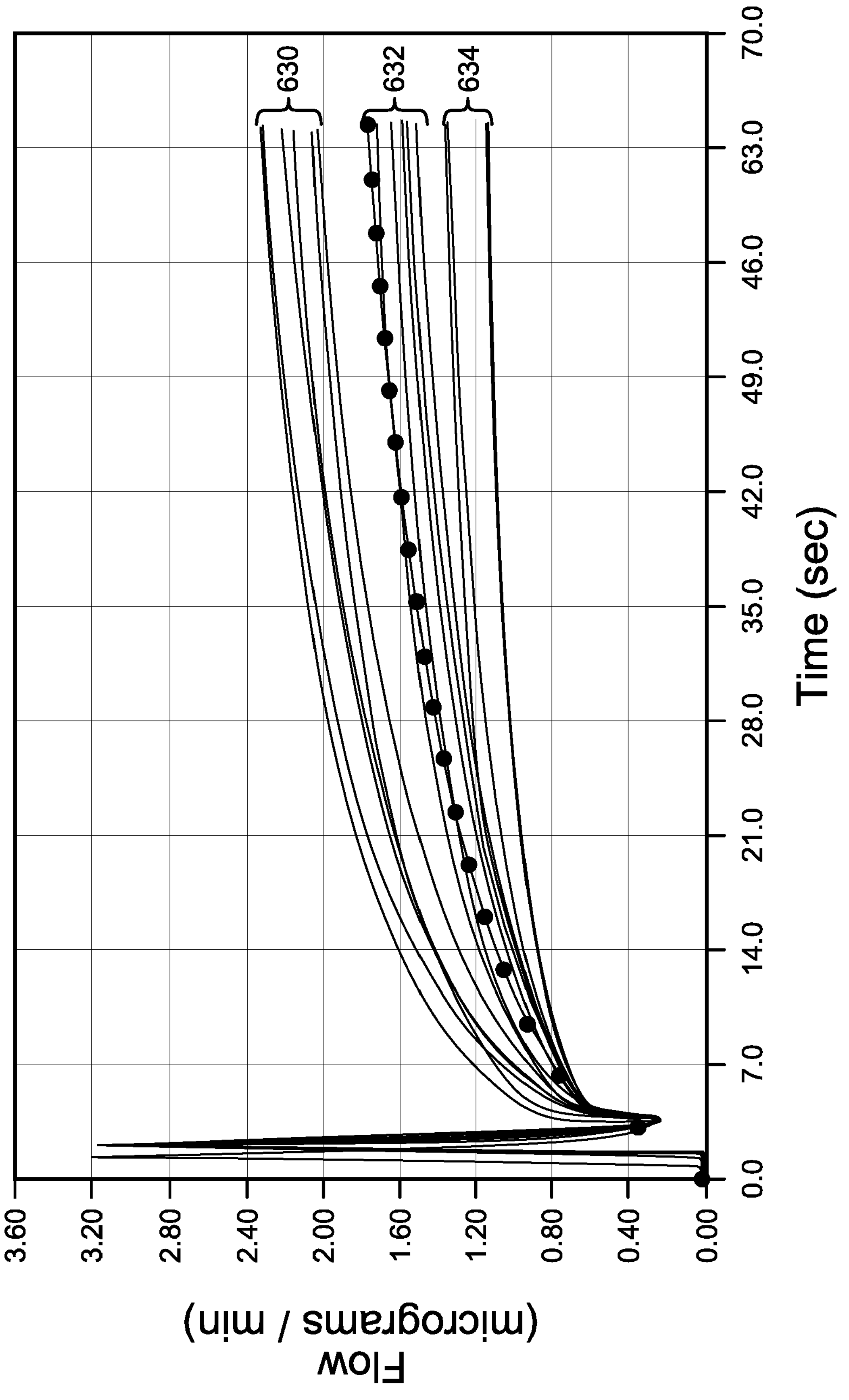
Flow
(micrograms / min)
3.60
3.20
2.80
2.40
2.00
1.60
1.20
0.80
0.40
0.00
0.0
7.0
14.0
21.0
28.0
35.0
42.0
49.0
46.0
63.0
70.0
Time (sec)
630
632
634

FIG. 13

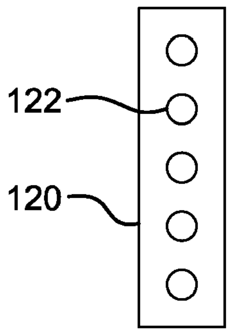


FIG. 14

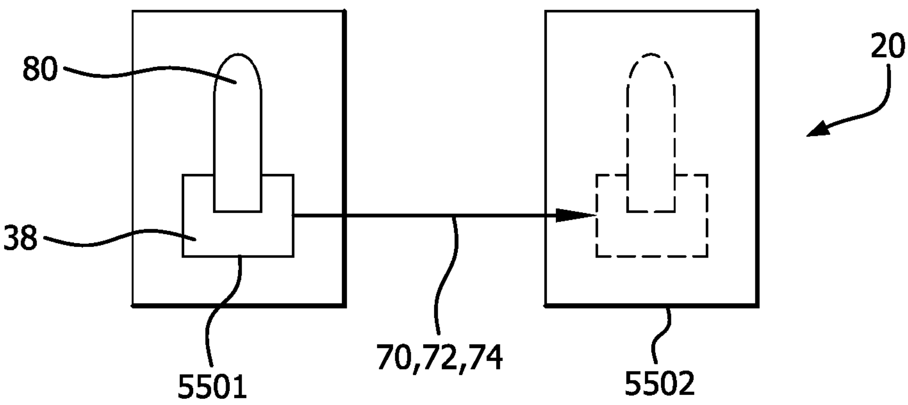


FIG. 15

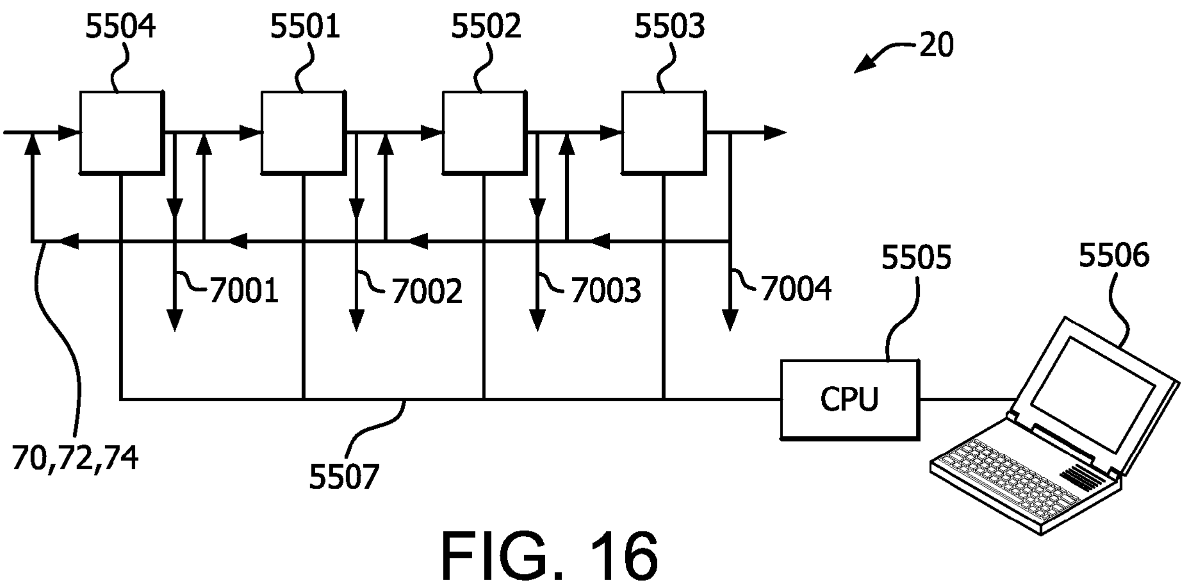


FIG. 16

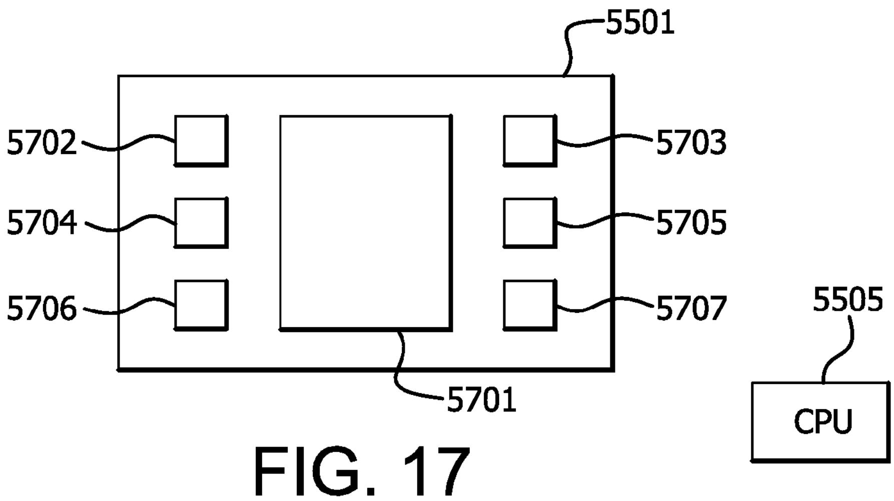


FIG. 17

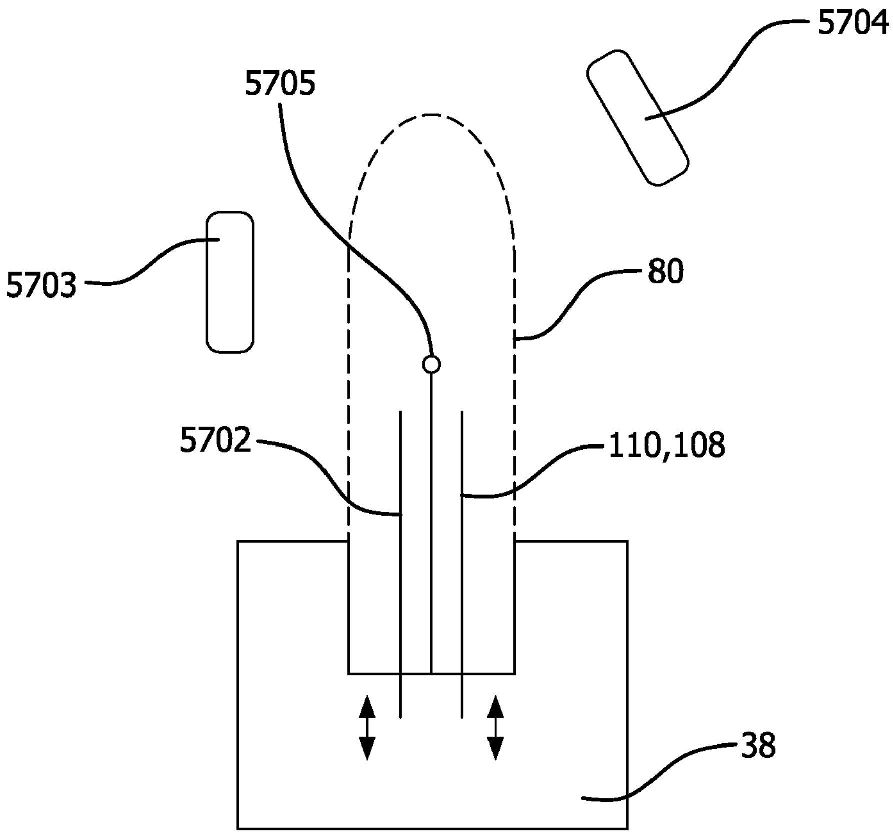


FIG. 18

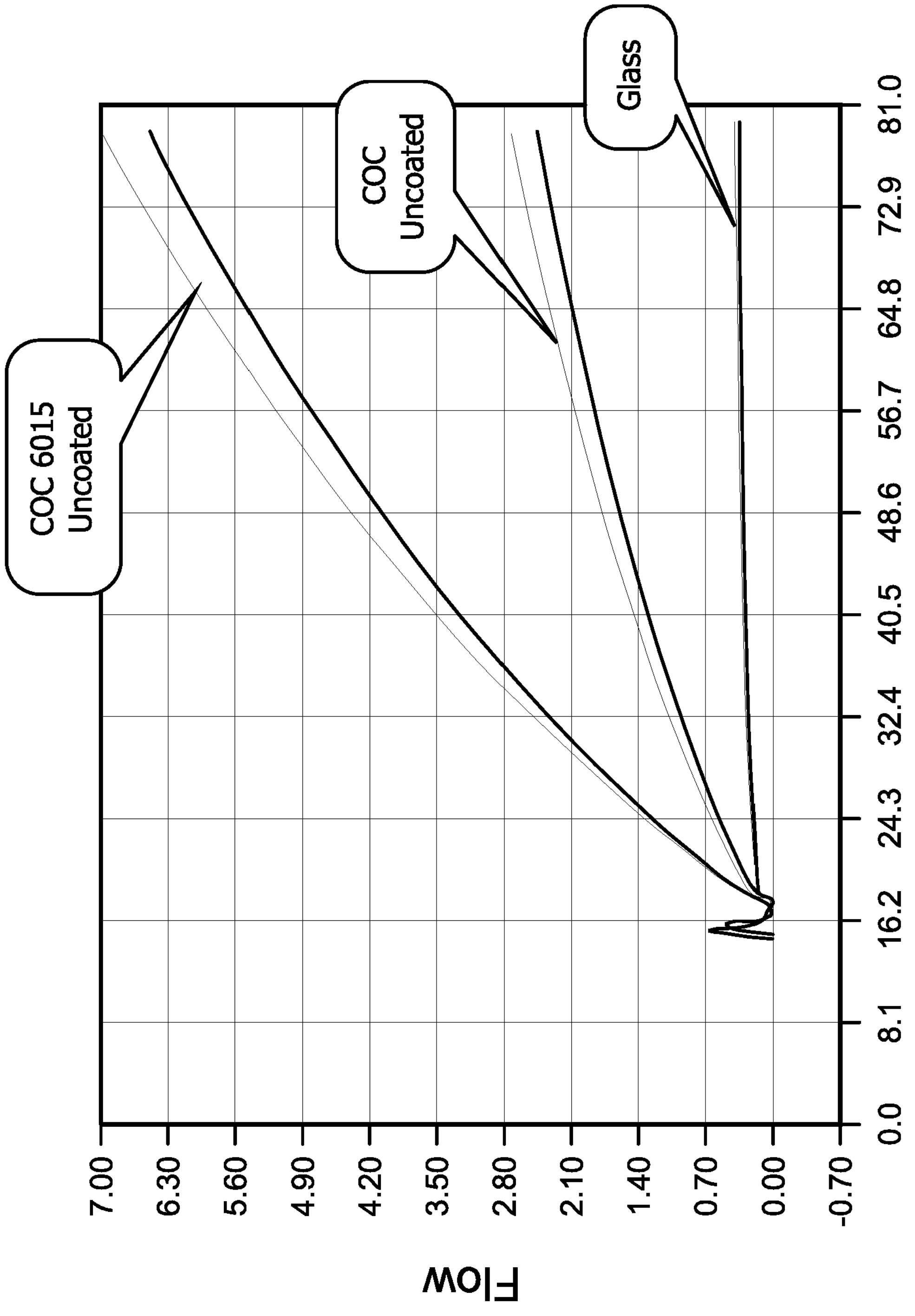
COC 6015
Uncoated
COC
Uncoated
Glass
Flow
7.00
6.30
5.60
4.90
4.20
3.50
2.80
2.10
1.40
0.70
0.00
-0.70
0.0
8.1
16.2
24.3
32.4
40.5
48.6
56.7
64.8
72.9
81.0

FIG. 19

Europäisches Patentamt
European Patent Office
Office européen des brevets

# EUROPEAN SEARCH REPORT

Application Number
EP 18 19 7314

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/231926 A1 (SAKHRANI VINAY G [US] ET AL) 25 November 2004 (2004-11-25)<br>* paragraphs [0036], [0042], [0044], [0045], [0054]; figure 1 *<br>----- | 1-16 | INV.<br>B05D5/08<br>A61M5/315<br>B05D7/02<br>B05D1/00<br>B65D25/14<br>C23C16/04<br>C23C16/40<br>C23C16/50<br>C23C16/505<br>C23C16/54<br>C23C16/52<br>G01N15/08<br>C03C17/30<br>C03C17/00<br><br>ADD.<br>G01N33/00 |
| X | US 4 767 414 A (WILLIAMS JOEL L [US] ET AL) 30 August 1988 (1988-08-30)<br>* Summary, example I in column 5; column 3, lines 29-31 *<br>----- | 1-16 | |
| X | US 2010/075077 A1 (BICKER MATTHIAS [DE] ET AL) 25 March 2010 (2010-03-25)<br>* paragraphs [0003], [0010] - [0012], [0019], [0025], [0063] - [0064] *<br>----- | 1-16 | |
| | | | TECHNICAL FIELDS SEARCHED (IPC) |
| | | | C03C |

The present search report has been drawn up for all claims

1

| Place of search | Date of completion of the search | Examiner |
|---|---|---|
| Munich | 20 December 2018 | Diamantouros, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 19 7314

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2018

| Patent document cited in search report | Publication date | Patent family member(s) | Publication date |
|---|---|---|---|
| US 2004231926 A1 | 25-11-2004 | EP 1732708 A2 | 20-12-2006 |
| | | EP 2676741 A2 | 25-12-2013 |
| | | JP 4747253 B2 | 17-08-2011 |
| | | JP 2007527317 A | 27-09-2007 |
| | | US 2004231926 A1 | 25-11-2004 |
| | | US 2008145565 A1 | 19-06-2008 |
| | | US 2008254304 A1 | 16-10-2008 |
| | | WO 2005094214 A2 | 13-10-2005 |
| US 4767414 A | 30-08-1988 | NONE | |
| US 2010075077 A1 | 25-03-2010 | AT 552360 T | 15-04-2012 |
| | | DE 102006058771 A1 | 19-06-2008 |
| | | EP 2106461 A1 | 07-10-2009 |
| | | JP 5197625 B2 | 15-05-2013 |
| | | JP 2010512260 A | 22-04-2010 |
| | | US 2010075077 A1 | 25-03-2010 |
| | | WO 2008071458 A1 | 19-06-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61177984 B
- US 61222727 B
- US 61213904 B
- US 61234505 B
- US 61261321 B
- US 61263289 B
- US 61285813 B
- US 61298159 B
- US 61299888 B
- US 61318197 B
- US 61333625 B
- US 61413334 B
- US 12779007 B
- EP 10162755 A
- EP 10162760 A
- EP 10162756 A
- EP 10162758 A
- EP 10162761 A
- EP 10162757 A
- US 1136097 W
- EP 2251671 A2
- US 6068884 A
- US 4844986 A
- US 20060046006 A
- US 20040267194 A
- EP 2251455 A2
- US 6165566 A
- US 5861546 A
- US 6308556 B
- US 6584828 B
- EP 1356260 A
- US 5904952 A
- US 61359434 B
- EP 2251455 A


### Non-patent literature cited in the description


- *CHEMICAL ABSTRACTS,* 2288-13-3
- **D. W. VAN KREVELEN.** Properties of Polymers. Elsivier, 1990, 538-542
- **HAYATO SONE ; YOSHINORI FUJINUMA ; SUMIO HOSAKA.** Picogram Mass Sensor Using Resonance Frequency Shift of Cantilever. *Jpn. J. Appl. Phys.,* 2004, vol. 43, 3648
- **HAYATO SONE ; AYUMI IKEUCHI ; TAKASHI IZUMI ; HARUKI OKANO ; SUMIO HOSAKA.** Femtogram Mass Biosensor Using Self-Sensing Cantilever for Allergy Check. *Jpn. J. Appl. Phys.,* 2006, vol. 43, 2301
- **HANS PETER LANG ; CHRISTOPH GERBER.** STM and AFM Studies on (Bio)molecular Systems: Unravelling the Nanoworld, Topics. *Current Chemistry,* 2008, vol. 285
- **SHIMOJIMA.** *J. Mater. Chem.,* 2007, vol. 17, 658-663
- **RUBY CHAKRABORTY.** *Dissertation,* 2008